# EUROPEAN PATENT APPLICATION

(11) **EP 1 437 404 A1**
(43) Date of publication of application: **14.07.2004**
(21) Application number: 02799492.0
(22) Date of filing: 20.09.2002
(51) Int. Cl.: C12N 15/02, C12N 5/06, C12N 9/10, C12Q 1/02, A61L 27/38, A61K 45/00, A61K 48/00

(54) **TAILOR-MADE MULTIFUNCTIONAL STEM CELLS AND UTILIZATION THEREOF**

(30) Priority: 21.09.2001 JP 2001290005
(71) Applicant: ReproCELL Inc., Tokyo 100-0011 (JP); Nakatsuji, Norio, Kyoto-shi, Kyoto 606-0014 (JP); Tada, Takashi, Kyoto-shi, Kyoto 606-8314 (JP)
(72) Inventor: NAKATSUJI, Norio, Kyoto-shi, Kyoto 606-0014 (JP); TADA, Takashi, Kyoto-shi, Kyoto 606-8314 (JP); TADA, Masako, Kyoto-shi, Kyoto 606-8314 (JP)
(74) Representative: Holliday, Louise Caroline
(86) International application number: PCT/JP2002/009732
(87) International publication number: WO 2003/027278

(57) **Abstract**

An object of the present invention is to efficiently establish cells, tissues, and organs capable of serving as donors for treating diseases, without eliciting immune rejection reactions, without starting with an egg cell. This object was achieved by providing a pluripotent stem cell having a desired genome. The cell was produced by treating with a reprogramming agent, producing a fusion cell of an MHC deficient stem cell with a somatic cell, or after producing a fusion cell of a stem cell with a somatic cell, removing a gene derived from the stem cell by performing genetic manipulation with a retrovirus.

SEQUENCE LISTING

<110> NAKATSUJI, Norio
TADA, Takashi
ReproCELL Inc.
TADA, Masako

<120> Tailor-made pluripotent stem cells and use thereof

<130> TR004 PCT

<140> PCT/JP02/09732
<141> 2002-09-20

<150> JP 2001-290005
<151> 2001-09-21

<160> 52

<170> PatentIn version 3.1

<210> 1
<211> 20
<212> DNA
<213> Artificial sequence

<220>
<223> primer

<400> 1
ctaggtgagc cgtctttcca

<210> 2
<211> 20
<212> DNA
<213> Artificial sequence

<220>
<223> primer

<400> 2
ttcagggtca gcttgccgta 20

<210> 3
<211> 24
<212> DNA
<213> Artificial sequence

<220>
<223> primer

<400> 3
gtaggcacct gtggggaaga aact 24

<210> 4
<211> 25
<212> DNA
<213> Artificial sequence

<220>
<223> primer

<400> 4
tgagagctgt ctcctactat cgatt 25

<210> 5
<211> 27
<212> DNA
<213> Artificial sequence

<220>
<223> primer

<400> 5
acaagcttca aagcacaatg cctggct 27

<210> 6
<211> 30
<212> DNA
<213> Artificial sequence

<220>
<223> primer

<400> 6
gggtctagac tctcagccgg ctccctcagg 30

<210> 7
<211> 24
<212> DNA
<213> Artificial sequence

<220>
<223> primer

<400> 7
ctcggatcct acttctagct ttct 24

<210> 8
<211> 20
<212> DNA
<213> Artificial sequence

<220>
<223> primer

<400> 8
aaataccttg tgaaaacctg 20

<210> 9
<211> 20
<212> DNA
<213> Artificial sequence

<220>
<223> primer

<400> 9
cagatccttg cagttcatcc 20

<210> 10

<211>  20
<212>  DNA
<213>  Artificial sequence

<220>
<223>  primer

<400>  10
tccacagtca cttgggttcc                                                    20

<210>  11
<211>  23
<212>  DNA
<213>  Artificial sequence

<220>
<223>  primer

<400>  11
tttccctccc ggagattccc taa                                                23

<210>  12
<211>  29
<212>  DNA
<213>  Artificial sequence

<220>
<223>  primer

<400>  12
cctgaggaga cggtgactga ggttccttg                                          29

<210> 13
<211> 25
<212> DNA
<213> Artificial sequence

<220>
<223> primer

<400> 13
aatcgcatta aaaccctccg aacct 25

<210> 14
<211> 24
<212> DNA
<213> Artificial sequence

<220>
<223> primer

<400> 14
tagcacaagt ggaattgtgc tgcg 24

<210> 15
<211> 24
<212> DNA
<213> Artificial sequence

<220>

<223>  primer

<400>  15
gtaggcacct gtggggaaga aact                                              24

<210>  16
<211>  25
<212>  DNA
<213>  Artificial sequence

<220>
<223>  primer

<400>  16
tgagagctgt ctcctactat cgatt                                             25

<210>  17
<211>  27
<212>  DNA
<213>  Artificial sequence

<220>
<223>  primer

<400>  17
acaagcttca aagcacaatg cctggct                                           27

<210>  18
<211>  31
<212>  DNA

<213>  Artificial sequence

<220>
<223>  primer

<400>  18
gggtctagac tctcagccgg ctccctcagg g                                    31

<210>  19
<211>  20
<212>  DNA
<213>  Artificial sequence

<220>
<223>  primer

<400>  19
aaggagtgct gccatggtga                                                 20

<210>  20
<211>  20
<212>  DNA
<213>  Artificial sequence

<220>
<223>  primer

<400>  20
cctaggtttc ttgcagcctc                                                 20

<210>  21
<211>  18
<212>  DNA
<213>  Artificial sequence

<220>
<223>  primer

<400>  21
tcgtattcca acaggagg                                                    18

<210>  22
<211>  20
<212>  DNA
<213>  Artificial sequence

<220>
<223>  primer

<400>  22
cactcttcct tctggagatg                                                  20

<210>  23
<211>  22
<212>  DNA
<213>  Artificial sequence

<220>
<223>  primer

<400>  23

ttggggtcgc tgcggtctag cc                                                22

<210>  24
<211>  22
<212>  DNA
<213>  Artificial sequence

<220>
<223>  primer

<400>  24
ggtcgtctat caggttgtca cg                                                22

<210>  25
<211>  20
<212>  DNA
<213>  Artificial sequence

<220>
<223>  primer

<400>  25
tgtcagagga gcccgaggtc                                                   20

<210>  26
<211>  20
<212>  DNA
<213>  Artificial sequence

<220>

<223>  primer

<400>  26
ccaagagcag cccatcaaag                                                    20

<210>  27
<211>  20
<212>  DNA
<213>  Artificial sequence

<220>
<223>  primer

<400>  27
ggagtgtcgc ttagaggtgc                                                    20

<210>  28
<211>  20
<212>  DNA
<213>  Artificial sequence

<220>
<223>  primer

<400>  28
tccagaaagc caagagaagc                                                    20

<210>  29
<211>  23
<212>  DNA

<213>  Artificial sequence

<220>
<223>  primer

<400>  29
tgaagagagc ggacaaggag atc                                              23

<210>  30
<211>  24
<212>  DNA
<213>  Artificial sequence

<220>
<223>  primer

<400>  30
tctggagtta agaaatcgga gctg                                             24

<210>  31
<211>  24
<212>  DNA
<213>  Artificial sequence

<220>
<223>  primer

<400>  31
gccgagcaga ccaaggaggc catt                                             24

<210>  32
<211>  24
<212>  DNA
<213>  Artificial sequence

<220>
<223>  primer

<400>  32
ctggatggtg tcctggtagc tgct                                              24

<210>  33
<211>  19
<212>  DNA
<213>  Artificial sequence

<220>
<223>  primer

<400>  33
aggacggctc tctgaagaa                                                    19

<210>  34
<211>  20
<212>  DNA
<213>  Artificial sequence

<220>
<223>  primer

<400> 34
ttgaccgagt tgaaggcgaa 20

<210> 35
<211> 26
<212> DNA
<213> Artificial sequence

<220>
<223> primer

<400> 35
tgaaggtcgg tgtgaacgga tttggc 26

<210> 36
<211> 21
<212> DNA
<213> Artificial sequence

<220>
<223> primer

<400> 36
catgtaggcc atgaggtcca c 21

<210> 37
<211> 23
<212> DNA
<213> Artificial sequence

<220>
<223>  primer

<400>  37
gcccgcgctc caactgctct gat                                              23

<210>  38
<211>  22
<212>  DNA
<213>  Artificial sequence

<220>
<223>  primer

<400>  38
cctacggtgg tgcgccctct gc                                               22

<210>  39
<211>  21
<212>  DNA
<213>  Artificial sequence

<220>
<223>  primer

<400>  39
tgccatccgc tacattgaga g                                                21

<210>  40
<211>  22

<212>  DNA
<213>  Artificial sequence

<220>
<223>  primer

<400>  40
ccgggtagca ggctgtgagt tg                                                22

<210>  41
<211>  297
<212>  DNA
<213>  Artificial sequence

<220>
<223>  probe

<400>  41
gcaaagccag gttccttcct tcttccaaat attttcatat tttttttaaa gatttattta      60

ttcattatat gtaagtacac tgtagctgtc ttcagacact ccagaagagg gcgtcagatc     120

ttgttacgta tggttgtgag ccaccatgtg gttgctggga tttgaactcc tgaccttcgg     180

aagagcagtc gggtgctctt atccactgag ccatctcacc agcccctggt ttattttttt     240

aattattatt tgctttttgt ttatcaagac agggtttctc tgcatagctc taattgt        297

<210>  42
<211>  391
<212>  DNA

<213>  Artificial sequence

<220>
<223>  probe

<400>  42
gaattcgatt ggtggcctat ttgctcttat taaaagaaaa agggggagat gttgggagcc      60

gcccccacat tcgccgttac aagatggcgc tgacatcctg tgttctatgt ggtaaacaaa      120

taatctgcgc atgtgccaag ggtatcttat gactacttgt gctctgcctt ccccgtgacg      180

tcaactcggc cgatgggctg cagccaatca gggagtgaca cgtccgaggc gaaggagaat      240

gctccttaag agggacgggg tttcgttctc tctctctctt gcttttctct ctctcttgct      300

tttctctctc tcttgcttct tgctctcttg cttcttgcac tctgttcctg aagatgtaag      360

aataaagctt tgtcgaatca ctagtgaatt c                                     391

<210>  43
<211>  20
<212>  DNA
<213>  Artificial sequence

<220>
<223>  Primer

<400>  43
ctagacgggt gggtaagcaa                                                  20

<210> 44
<211> 20
<212> DNA
<213> Artificial sequence

<220>
<223> primer

<400> 44
caggaggcct tcattttcaa 20

<210> 45
<211> 19
<212> DNA
<213> Artificial sequence

<220>
<223> primer

<400> 45
cgcctcagtt tctcccacc 19

<210> 46
<211> 18
<212> DNA
<213> Artificial sequence

<220>
<223> primer

<400> 46

agccttgacc tctggccc 18

<210> 47
<211> 20
<212> DNA
<213> Artificial sequence

<220>
<223> primer

<400> 47
ctccaaagcc aaaacctgtc 20

<210> 48
<211> 20
<212> DNA
<213> Artificial sequence

<220>
<223> primer

<400> 48
gctgactgga ggtgttccat 20

<210> 49
<211> 20
<212> DNA
<213> Artificial sequence

<220>

<223>  primer

<400>  49
gggtgacaag aggtctggaa                                                    20

<210>  50
<211>  20
<212>  DNA
<213>  Artificial sequence

<220>
<223>  primer

<400>  50
cagcgtgtag ctcatcttgg                                                    20

<210>  51
<211>  20
<212>  DNA
<213>  Artificial sequence

<220>
<223>  primer

<400>  51
cagggaagtt atgggggtct                                                    20

<210>  52
<211>  20

<212>  DNA
<213>  Artificial sequence

<220>
<223>  primer

<400>  52
agaagaacgg gggagaagag                                                    20

## Description

### TECHNICAL FIELD

The present invention relates to a tailor-made pluripotent stem cell suited to an individual. More particularly, the present invention relates to a pluripotent stem cell and use of the same, in which no ES cell is directly used. Specifically, the present invention relates to a method for producing a pluripotent stem cell in which a part or the whole of an embryonic stem cell (hereinafter also referred to as an ES cell)-derived transplantation antigen is deleted, and a method for producing a cell, tissue or organ, comprising differentiating the fusion cell into a cell, tissue or organ in which only the somatic cell-derived major histocompatibility antigen is expressed. In addition, the present invention relates to a pluripotent stem cell and a cell, tissue and organ produced by the method, in which only the somatic cell-derived major histocompatibility antigen is expressed.

### BACKGROUND ART

An embryonic stem (ES) cell is an undifferentiated totipotent cell which is induced from an embryo in an early stage and grows rapidly and has similar properties as those of an embryonic tumor cell. ES cells were first established by culturing an inner cell mass (ICM) of a mouse blastocyst on a feeder cell layer of mouse fibroblasts. ES cells have infinite lifetime under the conditions that undifferentiated states thereof are maintained in the presence of the feeder cell layer and/or leukemia inhibiting factor (LIF) [R.Williams et al., Nature 336:684-687(1988)]. Further, ES cells are known to have a high in vitro differentiating capability and can be differentiated into various types of cells by only culturing as an aggregate mass. ES cells are established from embryos at a stage before implantation and have pluripotency to be differentiated into various cell types derived from 3 germ layers, i.e., ectoderm, mesoderm and endoderm [M. J. Evans and M. H. Kaufman. Nature 292: 154-156 (1981); G. R. Martin, Proc. Natl. Acad. Sci. USA. 78: 7634-7638 (1981)]. More specifically, ES cells are capable of differentiating into any mature cell of an adult, and, for example, ES cells can be differentiated into both somatic cells and germ cells of a chimera animal by being introduced into a normal embryo at an early stage to form a chimeric embryo [R. L. Brinster, J. Exp. Med. 140: 1949-1956 (1974): A. Bradley et al., Nature 309: 255-256 (1984)]. By mating chimera animals having cells derived from the ES cells introduced into germ cells such as testis, ovary, and the like, offspring composed of only cells derived from ES cells can be obtained. This means that animals can be produced with an artificially controllable genetic predisposition. With such an animal, it is possible to research a mechanism of growth and differentiation not only in vitro but also at an individual level. Unlike embryonic tumor cells, many of ES cells are normal cells with the normal diploid karyotype maintained, have high rate of chimera formation, and high probability of differentiation into cells of germ line [A. Bredley et al. , Nature 309: 255-256 (1986)]. Thus, the scope of use of the ES cells is spreading outside the field of embryology.

For example, ES cells are the particularly useful in research on cells and on genes which control cell differentiation. For example, for functional analysis of genes having a known sequence, mouse ES cells have been used for a production of a mouse strain with a disrupted gene, introduced by genetic modification. The use of undifferentiated ES cells may be efficient and effective in functional analysis work after human genome analysis. Since ES cells can be differentiated into a wide variety of cell types in vitro, ES cells have been used for research on cell differentiation mechanisms in embryogenesis. It is becoming possible to induce the ES cells to differentiate into clinically advantageous cells, such as hematopoietic cells, cardiac muscle cells, and neurons of certain types by adding growth factors or forming germ layers [M. Wiles et al., Development 111: 259-267 (1991); W. Miller-Hance et al. , J. Biol. Chem. 268: 25244-25252 (1993); V. A. Maltsev et al., Mech. Dev. 44: 41-50 (1993); G. Bain et al., Dev. Biol. 168: 342-357 (1995)]. Attempts to inducemouse SE cells to differentiate into advantageous cells have succeeded in the production of hematopoietic cells, cardiac muscle cells, specific neurons, and blood vessels [T. Nakano et al., Science 265:1098-1101(1994); R.Pacacios et al., Proc. Natl. Acad. Sci. USA 92: 7530-7534 (1995): V. A. Maltsev et al., Mech. Dev. 44: 41-50 (1993); S. H. Lee et al., Nat. Biotechnol. 18: 675-679 (1999); H. Kawasaki et al., Neuron 28:31-40 (2000); S.-I. Nishikawa, Development 125: 1747-1757 (1998); M.Hirashima et al., Blood 93:1253-1263(1999)].

Currently, ES cells are established for the following animals: hamster [Doetshman T. et al., Dev. Biol. 127:224-227 (1988)], pig [Evans M. J. et al., Theriogenology 33: 125128 (1990): Piedrahita J. A. et al., Theriogenology 34: 879-891 (1990); Notarianni E. et al., J. Reprod. Fert. 40: 51-56 (1990); Talbot N. C. et al., Cell. Dev. Biol. 29A: 546-554 (1993)]; sheep [Notarianni E. et al., J. Reprod. Fert. Suppl. 43: 255-260 (1991)]; bovine [Evans M. J. et al., Theriogenology 33: 125-128(1990); Saito S. et al., Roux. Arch. Dev. Biol. 201: 134-141(1992)]; mink [Sukoyan M. A. et al., Mol. Reorod. Dev. 33: 418-431 (1993)]; rabbit [Japanese National Phase PCT Laid-Open Publication No. 2000-508919]; and primates such as rhesus monkey, marmoset and the like [Thomson J. A. et al., Proc. Natl. Acad. Sci. USA 92: 7844-7848 (1995); Thomson J. A. et al., Biol. Reprod. 55: 254-259 (1996)]. Human ES cells are also established, and they show differentiating capability similar to those of mouse ES cells [J. A. Thomson et al., Science 282: 1145-1147 (1998); J. A. Thomson et al., Dev. Biol. 38: 133-165 (1998); B. E. Reubinoff et al., Nat. Biotechnol. 18: 399-404 (2000)]. It is expected that, by applying the enormous knowledge accumulating for differentiation induction and adjustment achieved by using mouse ES cells, human ES cells will become an infinite material for various cells and/or tissues for transplantation therapy for diseases including myocardial infarct, Parkinson's disease, diabetes, and leukemia and will solve the problem of a shortage of donors for transplantation therapy. In June 23, 2000, three research teams of Australia, the United States, and Germany reported in International Symposium on Stem Cell that they had succeeded in producing neuron and muscle cells from human ES cells for the first time. Further, a recent method for differentiating human ES cells into hematopoietic cell has been developed. However, even in the case where ES cells are used in transplantation therapy, the problem that immune rejection reaction occurs as in existing organ transplantation still remains.

Living tissue transplantation is conducted for various reasons. By organ transplantation, defective functions can be compensated. For example, fetal diseases for important organs, such as kidney, can be cured. Transplantation, which is performed in other sites of the same individual, is called autotrans plantation. Autografts are not rejected. Transplantation between identical twins or incross is called isotransplantation. In this case, the graft is perpetually accepted by the host. Transplantation between the same species is called allotransplantation. In this case, grafts are rejected unless a special treatment is performed for preventing rejection. Transplantation between different species is called heterotransplantation. In this case, grafts are quickly destroyed by the host.

Agents which elicit graft rejection are called transplantation antigens or histocompatibility antigens. All somatic cells other than red blood cells have transplantation antigens. Red blood cells have their own blood type (ABO) antigens. Major human transplantation antigens are called major histocompatibility antigens or HLAs (human leukocyte group A), and are encoded by genes on chromosome 6. HLA antigens are divided into two categories: class I antigens targeted by rejection reactions; and class I I antigens playing a role in initiation of rejection reactions. Class I antigens are present in all tissues, while class II antigens are not present in all tissues and are highly expressed in dendritic cells having finger-like projects, which are macrophage-like cells. An attempt has been made to remove such cells from transplanted tissue so as to prevent the start of a rejection reaction. While there have been some successful experiments, it is not practical and has not been clinically applied.

Rejection reactions occurring after transplantation are divided into categories: hyperacute rejection reactions; accelerated acute rejection reactions; acute rejection reactions; and chronic rejection reactions. Hyperacute rejection reactions occur when there are existing antibodies in the recipient serum, which react with HLA antigens in the donor. Transplant organs are immediately destroyed by intense rejection reactions which occur within several hours when blood vessel ligation is released and blood circulation is resumed into the organ. At present, no therapeutic method is available. To prevent this, a lymphocyte cross test is performedbefore transplantation. When it is confirmed that the recipient serum has antibodies reacting with donor's lymphocytes, transplantation is given up for prophylaxis. Accelerated hyper rejection reactions occur when T lymphocytes reactive to donor's HLA antigens exist in the recipient body before transplantation. Accelerated hyper rejection reactions usually occur within 7 days after transplantation and are as intense as hyperacute rejection reactions. Recent progress in therapeutic drugs is making it possible to cure such rejection reactions. Acute rejection reactions are caused as a result of cellular immune reactions elicited mainly by T lymphocytes associated with the donor's HLA antigens of a transplant organ. Acute rejection reactions are most often observed and are typically recognized about 2 weeks to 1 month after transplantation. Chronic rejection reactions are characterized by a reduction in organ function gradually proceeding against clinical therapies, and occur 6 months to 1 year after transplantation. Basically, it is considered that recipient's immune reactions activated by invation of donor's HLA antigens elicits tissue disorders in a transplant organ, and reactions thereto of the organ tissue proceed tissue degeneration over a long period of time. Unless an organ having the same MHC molecule structure as that of the recipient is transplanted, rejection reactions unavoidably occur. At present, the lack of means for control rejection reactions is a significant problem.

Examples of immunosuppresion techniques for preventing rejection reactions include use of immunosuppressants, surgeries, irradiation, and the like. Examples of immunosuppressants mainly include adrenocorticosteroid, cyclosporine, FK506, and the like. Adrenocorticosteroid reduces the number of circulatory T cells to inhibit the nucleic acid metabolism of lymphocytes and production of cytokines and suppress T cell functions. Thereby, the migration and metabolism of macrophages are inhibited, resulting in suppressing of immune reactions. Cyclosporine and FK506 have similar actions, binding to receptors on the surface of helper T cells, entering the cells, and acting directly on DNA to inhibit the production of interleukin 2. Eventually, the function of killer T cells is impaired, resulting in immunosuppression. Use of these immunosuppressants raises adverse side effects. Particularly, steroids often cause side effects. Cyclosporine is toxic to the liver and kidneys. FK506 is toxic to the kidneys. Examples of surgeries include extraction of lymph node, extraction of spleen, and extraction of thymus, whose effect has not been fully demonstrated. Among surgeries, thoracic duct drainage is to remove circulating lymphocytes from the thoracic duct and its effect has been confirmed. However, this technique causes the loss of a large amount of serum protein and lipid, leading to nutrition disorders. Irradiation includes whole body irradiation and graft irradiation. Its effect is not reliable and the impact on recipients is great. Therefore, irradiation is used in combination with the above-described immunosuppressant. Clearly, none of the above-described techniques is ideal for prevention of rejection reactions.

It is now known that, by introducing a somatic cell nucleus into enucleated egg cells, the somatic cell nucleus is reprogrammed to be totipotent in mammal. In this way, clone sheep, bovine, mouse, pig and the like have been produced [Wilmut I. et al., Nature 385:810-813(1997); Kato Y. et al., Science 282: 2095-2098 (1998); Wakayama T. et al., Nature 394: 369-374 (1998); Onishi A. et al., Science 289: 1188-1190 (2000); Polejaeva I. A. et al., Nature 407: 86-90 (2000)]. By utilizing this technique, it is considered that it is possible to reprogram the nucleus of a somatic cell derived from a host which is to receive a transplant by using egg cells and producing a totipotent cell to produce a transplantation graft which does not cause immune rejection reaction. Further, with such a method of cell culturing, shortage of donors can be overcome.

However, cloning for treating humans encounters social problems, i.e., biomedical ethical problems (Weissman, I.L., N. Engl. J. Med., 346, 1576-1579 (2002)). The above-described techniques require egg cells, which is problematic from an ethical viewpoint. For humans, ES cells are derived from undifferentiated cells of early embryos, and no adult early embryo exists. Accordingly, in principle, it is not possible to establish ES cells after the stage of early embryos, particularly from adult hosts. Therefore, no pluripotent stem cell suited to an individual has been obtained. There is a serious demand for such a cell in the art.

### (Problems to be Solved by the Invention)

An object of the present invention is to provide an easily obtained pluripotent stem cell suitied to an individual. More particularly, an object of the present invention is to efficiently establish a cell, tissue and organ which elicit no immune rejection reaction and may be used as donor tissue for treatment of diseases, without extracting stem cells, such as ES cells, or the like, and without using egg cells as material.

### DISCLOSURE OF THE INVENTION

The present inventors succeeded in producing stem cells, which have a desired genome derived from an individual, such as a subject individual targeted by therapeutic treatment, elicit a reduced level of immune rejection reaction, and have pluripotency. Thereby, the above-described problems were solved.

Initially, the present inventors produced a tetraploid somatic cell by fusing a stem cell (e.g., an ES cell) with a somatic cell and revealed that the cell could be grown in vivo and in vitro, and the somatic cell nucleus was reprogrammed and had pluripotency. According to the present invention, in such a tetraploid somatic cell, an agent derived from the stem cell (e.g., an ES cell) which elicits an immune rejection reaction in a host, i.e. , a stem cell (e.g., an ES cell) which does not express a part or the whole of the stem cell-derived transplantation antigen, is utilized in production of pluripotent stem cells suited to individuals.

The pluripotent stem cell suited to an individual, which does not express a part or the whole of the stem cell (e.g., an ES cell)-derived transplantation antigen, can be achieved by, for example, fusing a stem cell (e.g., an ES cell) in which a part or the whole of the transplantation antigen (particularly, major histocompatibility antigens) is deleted, with a somatic cell. In this case, the stem cell - derived transplantation antigen was reduced or removed from the pluripotent stem cells suited to individuals. Thereby, the transplantation rejection reaction could be significantly reduced.

The pluripotent stem cell suited to an individual, which does not express a part or the whole of the stem cell (e.g., an ES cell)-derived transplantation antigen, can be achieved by, for example, fusing a stem cell (e.g., an ES cell) with a somatic cell, followed by removal of the stem cell (e.g., an ES cell)-derived genome using genetic manipulation. In this case, the stem cell-derived genome could be completely removed from the fusion cell, and the "complete" pluripotent stem cell suited to an individual free from a rejection reaction could be obtained.

Further, a stem cell (e.g., an ES cell)-derived reprogramming agent was unexpectedly identified. The reprogramming agent was used to confer pluripotency to a cell (e.g., a somatic cell) having a desired genome, thereby succeeding in producing a pluripotent stem cell. In this case, the "complete" pluripotent stem cell suited to an individual free from a rejection reaction, which has no gene other than the desired genome, could be obtained.

When acell, tissue andorgan, which is differentiated from a pluripotent stem cell having a desired genome, such as a fusion cell, a reprogrammed somatic cell, or the like, is introduced into a recipient, the rejection reaction of the recipient is reduced as compared to those differentiated from a cell having all of the stem cell (e.g., an ES cell)-derived transplantation antigens, or completely removed. Thus, the pluripotent stem cell of the present invention may be an ideal material for establishing a cell, tissue and organ which is a donor for treatment of diseases. These cells, tissues and organs have a wide variety of applications in tailor-made medical therapies and are highly industrially useful.

The present invention specifically provides the following.
1. An isolated pluripotent stem cell, comprising a desired genome.
2. A pluripotent stem cell according to item 1, which is a non-ES cell.
3. A pluripotent stem cell according to item 1, wherein at least a part of a transplantation antigen is deleted.
4. A pluripotent stem cell according to item 1, wherein the whole of a transplantation antigen is deleted.
5. A pluripotent stem cell according to item 1, wherein the transplantation antigen comprises at least a major histocompatibility antigen.
6. A pluripotent stem cell according to item 3, wherein the major histocompatibility antigen comprises a class I antigen.
7. A pluripotent stem cell according to item 1, wherein the genome is reprogrammed.
8. A pluripotent stem cell according to item 1, which is produced by reprogramming a cell.
9. A pluripotent stem cell according to item 8, wherein the cell is a somatic cell.
10. A pluripotent stem cell according to item 1, which is produced by fusing a stem cell and a somatic cell.
11. A pluripotent stem cell according to item 10, wherein the stem cell is an ES cell.
12. A pluripotent stem cell according to item 10, wherein the stem cell is a tissue stem cell.
13. A pluripotent stem cell according to item 1, which has a genome derived from a desired individual and is not an ES cell or an egg cell of the desired individual.
14. A pluripotent stem cell according to item 1, which has a chromosome derived from a somatic cell of a desired individual.
15. A pluripotent stem cell according to item 1, which is not directly derived from an embryo.
16. A pluripotent stem cell according to item 1, which is derived from a somatic cell.
17. A pluripotent stem cell according to item 1, wherein a transplantation antigen other than that of a desired individual is reduced.
18. A pluripotent stem cell according to item 1, which is derived from a cell other than an egg cell of a desired individual.
19. A pluripotent stem cell according to item 1, wherein the desired genome is of an individual in a state other than the early embryo.
20. A pluripotent stem cell according to item 1, which is an undifferentiated somatic cell fusion cell of an ES cell and a somatic cell, wherein a part or the whole of a transplantation antigen is deleted in the ES cell.
21. A pluripotent stem cell according to item 1, which is an undifferentiated somatic cell fusion cell of an ES cell and a somatic cell, wherein the whole of a transplantation antigen is deleted in the ES cell.
22. A pluripotent stem cell according to item 20, wherein the transplantation antigen is a major histocompatibility antigen.
23. A pluripotent stem cell according to item 22, wherein the major histocompatibility antigen is a class I antigen.
24. A pluripotent stem cell according to item 20, wherein the somatic cell is a lymphocyte, a spleen cell or a testis-derived cell derived from a transplantation individual.
25. A pluripotent stem cell according to item 20, wherein at least one of the ES cell and the somatic cell is a human-derived cell.
26. A pluripotent stem cell according to item 20, wherein the somatic cell is a human-derived cell.
27. A pluripotent stem cell according to item 20, wherein at least one of the somatic cell and the stem cell is genetically modified.
28. A method for producing a pluripotent stem cell having a desired genome, comprising the steps of:
   1) deleting a part or the whole of a transplantation antigen in the stem cell; and
   2) fusing the stem cell with a somatic cell having the desired genome.
29. A method according to item 28, wherein the stem cell is an ES cell.
30. A method according to item 28, wherein the ES cell is an established ES cell.
31. A method according to item 28, wherein the transplantation antigen is a major histocompatibility antigen.
32. A method according to item 31, wherein the major histocompatibility antigen is a class I antigen.
33. A method according to item 28, wherein the somatic cell is a lymphocyte, a spleen cell or a testis-derived cell derived from a transplantation individual.
34. A method according to item 28, wherein at least one of the stem cell and the somatic cell is a human-derived cell.
35. A method according to item 28, comprising deleting the whole of the transplantation antigen.
36. A method for producing a pluripotent stem cell having a desired genome, comprising the steps of:
   1) providing a cell having the desired genome; and
   2) exposing the cell to a composition comprising a reprogramming agent.
37. A method according to item 36, wherein the cell is a somatic cell.
38. A method according to item 36, wherein the reprogramming agent is prepared with at least one agent selected from the group consisting of a cell cycle regulatory agent, a DNA helicase, a histone acetylating agent, and a transcription agent directly or indirectly involved in methylation of histone H3 Lys4.
39. A cell, tissue or organ, which is differentiated from a pluripotent stem cell having a desired genome.
40. A cell according to item 39, wherein the cell is amyocyte, a chondrocyte, an epithelial cell, or a neuron.
41. A tissue according to item 39, wherein the tissue is muscle, cartilage, enpithelium, or nerve.
42. An organ according to item 39, wherein the organ is selected from the group consisting of brain, spinal cord, heart, liver, kidney, stomach, intestine, and pancreas.
43. A cell, tissue or organ according to item 39, wherein the cell, tissue or organ is used for transplantation.
44. A cell, tissue or organ according to item 39, wherein the desired genome is substantially the same as the genome of a host to which the cell, tissue or organ is transplanted.
45. A medicament, comprising a cell, tissue or organ having a desired genome, wherein the cell, tissue or organ is differentiated from a pluripotent stem cell.
46. A medicament for treatment or prophylaxis of a disease or disorder due to a defect in a cell, tissue or organ of a subject, comprising a pluripotent stem cell having substantially the same genome as that of the subject.
47. A method for treatment or prophylaxis of a disease or disorder due to a defect in a cell, tissue or organ of a subject, comprising the steps of:
   preparing a pluripotent stem cell having substantially the same genome as that of the subject;
   differentiating the cell, tissue or organ from the pluripotent stem cell; and
   administering the cell, tissue or organ into the subject.
48. A method for treatment or prophylaxis of a disease or disorder due to a defect in a cell, tissue or organ of a subject, comprising the step of:
   administering a pluripotent stem cell having substantially the same genome as that of the subject, into the subject.
49. A method for treatment or prophylaxis of a disease or disorder due to a defect in a cell, tissue or organ of a subject, comprising the step of:
   administering, into the subject, a medicament comprising a cell, tissue or organ differentiated from a pluripotent stem cell having substantially the same genome as that of the subject.
50. Use of a pluripotent stem cell for producing a medicament for treatment or prophylaxis of a disease or disorder due to a defect in a cell, tissue or organ of a subject, wherein the medicament comprises the pluripotent stem cell having substantially the same genome as that of the subject.
51. Use of a pluripotent stem cell for producing a medicament for treatment or prophylaxis of a disease or disorder due to a defect in a cell, tissue or organ of a subject, wherein the medicament comprises the cell, tissue or organ differentiated from the pluripotent stem cell having substantially the same genome as that of the subject.
52. Use of a pluripotent stem cell comprising a desired genome for producing a medicament comprising the pluripotent stem cell.
53. Use of a pluripotent stem cell having a desired genome for producing a medicament comprising a cell, tissue or organ differentiated from the pluripotent stem cell.
54. A reprogramming agent, which is selected from the group consisting of an enzyme methylating histone H3-Lys4 or an agent involved in methylation of histone H3-Lys4, a cell cycle agent, DNA helicase, a histone acetylating agent, and a transcription agent.
55. A reprogramming agent according to item 54, wherein the agent is a transcription agent Sp1 or Sp3, or a cofactor thereof.

### BRIEF DESCRIPTION OF THE DRAWINGS

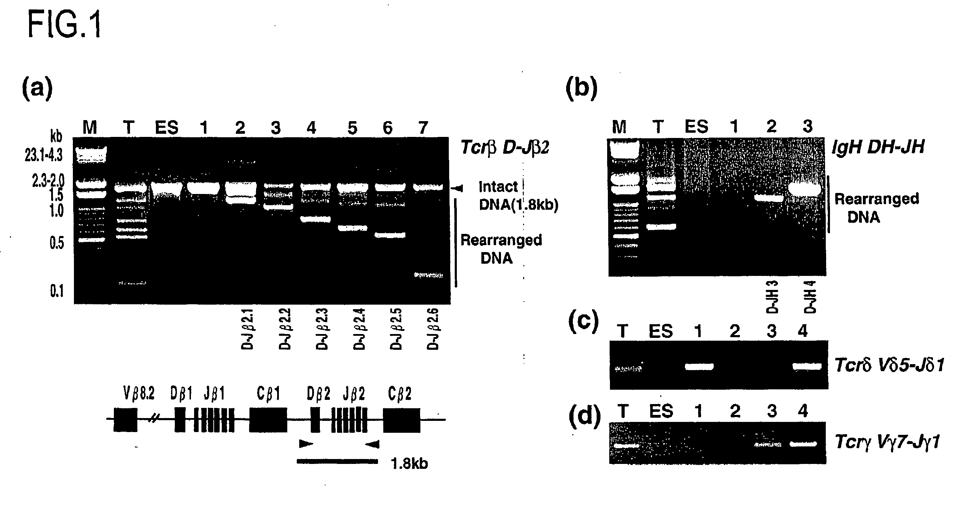

Figure **1** shows pictures showing the result of PCR analysis demonstrating DNA rearrangement of Tcrβ, Tcrδ, Torγ and IgH genes derived from thymocyte in ES fusion cells. Pictures (**a**) to (**d**) respectively show the results of PCR analysis using primer sets specific to the following regions: (**a**) D-J region of Tcrβ; (**b**) D-J region of IgH; (**c**) V-J region of Tcrδ; and (**d**) V-J region of Torγ. DNA samples used are as follows: T; derived from thymocytes from a (Rosa26×Oct4-GFP) F1 mouse, ES; derived from ES cells , M; marker mixture of λ/HindIII DNA and an 100bp ladder DNA, 1 to 7; derived from ES hybrid clones.

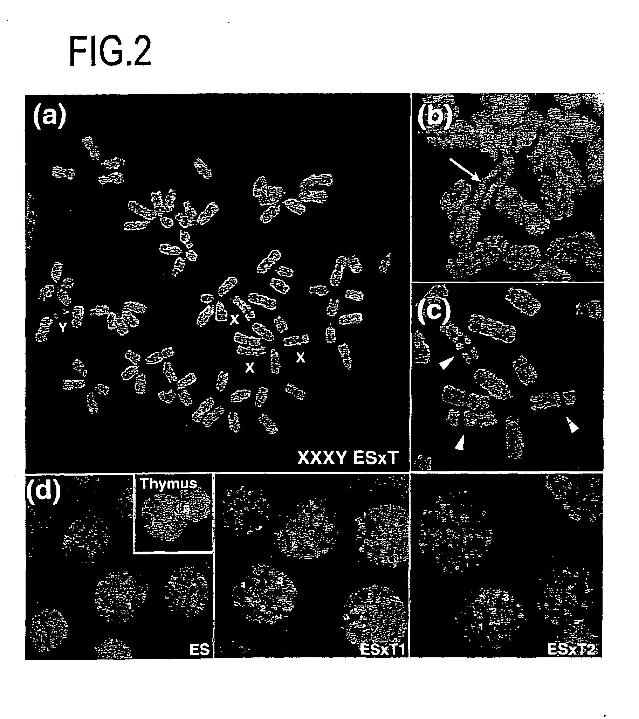

Figure 2 shows pictures showing reactivation of the X chromosome derived from thymocyte in ES fusion cells. (**a**) Results of R differential staining analysis at the time of replication of the X chromosome in ES fusion cells. In ES fusion cells, three X chromosomes (two X chromosomes from the female derived thymocyte, and one X chromosome from the male derived ES cell) are detected to be red and green, and are shown to be active. In (**a**), three X chromosomes replicated at the same time, and are shown enlarged in (**c**) (arrows). X chromosomes in female somatic cells (shown by arrows in (**b**)), and Y chromosome (in the middle of (**a**)) are uniformly stained red, and shown to be inactive. (**d**) Xist RNA is detected as spots of red signals on active X chromosome of male ES cell, while inactive X chromosome of female thymocyte is stained entirely giving a large red signal. In two ES hybrid cell lines(ESX T1 and ESX T2) examined, three spot red signals were detected for each nucleus.

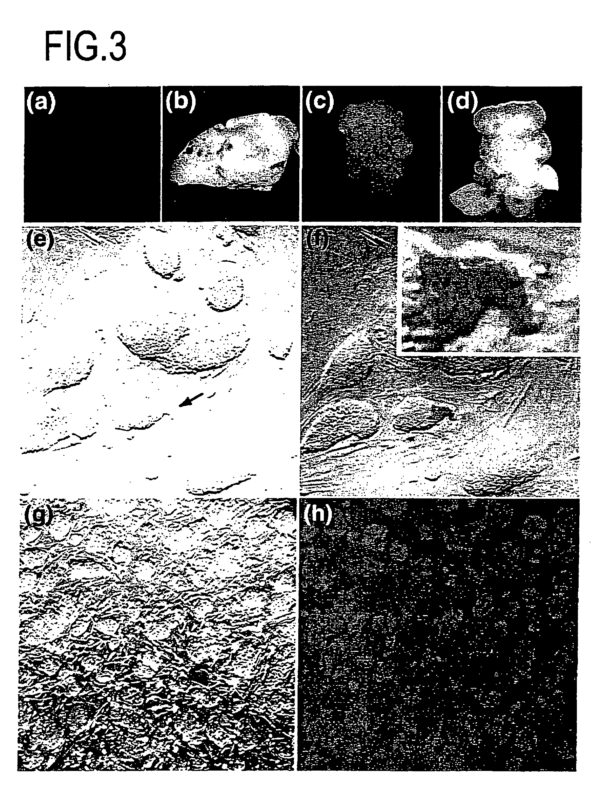

Figure **3** shows photomicrographs showing reactivation of ES fusion cells. GFP fluorescence images and bright field images are shown of thymus (**a, b**), and ovary (**c**, **d**) of (Rosa26 × Oct4-GFP) F1 mouse used for production of ES fusion cells. (**e**) Bright field images of colonies two days after fusion with the arrow indicating a GFP-positive colony as shown in (**f**). (**f**) GFP fluorescence image two days after fusion. The small GFP-positive colony amongst non-expressed ES cell colony. The picture of the positive colony is shown enlarged in an upper portion. (**g**) Bright-field image of (**h**). (**h**) Picture of a GFP-positive cell expanded from the colony after selection in G418.

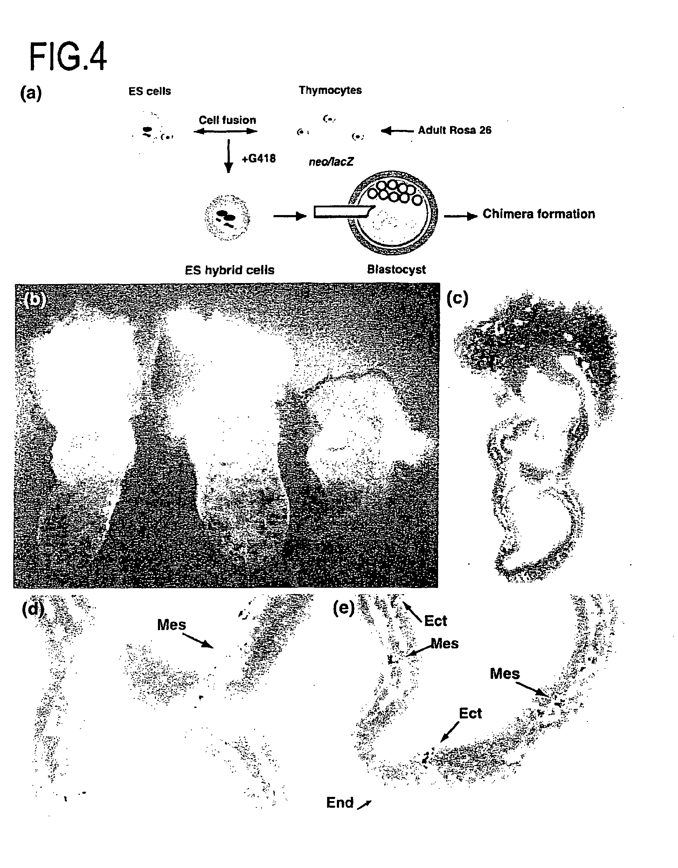

Figure **4** shows a diagram and pictures showing the development capability of ES fusion cells in vivo. (**a**) Schematic view showing a method of producing ES fusion cells and chimeric embryos. (**b**) Picture showing results of β-galactosidase active staining of E7.5 chimeric embryos having ES fusion cells. The cells derived from fusion cells are shown blue. (**c**) Picture showing results of histological analysis of a section of a E7.5 chimeric embryo which is sectioned along a longitudinal axis. (**d, e**) pictures showing chimeric embryo sections at higher magnification. Ect; ectoderm, Mes; mesoderm, and End; endoderm.

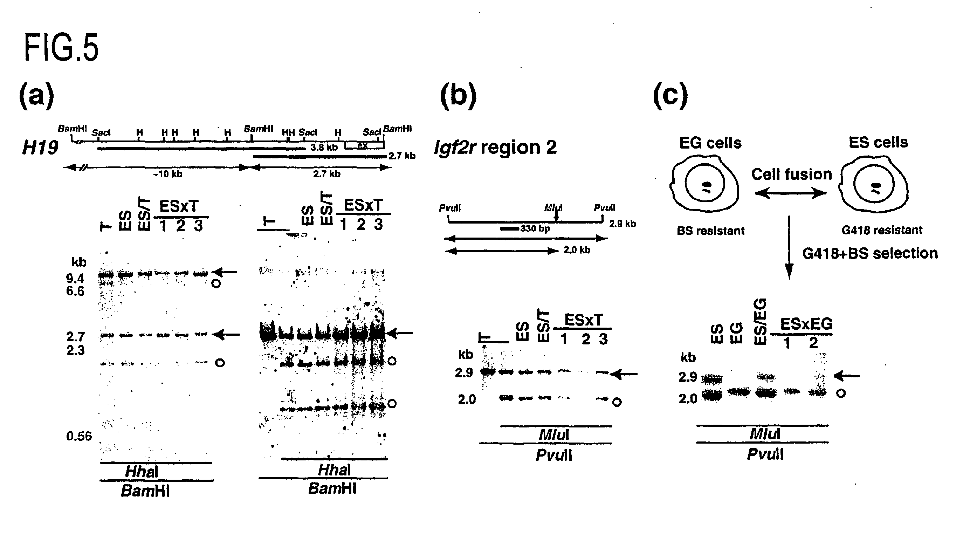

Figure **5** shows diagrams related to analysis of methylation of H19 genes and Igf2r genes in ES hybrid and ES × EG fusion cells and pictures showing analysis results. (**a**): Analysis results for H19 gene. (**b**) and (**c**): Analysis results for Igf2r gene. Arrows indicate methylated DNA fragments, and ○ represent unmethylated DNA fragments. A summary of experimentation methods is illustrated in (**c**). Abbreviations are as follows: T; thymocyte, ES/T; mixture of ES and thymocyte DNA at 1:1, ES/EG; mixture of ES and EG DNA at 1:1, ESX T; ES hybrid clone of ES cell and Rosa 26 thymocyte.

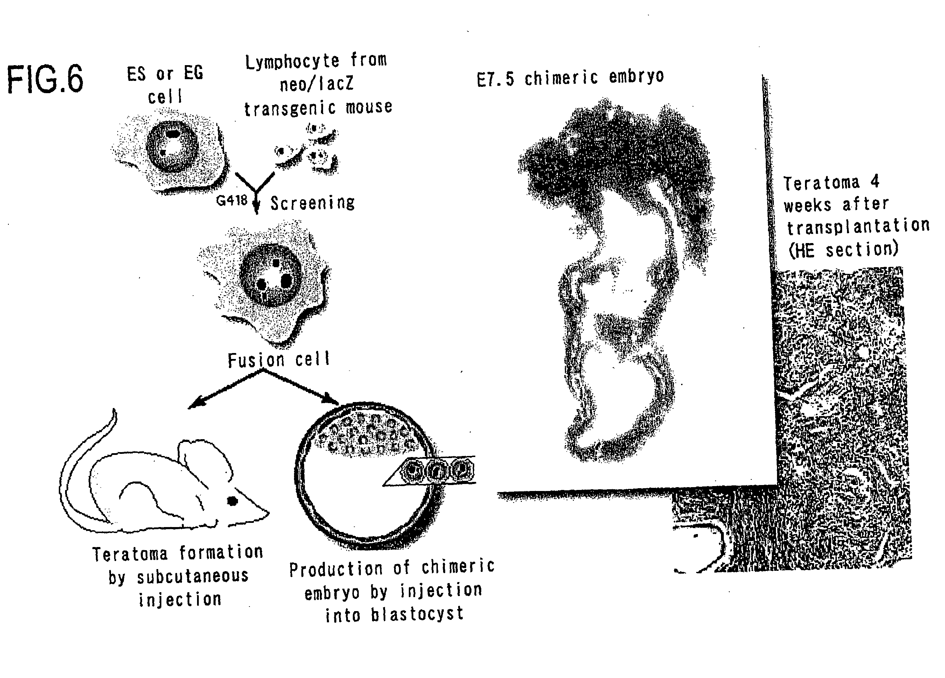

Figure **6** shows schematic views of teratoma formation and production of chimeric embryos and photomicrographs of chimeric embryos and teratoma.

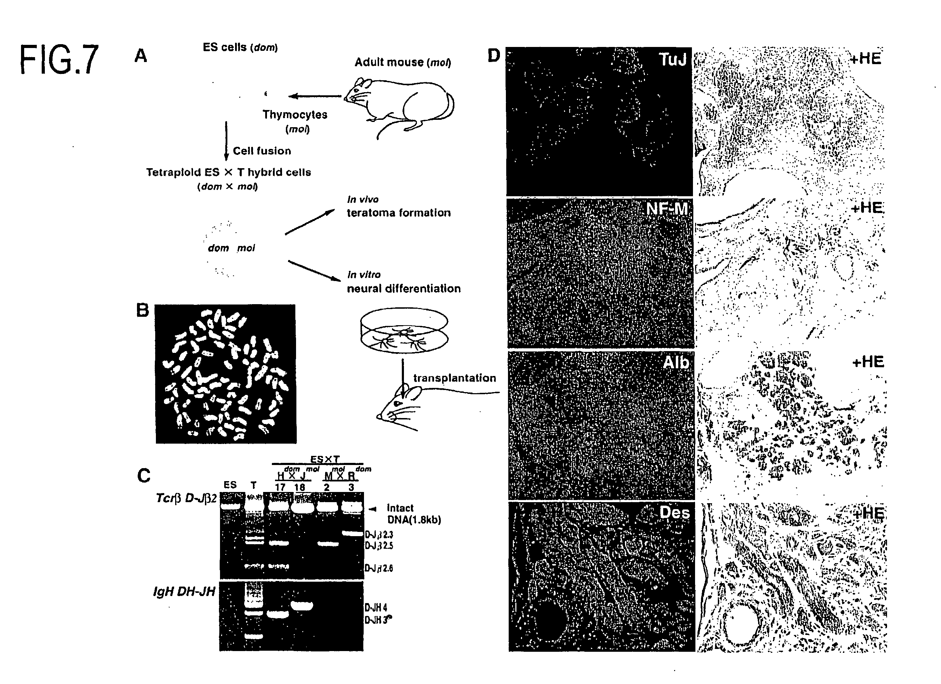

Figure **7** is a diagram showing characterization of fusion cells between ES cells and adult lymphocytes, and their pluripotency. (**A**) experimental scheme of production and differentiation of inter-subspecific fusion cells between domesticus (dom) ES cells and molossinus (mol) thymocytes; (**B**) a representative metaphase spread of a tetraploid fusion cell clone, HxJ-18; (**C**) genomic PCR analysis of the D-J DNA rearrangements of the Tcrβ and IgH genes; an (**D**) expression of the ectodermal, mesodermal and endodermal tissue-specific marker proteins, Class III β-Tublin (TuJ), Neurofilament-M (NF-M), Albumin (Alb) and Desmin (Des) in paraffin sections of fusion cell-derived teratomas. The sections were counter-stained with hemotoxylin and eosin (HE).

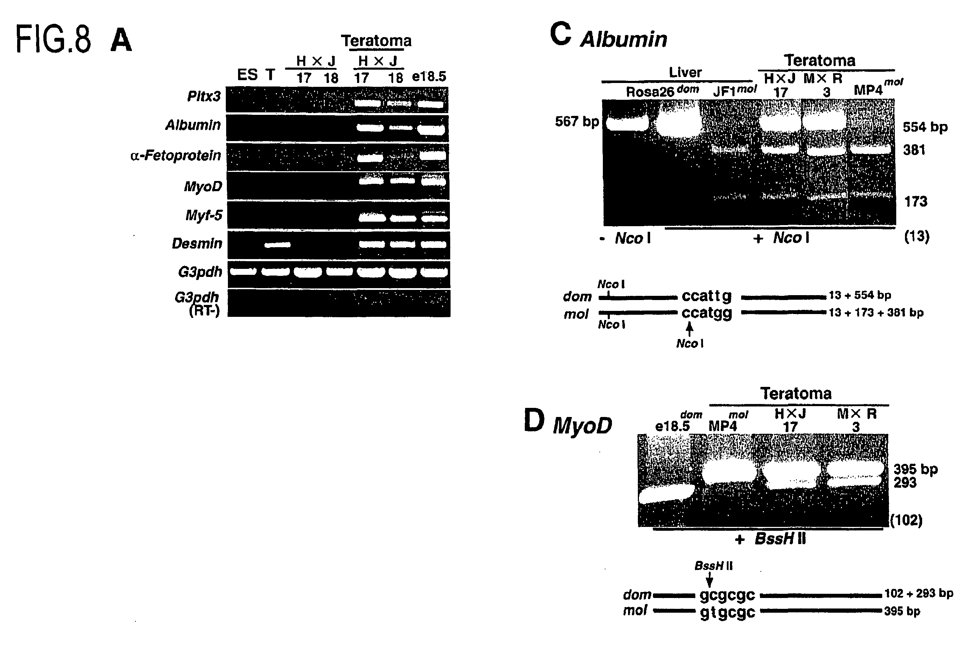

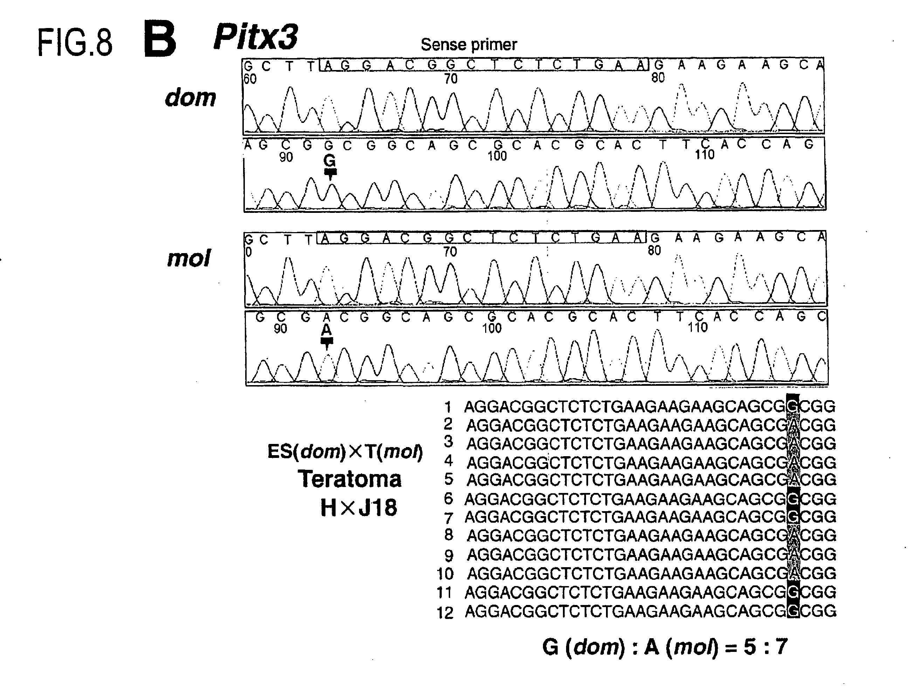

Figure **8** shows somatic genome-specific RT-PCR products in the ectodermal, mesodermal and endodermal derivatives of fusion cells. (**A**) Ectodermal Pitx3, mesodermal MyoD, Myf-5 and Desmin and endodermal Albumin and α-Fetoprotein in the undifferentiated and differentiated HxJ-17 and 18 fusion clones. An e18.5 embryo is control. (**B**) Ectodermal Pitx3 transcripts from reprogrammed somatic genomes. The guanine residue of mRNA in the domesticus (dom) ES genomes is replaced to the adenine residue in the molossinus (mol) somatic genomes. (**C**) Endodermal Albumin transcripts from reprogrammed somatic genomes. The domesticus type RT-PCR products have a single NcoI digestion site, while the molossinus type products have two NcoI sites. (**D**)Mesodermal MyoD transcripts from reprogrammed somatic genomes. The domesticus type RT-PCR products is sensitive to the BssHI digestion, whereas the molossinus type products is BssHI-insensitive.

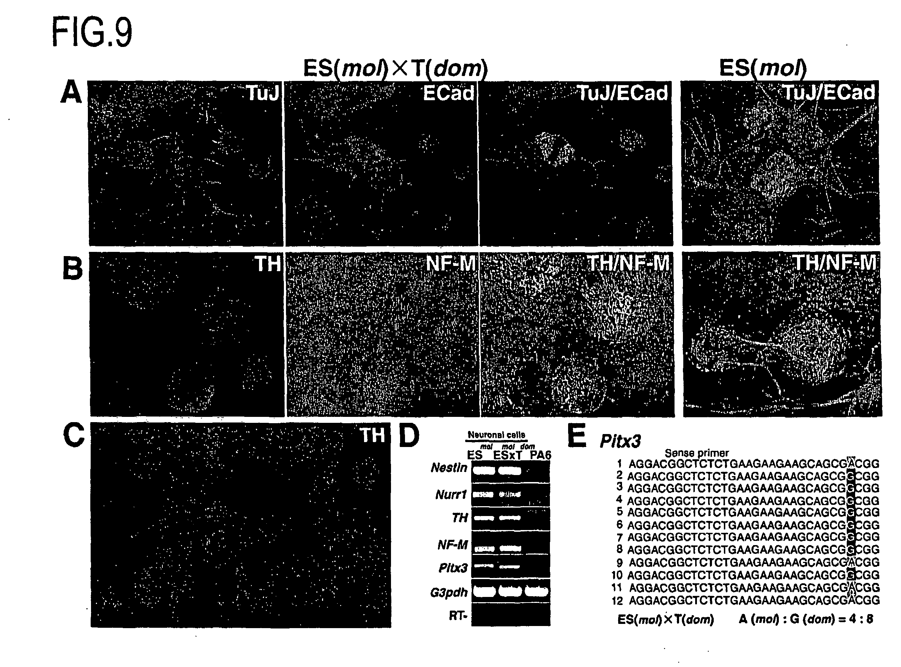

Figure **9** shows neural differentiation induction of fusion cells on PA6 feeder cells in vitro. (**A**) Neural cells differentiated from host molossinus MP4 ES cells as control. TuJ (red); a post-mitotic neuron-specific marker protein and Ecad (green); a stem cell-specific maker. (**B**) Neural cells differentiated from the MxR-3 fusion cells. Most colonies are positively immunoreacted with TH antibody specific to the mesencephalic dopaminergic neurons (red) and NF-M (green) which is a neural cell marker. (**C**) Effective and reproducible differentiation induction to TH-positive neurons. (**D**) Expression of neural cell-specific genes in neural cells differentiated in vitro from fusion cells 11 days after induction: Nestin (neuroepithelial stem cell-specific marker) and NF-M (post-mitotic neuron-specific marker). Expression of dopaminergic neuron-specific markers, Nurr1, TH and Pitx3 are transcribed in fusion cell derivatives after neural differentiation induction. No signal in control PA6. (**E**) Expression of Pitx3 (transcriptional activator of TH) from reprogrammed somatic genomes. Conversion of the guanine residue in the domesticus (dom) genomes to the adenine residue in the molossinus (mol) genomes.

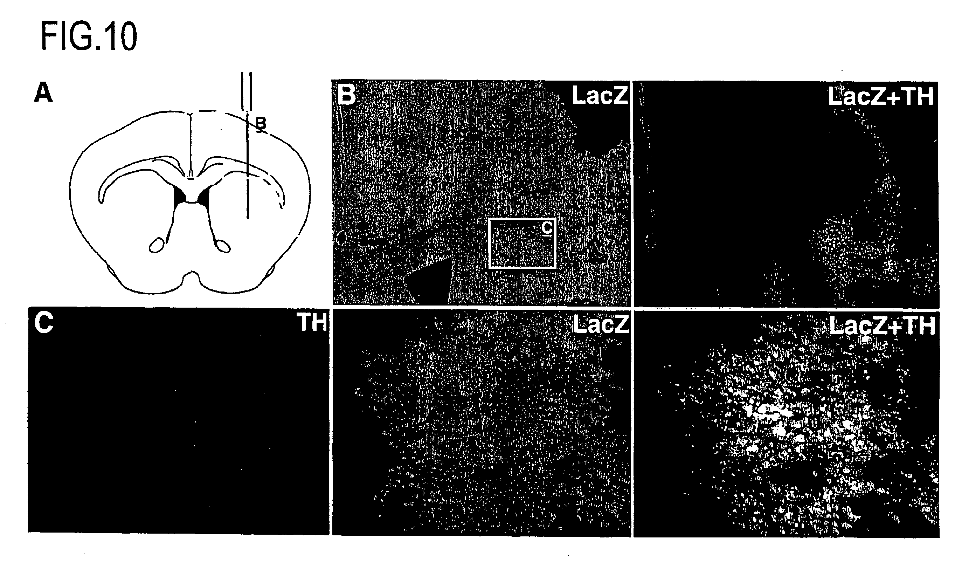

Figure **10** shows a graft of fusion cell-derived TH-positive neurons in mouse brain. (**A**) Transplantation of the MxR-3 fusion cell-derived neural cells characterized in Figures **3B, C, D** and **E** into the striatum of mouse brain. (**B**) Fusion cell-derived neurons expressing TH in mouse brain. MxR-3 fusion cell derived neural cells carrying the lacZ/neo reporter gene are positively detected by LacZ antibody (green). Double staining with lacZ and TH antibodies shows that the fusion cell derived neurons express TH (red). In a merged image, lacZ and TH double positive cells are visualized as yellow cells. (**C**) High magnification images in the area (**C**) in (**B**). LacZ-positive fusion cell-derivatives (green) express TH (red) in the injection site. In a merged image, LacZ and TH double positive cells are visualized as yellow cells.

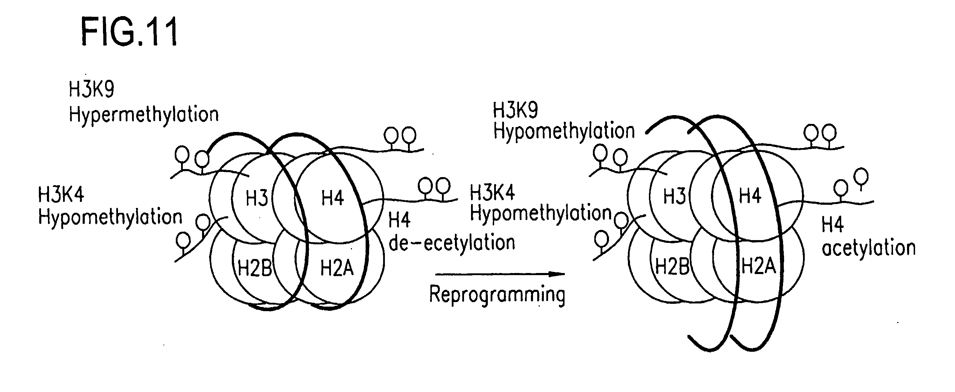

Figure **11** shows a schematic diagram of reprogramming.

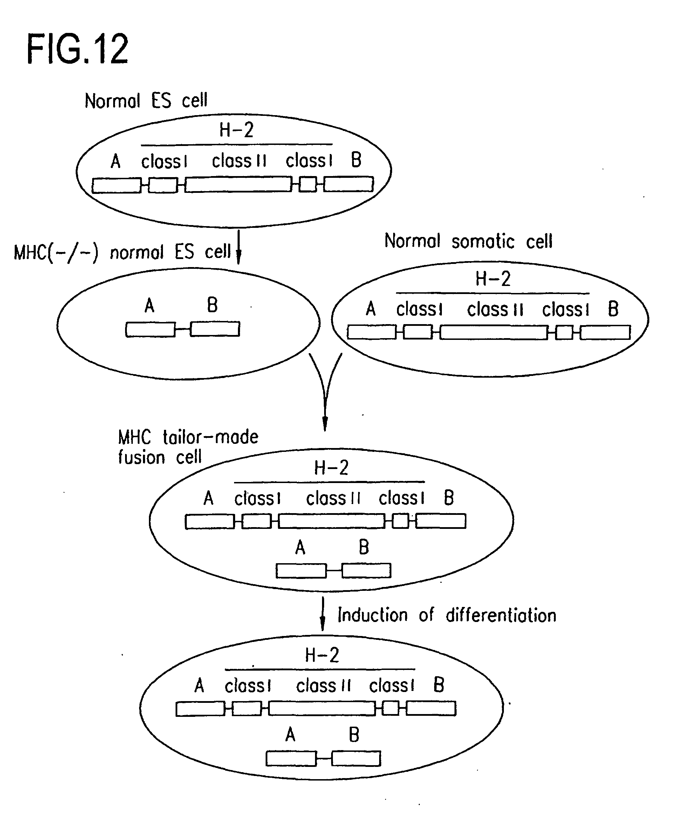

Figure **12** shows a schematic diagram of production of MHC deficient ES cell-somatic cell fusion cells.

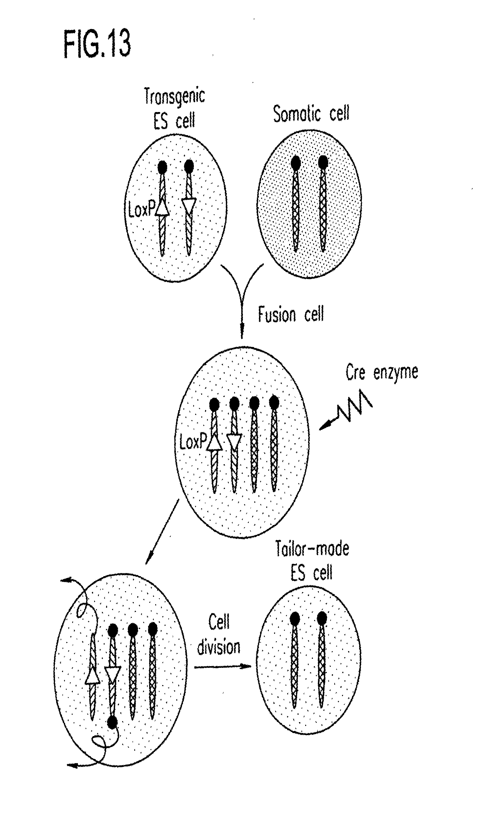

Figure **13** shows a schematic diagram of production of genome deficient ES cell-somatic cell fusion cells.

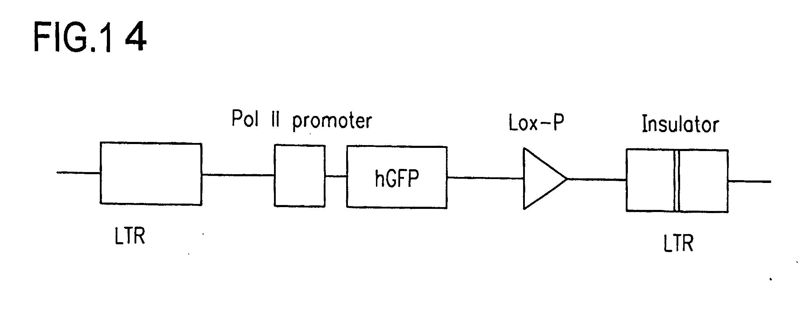

Figure **14** shows a structure of Insulator-Polymerase II promoter-GFP-LoxP-Insulator used in the schematic diagram of Figure **13**.

### BEST MODE FOR CARRYING OUT THE INVENTION

Hereinafter, the present invention will be described. It should be understood throughout the present specification that articles for singular forms (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include plural referents unless the context clearly dictates otherwise. It should be also understood that the terms as used herein have definitions typically used in the art unless otherwise mentioned.

### (Description of terms)

Terms used herein are defined as follows.

The term "cell" is herein used in its broadest sense in the art and refers to a living body which is a structural unit of tissue of a multicellular organism, is surrounded by a membrane structure which isolates it from the outside, has the capablity of self replicating, and has genetic information and a mechanism for expressing it. Cells used herein may be naturally-occurring cells or artificially modified cells (e.g., fusion cells, genetically modified cells, etc.).

As used herein, the term "stem cell" refers to a cell capable of self replication and pluripotency. Typically, stem cells can regenerate an injured tissue. Stem cells used herein may be, but are not limited to, embryonic stem (ES) cells or tissue stem cells (also called tissue-specific stem cell, or somatic stem cell). Any artificially produced cell which can have the above-described abilities (e.g., fusion cells, reprogrammed cells, or the like used herein) may be a stem cell. ES cells are pluripotent stem cells derived from early embryos. An ES cell was first established in 1981, which has also been applied to production of knockout mice since 1989. In 1998, a human ES cell was established, which is currently becoming available for regenerative medicine. Tissue stem cells have a limited level of differentiation unlike ES cells. Tissue stem cells are present at particular locations in tissues and have an undifferentiated intracellular structure. Therefore, the pluripotency of tissue stem cells is low. Tissue stem cells have a higher nucleus/cytoplasm ratio and have few intracellular organelles. Most tissue stem cells have pluripotency, a long cell cycle, and proliferative ability beyond the life of the individual. As used herein, stem cells may be preferably ES cells, though tissue stem cells may also be employed depending on the circumstance.

Tissue stem cells are separated into categories of sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, liver stem cells, and the like. Tissue stem cells in the bone marrow system include hematopoietic stem cells, mesenchymal stem cells, and the like. Tissue stem cells in the nervous system include neural stem cells, retinal stem cells, and the like.

As used herein, the term "somatic cell" refers to any cell other than germ cells, such as an egg, a sperm, or the like, which does not directly transfer its DNA to the next generation. Typically, somatic cells have limited or no pluripotency. Somatic mcells used herein may be naturally-occurring or genetically modified.

The origin of a cell is categorized into a stem cell derived from the ectoderm, endoderm, or mesoderm. Stem cells of ectodermal origin are mostly present in brain, including neural stem cells. Stem cells of endodermal origin are mostly present in bone marrow, including blood vessel stem cells, hematopoietic stem cells, mesenchymal stem cells, and the like. Stem cells of mesoderm origin are mostly present in organs, including liver stem cells, pancreatic stem cells, and the like. Somatic cells may be herein derived from any germ layer. Preferably, somatic cells, such as lymphocytes, spleen cells or testis-derived cells, may be used.

As used herein, the term "isolated" means that materials naturally accompanying in normal circumstances are at least reduced, or preferably substantially completely eliminated. Therefore, the term "isolated cell" refers to a cell substantially free from other accompanying in natural circumstances substances (e.g., other cells, proteins, nucleic acids, etc.). The term "isolated" in relation to nucleic acids or polypeptides means that, for example, the nucleic acids or the polypeptides are substantially free from cellular substances or culture media when they are produced by recombinant DNA techniques; or precursory chemical substances or other chemical substances when they are chemically synthesized. Isolated nucleic acids are preferably free from sequences naturally flanking the nucleic acid within an organism from which the nucleic acid is derived (i.e., sequences positioned at the 5' terminus and the 3' terminus of the nucleic acid).

As used herein, the term "established" in relation to cells refers to a state of a cell in which a particular property (e.g., pluripotency) of the cell is maintained and the cell undergoes stable proliferation under culture conditions. Therefore, established stem cells maintain pluripotency. In the present invention, the use of established stem cells is preferable since the step of collecting stem cells from a host can be avoided.

As used herein, the term "non-embryonic" refers to not being directly derived from early embryos. Therefore, the term "non-embryonic" refers to cells derived from parts of the body other than early embryos. Also, modified ES cells (e.g., genetically modified or fusion ES cells, etc.) are encompassed by non-embryonic cells.

As used herein, the term "differentiated cell" refers to a cell having a specialized function and form (e.g., myocytes, neurons, etc.). Unlike stem cells, differentiated cells have no or little pluripotency. Examples of differentiated cells include epidermic cells, pancreatic parenchymal cells, pancreatic duct cells, hepatic cells, blood cells, cardiac myocytes, skeletal myocytes, osteoblasts, skeletal myoblasts, neurons, vascular endothelial cells, pigment cells, smooth myocytes, fat cells, bone cells, chondrocytes, and the like. Therefore, in one embodiment of the present invention, a given differentiated cell which can be conferred pluripotency may be used as or instead of a somatic cell in the present invention.

As used herein, the terms "differentiation" or "cell differentiation" refers to a phenomenon that two or more types of cells having qualitative differences in form and/or function occur in a daughter cell population derived from the division of a single cell. Therefore, "differentiation" includes a process during which a population (family tree) of cells, which do not originally have a specific detectable feature, acquire a feature, such as production of a specific protein, or the like. At present, cell differentiation is generally considered to be a state of a cell in which a specific group of genes in the genome are expressed. Cell differentiation can be identified by searching for intracellular or extracellular agents or conditions which elicit the above-described state of gene expression. Differentiated cells are stable in principle. Particularly, animal cells which have been once differentiated are rarely differentiated into other types of cells. Therefore, the acquired pluripotent cells of the present invention are considerably useful.

As used herein, the term "pluripotency" refers to a nature of a cell, i.e., an ability to differentiate into one or more, preferably two or more, tissues or organs. Therefore, the terms "pluripotent" and "undifferentiated" are herein used interchangeably unless otherwise mentioned. Typically, the pluripotency of a cell is limited as the cell is developed, and in an adult, cells constituting a tissue or organ rarely alter to different cells, where the pluripotency is usually lost. Particularly, epithelial cells resist altering to other types of epithelial cells. Such alteration typically occurs in pathological conditions, and is called metaplasia. However, mesenchymal cells tend to easily undergo metaplasia, i.e., alter to other mesenchymal cells, with relatively simple stimuli. Therefore, mesenchymal cells have a high level of pluripotency. ES cells have pluripotency. Tissue stem cells have pluripotency. As used herein, the term "totipotency" refers to the pluripotency of a cell, such as a fertilized egg, to differentiate into all cells constituting an organism. Thus, the term "pluripotency" may include the concept of totipotency. An example of an in vitro assay for determining whether or not a cell has pluripotency, includes, but is not limited to, culture under conditions for inducing the formation and differentiation of embryoid bodies. Examples of an in vivo assay for determining the presence or absence of pluripotency, include, but are not limited to, implantation of a cell into an immunodeficient mouse so as to form teratoma, injection of a cell into a blastocyst so as to form a chimeric embryo, implantation of a cell into a tissue of an organism (e.g., injection of a cell into ascites) so as to undergo proliferation, and the like.

Cells used in the present invention include cells derived from any organisms (e.g., any multicellular organisms (e.g., animals (e.g., vertebrates, invertebrate), plants (monocotyledons, dicotyledons, etc.))). Preferably, the animal is a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). More preferably, Primates (e.g., chimpanzee, Japanese macaque, human, etc.) are used. Most preferably, a human is used.

Any organ may be targeted by the present invention. A tissue or cell targeted by the present invention may be derived from any organ. As used herein, the term "organ" refers to a morphologically independent structure localized at a particular portion of an individual organism in which a certain function is performed. In multicellular organisms (e.g., animals, plants), an organ consists of several tissues spatially arranged in a particular manner, each tissue being composed of a number of cells. An example of such an organ includes an organ relating to the vascular system. In one embodiment, organs targeted by the present invention include, but are not limited to, skin, blood vessel, cornea, kidney, heart, liver, umbilical cord, intestine, nerve, lung, placenta, pancreas, brain, peripheral limbs, retina, and the like. Examples of cells differentiated from pluripotent cells include epidermic cells, pancreatic parenchymal cells, pancreatic duct cells, hepatic cells, blood cells, cardiac myocytes, skeletal myocytes, osteoblasts, skeletal myoblasts, neurons, vascular endothelial cells, pigment cells,smooth myocytes,fatcells, bone cells, chondrocytes, and the like.

As used herein, the term "tissue" refers to an aggregate of cells having substantially the same function and/or form in a multicellular organism. "Tissue" is typically an aggregate of cells of the same origin, but may be an aggregate of cells of different origins as long as the cells have the same function and/or form. Therefore, when stem cells of the present invention are used to regenerate tissue, the tissue may be composed of an aggregate of cells of two or more different origins. Typically, a tissue constitutes a part of an organ. Animal tissues are separated into epithelial tissue, connective tissue, muscular tissue, nervous tissue, and the like, on a morphological, functional, or developmental basis. Plant tissues are roughly separated into meristematic tissue and permanent tissue according to the developmental stage of the cells constituting the tissue. Alternatively, tissues may be separated into single tissues and composite tissues according to the type of cells constituting the tissue. Thus, tissues are separated into various categories.

As used herein, the term "protein", "polypeptide" and "peptide" are used interchangeably and refer to a macromolecule consisting of a series of amino acids.

As used herein, the term "amino acid" may refer to a naturally-occurring or non-naturally-occurring amino acid. As used herein, the term "amino acid derivative" or "amino acid analog" refers to an amino acid which is different from a naturally-occurring amino acid and has a function similar to that of the original amino acid. Such amino acid derivatives and amino acid analogs are well known in the art. The term "naturally-occurring amino acid" refers to an L-isomer of a naturally-occurring amino acid. The naturally-occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, y-carboxyglutamic acid, arginine, ornithine, and lysine. Unless otherwise indicated, all amino acids as used herein are L-isomers. The term "non-naturally-occurring amino acid" refers to an amino acid which is ordinarily not found in nature. Examples of non-naturally-occurring amino acids include norleucine, para-nitrophenylalanine, homophenylalanine, para-fluorophenylalanine, 3-amino-2-benzyl propionic acid, D- or L-homoarginine, and D-phenylalanine. As used herein, the term "amino acid analog" refers to a molecule having a physical property and/or function similar to that of amino acids, but is not an amino acid. Examples of amino acid analogs include, for example, ethionine, canavanine, 2-methylglutamine, and the like. An amino acid mimic refers to a compound which has a structure different from that of the general chemical structure of amino acids but which functions in a manner similar to that of naturally-occurring amino acids.

Molecular biological techniques, biochemical techniques, and microorganism techniques as used herein are well known in the art and commonly used, and are described in, for example, Maniatis, T. et al. (1989), Molecular Cloning: A Laboratory Manual, Cold Spring Harbor and its 3rd Ed. (2001); Ausubel, F.M. (1987), Current Protocols in Molecular Biology, Greene Pub. Associates and Wiley-interscience; Ausubel, F.M. (1989), Short Protocols in Molecular Biology: A Compendium of Methods from Current Protocols in Molecular Biology, Greene Pub. Associates and Wiley-interscience; Sambrook, J. et al. (1989), Molecular Cloning: A Laboratory Manual, Cold Spring Harbor; Innis, M.A. (1990), PCR Protocols: A Guide to Methods and Applications, Academic Press; Ausubel, F.M. (1992), Short Protocols in Molecular Biology: A Compendium of Methods from Current Protocols in Molecular Biology, Greene Pub. Associates; Ausubel, F.M. (1995), Short Protocols in Molecular Biology: A Compendium of Methods from Current Protocols in Molecular Biology, GreenePub. Associates; Innis, M.A. et al. (1995), PCR Strategies, Academic Press; Ausubel, F.M. (1999), Short Protocols in Molecular Biology: A Compendium of Methods from Current Protocols in Molecular Biology, Wiley, and annual updates; Sninsky, J.J. et al. (1999), PCR Applications: Protocols for Functional Genomics, Academic Press, Special issue, Jikken igaku [Experimental Medicine] "Idenshi Donyu & Hatsugenkaiseki Jikkenho [Experimental Method for Gene introduction & Expression Analysis]", Yodo-sha, 1997; and the like. Relevant portions (or possibly the entirety) of each of these publications are herein incorporated by reference.

As usedherein, the term "biological activity" refers to activity possessed by an agent (e.g., a polynucleotide, a protein, etc.) within an organism, including activities exhibiting various functions. For example, when a certain agent is an enzyme, the biological activity thereof includes its enzyme activity. When a certain agent is a reprogramming agent, the biological activity thereof includes its reprogramming activity.

As used herein, the term "variant" refers to a substance, such as a polypeptide, a polynucleotide, or the like, which differs partially from the original substance. Examples of such a variant include a substitution variant, an addition variant, a deletion variant, a truncated variant, an allelic variant, and the like. The term "allele" as used herein refers to a genetic variant located at a locus identical to a corresponding gene, where the two genes are distinguished from each other. Therefore, the term "allelic variant" as used herein refers to a variant which has an allelic relationship with a given gene. The term "species homolog" as used herein refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. Cells used in the present invention may contain a modified nucleic acid or polypeptide.

Any method for introducing DNA into cells can be used as an vector introduction method, including, for example, transfection, transduction, transformation, and the like (e.g., an electroporation method, a particle gun (gene gun) method, and the like).

When a gene is mentioned herein, the term "vector" or "recombinant vector" refers to a vector capable of transferring a polynucleotide sequence of interest to a target cell. Such a vector is capable of self-replication or incorporation into a chromosome in a host cell (e.g., a prokaryotic cell, yeast, an animal cell, a plant cell, an insect cell, an individual animal, and an individual plant, etc.), and contains a promoter at a site suitable for transcription of a polynucleotide of the present invention.

Examples of a "recombinant vector" for prokaryotic cells include pBTrp2, pBTac1, pBTac2 (all commercially available from Roche Molecular Biochemicals), pKK233-2 (Pharmacia), pSE280 (Invitrogen), pGEMEX-1 [Promega], pQE-8 (QIAGEN), pKYP10 (Japanese Laid-Open Publication No. 58-110600), pKYP200 [Agric. Biol. Chem. , 48, 669(1984)], pLSA1 [Agric. Biol. Chem., 53, 277(1989)], pGEL1 [Proc. Natl. Acad. Sci. USA, 82, 4306(1985)], pBluescript II SK+ (Stratagene), pBluescript II SK(-) (Stratagene), pTrs30 (FERM BP-5407), pTrs32 (PERM BP-5408), pGHA2 (FERM BP-400), pGKA2 (FERM B-6798), pTerm2 (Japanese Laid-Open Publication No. 3-22979, US4686191, US4939094, US5160735), pEG400 [J. Bacteriol., 172, 2392(1990)], pGEX (Pharmacia), pETsystem (Novagen), pSupex, pUB110, pTP5, pC194, pTrxFus (Invitrogen), pMAL-c2 (New England Biolabs), pUC19 [Gene, 33, 103(1985)], pSTV28 (Takara), pUC118 (Takara), pPA1(Japanese Laid-Open Publication No. 63-233798), and the like.

Examples of a "recombinant vector" for yeast cells include YEp13 (ATCC37115), YEp24 (ATCC37051), YCp50 (ATCC37419), pHS19, pHS15, and the like.

Examples of a "recombinant vector" for animal cells include pcDNAI/Amp, pcDNAI, pCDM8 (all commercially available from Funakoshi), pAGE107 [Japanese Laid-Open Publication No. 3-229 (Invitrogen), pAGE103 [J. Biochem., 101, 1307(1987)], pAMo, pAMoA [J. Biol. Chem., 268, 22782-22787(1993)], retroviral expression vectors based on murine stem cell viruses (MSCV), and the like.

A "retrovirus vector" used in the present invention includes, for example, without limitation, retroviral expression vectors based on Moloney Murine Leukemia Virus (MMLV) or Murine Stem Cell Virus (MSCV), and the like.

Examples of a "recombinant vector" for plant cells include Tiplasmid, tobacco mosaic virus vector, and the like.

Examples of a "recombinant vector" for insect cells include pVL1392, pVL1393, pBlueBacIII (all available from Invitrogen), and the like.

As used herein, the term "transformant" refers to the whole or a part of an organism, such as a cell, which is produced by transformation. Examples of a transformant include a prokaryotic cell, yeast, an animal cell, a plant cell, an insect cell, and the like. Transformants may be referred to as transformed cells, transformed tissue, transformed hosts, or the like, depending on the subject. A cell used herein may be a transformant.

When a prokaryotic cell is used herein for genetic operations or the like, the prokaryotic cell may be of, for example, genus Escherichia, genus Serratia, genus Bacillus, genus Brevibacterium, genus Corynebacterium, genus Microbacterium, genus Pseudomonas, or the like, including, for example, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Escherichia coli BL21(DE3), Escherichia coli BL21(DE3)pLysS, Escherichia coli HMS174(DE3), Escherichia coli HMS174(DE3)pLysS, Serratia ficaria, Serratia fonticola, Serratia liquefaciens, Serratia marcescens, Bacillus subtilis, Bacillus amyloliquefaciens, Brevibacterium ammmoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, Pseudomonas sp. D-0110, and the like.

Examples of an animal cell as used herein include a mouse myeloma cell, a rat myeloma cell, a mouse hybridoma cell, a Chinese hamster overy (CHO) cell, a BHK cell, an African green monkey kidney cell, a human leukemic cell, HBT5637 (Japanese Laid-Open Publication No. 63-299), ahuman colon cancer cell line, and the like. The mouse myeloma cell includes ps20, NSO, and the like. The rat myeloma cell includes YB2/0 and the like. A human embryo kidney cell includes HEK293 (ATCC: CRL-1573) and the like. The human leukemic cell includes BALL-1 and the like. The African green monkey kidney cell includes COS-1, COS-7, and the like. The human colon cancer cell line includes HCT-15, and the like.

Examples of plant cells as used herein, include cells of plants, such as potato, tobacco, maize, rice, crucifer, soybean, tomato, carrot, wheat, barley, rye, alfalfa, flax, and the like. Any recombinant vector introduction method which can introduce DNA into plant cells can be used, for example, an Agrobacterium method (Japanese Laid-Open Publication No. 59-140885, Japanese Laid-Open Publication No. 60-70080, W094/00977), electroporation (Japanese Laid-Open Publication No. 60-251887), a method using a particle gun (gene gun) (Japanese Patent No. 2606856, Japanese Patent No. 2517813), and the like.

Examples of insect cells as used herein include ovary cells of Spodoptera frugiperda, ovary cells of Trichoplusia ni, cultured cells derived from silkworm ovary, and the like. Examples of ovary cells of Spodoptera frugiperda include Sf9, Sf21 (Baculovirus Expression Vectors: A Laboratory Manual), and the like. Examples of ovary cells of Trichoplusia ni include High 5, BTI-TN-5B1-4 (Invitrogen), and the like. Examples of silkworm ovary-derived cultured cells include Bombyx mori N4, and the like.

Any method for introduction of DNA can be used herein as a method for introduction of a recombinant vector, including, for example, a calcium chloride method, an electroporation method (Methods. Enzymol., 194, 182 (1990)), a lipofection method, a spheroplast method (Proc.Natl.Acad.Sci.USA.84,1929(1978)), a lithium acetate method (J.Bacteriol.,153,163(1983)), a method described in Proc. Natl. Acad. Sci. USA, 75, 1929 (1978), and the like.

A retrovirus infection method as used herein is well known in the art as described in, for example, Current Protocols in Molecular Biology (supra) (particularly, Units 9.9-9.14), and the like. Specifically, for example, ES cells are trypsinized into a single-cell suspension, followed by co-culture with the culture supernatant of virus-producing cells (packaging cell lines) for 1-2 hours, thereby obtaining a sufficient amount of infected cells.

The transient expression of Cre enzyme, DNA mapping on a chromosome, and the like, which are used herein in a method for removing a genome, a gene locus, or the like, are well known in the art, as described in Kenichi Matsubara and Hiroshi Yoshikawa, editors, Saibo-Kogaku [Cell Engineering], special issue, Jikken Purotokoru Shirizu [Experiment Protocol Series], "FISH Jikken Purotokoru Hito · Genomu Kaiseki kara Senshokutai · Idenshishindan made [FISH Experiment Protocol From Human Genome Analysis to Chrmosome/Gene diagnosis]", Shujun-sha (Tokyo), and the like.

Gene expression (e.g., mRNA expression, polypeptide expression) may be "detected" or "quantified" by an appropriate method, including mRNA measurement and immunological measurement method. Examples of the molecular biological measurement method include a Northern blotting method, a dot blotting method, a PCR method, and the like. Examples of the immunological measurement method include an ELISA method, an RIA method, a fluorescent antibody method, a Western blotting method, an immunohistological staining method, and the like, where a microtiter plate may be used. Examples of a quantification method include an ELISA method, an RIA method, and the like.

As used herein, the term "expression level" refers to the amount of a polypeptide or mRNA expressed in a subject cell. The expression level includes the expression level at the protein level of a polypeptide of the present invention evaluated by any appropriate method using an antibody of the present invention, including immunological measurement methods (e.g., an ELISA method, an RIA method, a fluorescent antibody method, a Western blotting method, an immunohistological staining method, and the like, or the expression level at the mRNA level of a polypeptide of the present invention evaluated by any appropriate method, including molecular biological measurement methods (e.g., a Northern blotting method, a dot blotting method, a PCR method, and the like). The term "change in expression level" indicates that an increase or decrease in the expression level at the protein or mRNA level of a polypeptide of the present invention evaluated by an appropriate method including the above-described immunological measurement method or molecular biological measurement method.

As used herein, the term "transplantation antigen" refers to an antigenic substance which may introduce transplantation immune into specific individuals when undifferentiated somatic cellfusion cells,or cells,tissues or organs differentiated from the fusion cell, or the like are introduced into the specific individuals. Most transplantation antigens are expressed as codominant traits on cell membranes. Transplantation antigens can be roughly divided into two categories: major histocompatibility antigens (MHC) which are antigens presenting molecules capable of eliciting strong rejection reactions; and minor histocompatibility antigens which elicit chronic weak rejection reactions. Major histocompatibility complex antigens are allogenic antigens which elicit strong rejection reactions in allotransplantaion of organs, tissues and cells. Genes coding major histocompatibility antigens constitute a complex comprising a number of genetic loci, which has a high degree of polymorphism, and is called major histocompatibility complex (MHC). In humans, MHC is a human lymphocyte antigen (HLA) on the short arm of chromosome 6. In mice, MHC is an H-2 gene complex on chromosome 17. A single MHC is known to be present in all mammals and birds. In addition to humans and mice, rhesus monkey RhL-A, dog DLA, rat RT1, and the like are known. HLA antigens (human MHC) are divided into class I antigens which are expressed in all nucleated cells; and Class II antigens which are expressed in antigen presenting cells, such as macrophages, B cells, activated T cells, dendric cells, thymus epithelial cells, and the like. Class I antigens present intracellular antigens and are recognized by CD8 positive cytotoxic T cell receptors (CD8⁺ TCR). Class II antigens present foreign antigens and are recognized by CD4 positive helper T cell receptors (CD4⁺ TCR). On the other hand, the genetic loci of minor histcompatibility antigens are single genetic loci (minor histocompatibility loci (MIH)) and their polymorphism is low.

In the present invention, it is possible to produce pluripotent stem cells suited to transplant individuals by deleting a transplantation antigen in stem cells (e.g., ES cells) which are used for production of the pluripotent stem cells. The transplantation antigen to be deleted may include a part or the whole of the above-described major histocompatibility antigens and minor histocompatibility antigens. The degree of deletion is not particularly limited as long as when a pluripotent stem cell produced using the deleted stem cell (e. g. , ES cell), or a cell, tissue or organ differentiated from the pluripotent stem cell, is used for transplantation, the degree of rejection reactions in the recipient is reduced as compared to when no deletion is provided. Among other things, major histocompatibility antigens are preferable transplantation antigens to be deleted in the present invention, and particularly preferably class I antigens. A transplantation antigen can be deleted by, for example, deleting a gene encoding the transplantation antigen. A representative technique for deleting a gene is gene targeting utilizing homologous recombination [Mansour S.L. et al. , Nature, 336:348-352 (1988); Capecchi M.R., TIG, 5:70-76 (1989); Valancius and Smithies, Mol. Cell. Biol., 11:1402-1408 (1991); Hasty et al. , Nature, 350 (6351) 243-246 (1991)], and the like.

Class I and class II MHC antigens are heterodimers each consisting of 2 subunits α and β. A pluripotent stem cell of the present invention suited to a transplantation individual is, for example, a cell in which at least one copy, preferably both copies, of the subunits of an MHC antigen derived from a stem cell (e.g., an ES cell) have been inactivated. A subunit to be inactivated is one that is not compensated for by a somatic cell-derived subunit after a stem cell (e.g., an ES cell) in which the subunit is inactivated, is fused with the somatic cell. In other words, a subunit in which a transplantation antigen derived from a stem cell (e.g., an ES cell) is not expressed in the fusion cell has to be selected.

The inactivation may be achieved by deleting a gene encoding a subunit of a stem cell (e.g., an ES cell)-derived MHC antigen, or other genes having an influence on expression of an MHC antigen. Examples of genes having an influence on expression of MHC antigens include genes regulating expression of MHC antigens, such as, for example, the TAP1, TAP2, LMP2 and LMP7 genes in the Class II genetic loci regulating presentation depending on MHC antigens, and the like. For example, when a fusion cell lacking a stem cell (e.g., an ES cell)-derived MHC antigen is produced by gene targeting, a part of a gene encoding the stem cell (e.g., an ES cell)-derived MHC antigen is subjected to homologous recombination to construct a targeting vector having a deletion or disruption. The resultant vector is introduced into fusion cells by an appropriate technique, such as electroporation, calcium precipitation DNA, fusion, transfection, lipofection, or the like. Techniques for transforming mammalian cells have been reported in, for example, Keown et al. [Methods in Enzymology 185:527-537(1990)]. Screening for transformed cells can be achieved by, for example, introducing a selectable marker, such as neo, puro, or the like, which is typically used in gene targeting, into a deficient region of a gene of interest, and selecting only gene targeting cells using a pharmaceutical agent specific to the selectable marker. When the expression of a selectable marker gene, such as neo, puro, or the like, is considered to have a problem in future gene analysis or clinical applications, for example, such a selectable marker gene may be sandwiched Lox-P sequences and the Cre gene is introduced into the gene targeting cell so that the Cre enzyme is temporarily expressed. Thereby, the selectable marker gene can be removed in a cell engineering manner. Such a technique is well known in the art, which is described herein elsewhere and in the documents mentioned herein. The MHC antigen is expressed in the differentiated cell. Therefore, screening can be performed based on the absence of the target MHC antigen on the surface of the transformed cell. As a screening method, for example, a monoclonal antibody for any epitope of a target MHC antigen can be utilized with Complement, to kill cells having the antigen. Alternatively, a conjugate of an appropriate antibody, a lysine A chain, abrin, diphtheria toxin, and the like, can be used to kill cells having the antigen. More simply, affinity chromatography may be used to remove cells having a target antigen. For the resultant cells, at least one ES cell-derived MHC antigen is removed from the cell surface. When such a cell or a cell, tissue or organ induced from the cell is introduced into organsms, the cell is less likely to be immunologically rejected since there is less stem cell (e.g., an ES cell)-derived MHC antigen as compared to the original fusion cell.

As used herein, the term "reprogramming" means that a cell (e.g., a somatic cell) is caused to be in the undifferentiated state so that the cell increases or acquires pluripotency. Therefore, reprogramming activity may be measured as follows, for example. A differentiated cell (e.g., a somatic cell, etc.) is exposed to a predetermined amount of a certain agent for a predetermined period of time (e.g., several hours, etc.). Thereafter, the pluripotency of the cell is measured and compared with the pluripotency of the cell before exposure. By determining whether or not a significant difference is found, the reprogramming activity is determined. There are various reprogrammed levels, which correspond to the pluripotency levels of a reprogrammed cell. Therefore, when a reprogramming agent derived from a totipotent stem cell is used, reprogramming may correspond to imparting totipotency.

As used herein, the term "reprogramming agent" refers to an agent which acts on cells to cause the cells to be in the undifferentiated state. As indicated in the examples below, ES cells cannot reprogram imprints in the nuclei of somatic cells, and can reprogram the epigenetic state of the nuclei of somatic cells so that germ cells can be developed. Therefore, it is clear that ES cells have an agent capable of reprogramming. There is also a possibility that stem cells other than ES cells possess an agent capable of reprogramming somatic cells. Such a reprogramming agent is also encompassed by the present invention. Examples of an ES cell-derived component which is applied to somatic cells include, but are not limited to, components contained in ES cells, including cytoplasmic components, nuclear components, individual RNAs and proteins, and the like. When cytoplasmic or nuclear components including miscellaneous molecules are applied, the components may be fractioned to some degree with a commonly used technique (e.g., chromatography, etc.), and each fraction may be applied to somatic cells. If a specific fraction is revealed to contain a reprogramming agent, the fraction can be further purified so that a single molecule is eventually specified and such a molecule can be used. Alternatively, a fraction containing a reprogramming agent can be used without any purification to reprogram somatic cells. It may be considered that a single molecule achieves reprogramming. Alternatively, it may be considered that a plurality of molecules interact one another to alter somatic cells into the undifferentiated state. Therefore, the "reprogramming agent" of the present invention includes an agent consisting of a single molecule, an agent consisting of a plurality of molecules, and a composition comprising the single molecule or the plurality of molecules.

A reprogramming agent of the present invention can be selected as follows. Components derived from ES cells are caused to act on somatic cells by means of contact, injection, or the like. The action is detected based on the expression of the Oct4-GFP marker gene, the activation of the X chromosome, or the like, as an indicator for reprogramming. A component having reprogramming activity is selected.

A "reprogramming agent contained in an ES cell" of the present invention can be obtained by a screening method as described above. The reprogramming agent may be an enzyme for methylation of histone H3-Lys4 or an agent which is involved in the methylation. There is a possibility that such a component is contained in cells (e.g., tissue stem cells, etc.) other than ES cells. However, once a reprogramming agent is identified from an ES cell by the above-described method, such a reprogramming agent can be obtained or produced from other materials based on the identified reprogramming agent. For example, if a reprogramming agent obtained by the above-described method is RNA, the RNA can be sequenced and RNA having the same sequence can be synthesized using a well-known technique. Alternatively, if a reprogramming agent is a protein, antibodies for the protein are produced and the ability of the antibodies to the protein can be utilized to obtain the reprogramming agent from materials which contain the agent. Alternatively, the amino acid sequence of the protein is partially determined; a probe hybridizable to a gene encoding the partial amino acid sequence is produced; and cDNA and genomic DNA encoding the protein can be obtained by a hybridization technique. Such a gene can be amplified by PCR, though a primer needs to be prepared. A gene encoding a reprogramming agent obtained by any of the above-described methods can be used to produce the reprogramming agent by a well-known gene recombinant technique. Therefore, a "reprogramming agent contained in an ES cell" of the present invention is not necessarily obtained from ES cells and can be obtained from cells having pluripotency (e.g., tissue stem cells, etc.). Therefore, the reprogramming agent includes all agents capable of reprogramming a somatic cell.

A reprogramming agent maybe obtained by the following screening method. Embryonic stem cell-derived components are caused to act on an appropriate somatic cell. A component having an activity to reprogram the somatic cell is selected by detecting the activity. Illustrative examples of a somatic cell used herein include, but are not limited to, lymphocytes, spleen cells, testis-derived cells, and the like. Any somatic cells can be used, which have normal chromosomes, can be stably grown, and can be altered by action of a reprogramming agent into an undifferentiated cell having pluripotency. Particularly, it is preferable that a somatic cell used for screening is derived from the same species as that of an ES cell from which components are collected (e.g., a human-derived somatic cell when an ES cell is derived from a human). Previously established cell lines can be used.

In a method for producing a cell, a tissue, or an organ from a somatic cell, a stem cell (e.g., an ES cell) and/or a pluripotent stem cell of the present invention, the cell is differentiated by a method which is not particularly limited as long as the cell is differentiated into a cell, a tissue or an organ, while the karyotype of the cell is substantially retained. For example, by introducing a cell into a blastocyst, subcutaneously injecting a cell into an animal (e.g., a mouse, etc.) to form a teratoma, or the like, the cell can be differentiated into a cell, a tissue, and an organ. A desired cell, tissue, or organ can be isolated from the differentiated blastocyst or teratoma. A desired cell, tissue, or organ may be induced in vitro from a cell by adding a cell growth factor, a growth factor, or the like which is required for obtaining a cell of the type of interest. To date there have been reports for induction of blood vessel, neuron, muscle cell, hematopoietic cell, skin, bone, liver, pancreas, or the like from ES cells. These techniques can be applied when a cell, tissue, or organ corresponding to an implantation recipient is produced from a pluripotent stem cell according to the present invention (e.g., Kaufman, D . S . , Hanson, E. T. , Lewis, R.L., Auerbach, R., and Thomson, J.A. (2001), Proc. Natl. Acad. Sci. USA., 98, 10716-21; Boheler, K.R., Czyz, J., Tweedie, D., Yang, H.T., Anisimov, S.V., and Wobus, A.M. (2002), Circ. Res., 91, 189-201). In addition, in the present invention, when a tissue stem cell is used as a stem cell to produce a fusion cell, the fusion cell may have pluripotency similar to the pluripotency possessed by the original tissue stem cell.

When a stem cell (e.g., an ES cell, etc.) is used in a method for producing a cell, a tissue, or an organ from a cell according to the present invention, the stem cell can be established from an appropriate individual stem cell (e.g., an ES cell, etc.), or previously established stem cells (e.g., ES cells, etc.) derived from various organisms are preferably utilized. For example, examples of such a stem cell include, but are not limited to, stem cells (e.g., ES cells, etc.) of mouse, hamster, pig, sheep, bovine, mink, rabbit, primate (e.g., rhesus monkey, marmoset, human, etc.), and the like. Preferably, stem cells (e.g., ES cells, etc. ) derived from the sample species as that of somatic cells of interest are employed.

Examples of somatic cells used in the method of the present invention for producing cells, tissues or organs from pluripotent stem cells, include, but are not particularly limited to, lymphocytes, spleen cells, testis-derived cells, and the like. Such somatic cells also include any somatic cell having a normal chromosome, which can be stably grown as a fusion cell and can be altered into an undifferentiated cell having pluripotency when it is fused with a stem cell (e.g., an ES cell). When cells, tissues or organs produced by the method are intended to be used for implantation, somatic cells obtained from transplantation individuals are preferably used.

As used herein, the term "fusion cell" refers to an undifferentiated cell which is produced by fusing a stem cell (e.g., an ES cell) with a somatic cell as described above, can be stable grown, and has pluripotency. When chromosomes derived from a host stem cell (e.g., an ES cell) are successfully removed from a fusion cell, the fusion cell can become a diploid undifferentiated cell which has somatic cell-derived chromosomes. The resultant cell is a preferable donor for more ideal treatment of various diseases. Examples of techniques for removing chromosomes derived from stem cells (e.g., ES cells) include irradiation, chemical treatment, methods using genetic manipulation, and the like. For example, by treating stem cells (e.g., ES cells) with irradiation or chemicals before fusion with somatic cells, it is possible to destroy only chromosomes derived from the stem cell after fusion. An exemplary chemical used in removal of chromosomes may be bromodeoxyuridine (BrdU). Chromosomes are treated with BrdU as follows: initially, ES cells are treated with this chemical; and UV irradiation is performed after fusing the ES cells with somatic cells. By irradiation, only chromosomes derived from the stem cell (e.g., an ES cell) treated with BrdU are removed. A technique for removing chromosomes derived from a stem cell (e.g., an ES cell) from a fusion cell by genetic manipulation may be conceived as follows. Initially, a LoxP sequence is randomly introduced into the genome of a stem cell (e.g., an ES cell). After fusing the stem cell with a somatic cell, the Cre protein is forcedly expressed so that only chromosomes derived from the stem cell (e.g., an ES cell) are removed. Therefore, the above-described techniques can be used to remove a part or the whole of the genome derived from a stem cell (e.g., an ES cell).

As used herein, the term "fusion" or "cell fusion" in relation to a cell are used interchangeably and refers to a phenomenon that a plurality of cells are fused together into a multinucleated cell. Fusion naturally occurs in, for example, fertilization of germ cells, and is used as a cell engineering means. To achieve fusion, 2 types of different cells are chemically or physically fused and cultured using a selective medium in which only fusion cells can grow. For example, cell fusion can be induced by using a virus whose infectiosity is inactivated by ultraviolet (e.g., paramyxoviruses, such as HVJ (Sendai virus), parainfluenza virus, Newcastle disease virus, and the like). Also, by using chemical substances, cell fusion can be achieved. Such chemical substances include lysolecithin, polyethyleneglycol (PEG) 6000, glycerol oleate, and the like. As a physical technique, for example, cell fusion (electric fusion) with electric stimuli is performed. Cell fusion with chemical substances is preferably independent and non-specific to viruses.

In the present invention, a method of fusing a stem cell (e.g., an ES cell) with a somatic cell is not particularly limited as long as the stem cell (e.g., an ES cell) is fused with the somatic cell to produce a fusion cell. For example, as described in the examples, ES cells and somatic cells are mixed in a certain ratio, for example, in the case of producing fusion cells of ES cells and thymocyte, at 1:5, and then washed. The cells are suspended in an appropriate buffer such as mannitol buffer, and electrically fused. Besides such a high-voltage pulse cell fusion methods utilizing structural changes in cell membrane by electrical stimulation (electroporation) [for example, EMBO J. 1: 841-845 (1982)], a cell fusion method using a chemical cell fusion acceleration substance such as Sendai virus, lysolecithin, glycerol, oleic acid ester, polyethyleneglycerol and the like is also known. Any fusion method can be used in production of the pluripotent stem cell of the present invention, in which the cells formed by fusion of ES cells and somatic cells can stably proliferate as fusion cells and nuclei derived from somatic cells is reprogrammed such that the resulting cells are undifferentiated cells having pluripotency.

In the case where the cells, tissues, or organs of the present invention are used for transplantation, the cells, tissues, or organs may be used alone or may be used in combination with existing immunosuppression methods, such as immunosuppressants, surgical operations, or irradiation. Major immunosuppressants are adrenocorticosteroid, cyclosporine, FK506 and the like. Surgical operations may be, for example, extraction of lymph node, extraction of spleen, extraction of thymus, thoracic duct drainage, and the like. Irradiation may be total body irradiation and transplantation graft irradiation. By combining these methods appropriately, the rejection reaction in the recipient against the transplantation graft can be more efficiently suppressed.

Therefore, in one embodiment of the method of the present invention, the treatment method of the present invention may comprise avoiding a rejection reaction. Procedures for avoiding rejection reactions are known in the art (see, for example, "Shin Gekagaku Taikei, Zoki Ishoku [New Whole Surgery, Organ Transplantation] (1992). Examples of such methods include, but are not limited to, a method using immunosuppressants or steroidal drugs, and the like. For example, there are currently the following immunosuppressants for preventing rejection reactions: "cyclosporine" (SANDIMMUNE/NEORAL); "tacrolimus" (PROGRAF); "azathioprine" (IMURAN); "steroid hormone" (prednine, methylprednine); and "T-cell antibodies" (OKT3, ATG, etc.). A method which is used worldwide as a preventive immunosuppression therapy in many facilities, is the concurrent use of three drugs: cyclosporine, azathioprine, and steroid hormone. An immunosuppressant is desirably administered concurrently with a pharmaceutical agent of the present invention. The present invention is not limited to this. An immunosuppressant may be administered before or after a regeneration/therapeutic method of the present invention as long as an immunosuppression effect can be achieved.

The term "derived from a desired individual" refers to derived from an individual for which a treatment, such as therapy, prophylaxis, or the like, is desired. Therefore, when a certain target individual is determined, possession of substantially the same information, such as genetic information or the like (e.g., genome information), trait (phenotype traits, etc.) or function as the individual is said to be derived from the desired individual.

The term "not directly derived" in relation to an origin means "not derived from the origin" or "derived from the origin via at least one artificial manipulation (e.g., cell fusion). Therefore, stem cells "not directly derived from" ES cells may include all cells other than ES cells themselves.

The term "derived from a somatic cell" refers to possession of substantially the same information, such as genetic information or the like (e.g., genome information), trait (phenotype traits, etc.) or function as the somatic cell.

The term "clone technique" refers to a technique for producing a "clone", i.e., a genetically identical individual using genetic manipulation.

As used herein, the term "implant" and the terms "graft" and "tissue graft" are used interchangeably, referring to homologous or heterologous tissue or cell group which is inserted into a particular site of a body and thereafter forms a portion of the body. Examples of conventional grafts include, but are not limited to, organs or portions of organs, blood vessels, blood vessel-like tissue, skin segments, cardiac valves, pericardia, duramater, corneas, bone segments, teeth, bones, brain or a portion of brain, and the like. Therefore, grafts encompass any one of these which is inserted into a deficient portion so as to compensate for the deficiency. Grafts include, but are not limited to, autografts, allografts, and heterografts, which depend on the type of their donor. Organs, tissues and cells are typically used as autografts in the present invention, or alternatively, may be used as allografts and heterografts.

As used herein, the term "autograft" refers to a graft which is implanted into the same individual from which the graft is derived. As used herein, the term "autograft" may encompass a graft from a genetically identical individual (e.g. an identical twin) in a broad sense. Cells, tissues or organs differentiated from a cell of the present invention to be implanted are encompassed within the concept of autograft.

As used herein, the term "allograft" refers to a graft which is implanted into an individual which is the same species but is genetically different from that from which the graft is derived. Since an allograft is genetically different from an individual (recipient) to which the graft is implanted, the graft may elicit an immunological reaction. Such a graft includes, but is not limited to, for example, a graft derived from a parent.

As used herein, the term "heterograft" refers to a graft which is implanted from a different species. Therefore, for example, when a human is a recipient, a porcine-derived graft is called a heterograft.

The individual tailor-made technique of the present invention can be used to produce grafts which elicit substantially no rejection reaction. This is because the grafts (e.g., tissues, organs, etc.) produced by the method of the present invention are fitted to therapeutical purposes and side effects, such as immune reaction responses or the like, are significantly suppressed. Therefore, transplantation therapy can be carried out in the situation that only conventional autografts are used but it is difficult to obtain autografts, which is a significant effect of the present invention which cannot be achieved by conventional techniques. In addition, pluripotent cells obtained by the individual tailor-made technique of the present invention can be applied to patients, from which the genome of the pluripotent cell is derived, but also other patients. In this case, the above-described method for avoiding rejection reactions may be preferably used.

As used herein, the term "subject" or "specimen" refers to an organism which is treated (e.g., therapy, prophylaxis, prognostic treatment). A subject or specimen may be referred to as a "patient". A "patient", "subject" or "specimen" may be any organism to which the present invention can be applied. Preferably, a "patient", "subject" or "specimen" is a human.

When a stem cell (e.g., an ES cell, etc.) is used in the present invention, the stem cell can be established from a transplantation individual, or a previously established stem cell derived from various organisms is preferably utilized. For example, examples of such a stem cell include, but are not limited to, stem cells of mouse, hamster, pig, sheep, bovine, mink, rabbit, primate (e.g., rhesus monkey, marmoset, etc.), and a human. Preferably, stem cells derived from the sample species as that of somatic cells to be used are employed.

Examples of somatic cells used in the present invention include any cell, particularly, lymphocytes, spleen cells, testis-derived cells, and the like. Somatic cells derived from mammals (including humans) and various species can be used. The present invention is not particularly limited to this. Any somatic cell which has normal chromosomes and can be stably grown as a fusion cell when fused with a stem cell, such as an ES cell, and can be altered into an undifferentiated cell having pluripotency can be used. Somatic cells obtained from transplantation individuals are preferably used in production of undifferentiated somatic cell fusion cells which have a reduced level of rejection reaction in recipients.

The undifferentiated somatic cell fusion cell of the present invention is a pluripotent, undifferentiated cell which is produced by fusing a stem cell, such as an ES cell, with a somatic cell as described above, and can be stably grown.

In the present invention, a method of fusing stem cells (e. g. , ES cells) and somatic cells when producing cells, tissues, or organs from pluripotent stem cells is not particularly limited as long as ES cells and somatic cells are fused by contacting each other and form fusion cells. For example, as described in the examples, ES cells and somatic cells are mixed in a certain ratio, for example, in the case of producing fusion cells of ES cells and thymocyte, at 1:5, and then washed. The cells are suspended in an appropriate buffer such as mannitol buffer, and electrically fused. Besides such a high-voltage pulse cell fusion methods utilizing structural changes in cell membrane by electrical stimulation (electroporation) [for example, EMBO J. 1: 841-845 (1982)], a cell fusion method using a chemical cell fusion acceleration substance such as Sendai virus, lysolecithin, glycerol, oleic acid ester, polyethyleneglycerol and the like is also known. The fusion method may be any fusion method as long as the cells formed by fusion of ES cells and somatic cells can stably proliferate as fusion cells and nuclei derived from somatic cells is reprogrammed such that the resulting cells are undifferentiated cells having pluripotency.

In the method of the present invention for producing a cell, a tissue, or an organ in which a part or the whole of a transplantation antigen derived from an ES cell, the cell is differentiated by a method which is not particularly limited as long as the cell is differentiated into a cell, a tissue or an organ, while the karyotype of the cell is substantially retained. For example, by introducing a cell into a blastocyst, subcutaneously injecting a cell into an animal (e.g., a mouse, etc.) to form a teratoma, or the like, the cell can be differentiated into a cell, a tissue, and an organ. A desired cell, tissue, or organ can be isolated from the differentiated blastocyst or teratoma. A desired cell, tissue, or organ may be induced in vitro from a cell by adding a cell growth factor, a growth factor, or the like which is required for obtaining a cell of the type of interest. To date there have been reports for induction of blood vessel, neuron, muscle cell, hematopoietic cell, skin, bone, liver, pancreas, or the like from ES cells. These techniques can be applied when a cell, tissue, or organ corresponding to a transplantation individual is produced from a pluripotent stem cell according to the present invention.

### (Description of Preferred Embodiments)

In one aspect, the present invention provides an isolated pluripotent stem cell having a desired genome. Preferably, the pluripotent stem cell may be a non-ES cell. With non-ES pluripotent stem cells having a desired genome, various regenerative therapies can be performed without newly establishing ES cells or collecting egg cells.

The pluripotent stem cell is preferably deficient in at least a part of a transplantation antigen, and more preferably deficient in the whole transplantation antigen. As a transplantation antigen is reduced, the pluripotent stem cell of the present invention has an effect of having a desired genome and havinga reduced level of immune rejection to a host. Preferably, transplantation antigens to be deleted include at least major histocompatibility antigens. More preferably, the major histocompatibility antigen includes class I antigens. Deletion of these specific antigens reduces a major portion of immune rejection reaction, leading to a considerable reduction in side effects.

The pluripotent stem cell of the present invention may preferably have a reprogrammed genome. In one embodiment, the pluripotent stem cell of the present invention may be produced by reprogramming another cell as a supply source. The other cell may be a somatic cell. The somatic cell may be preferably, without limitation, a thymocyte, a lymphocyte, a bone marrow cell, or the like.

In another embodiment, the pluripotent stem cell of the present invention may be produced by fusing a stem cell with a somatic cell, where stem cells and somatic cells are used as supply sources. Stem cells as a supply source may be either ES cells or tissue stem cells, preferably ES cells. This is because the totipotency of an ES cell is transferred into the pluripotent stem cell of the present invention.

The pluripotent stem cell of the present invention preferably has a genome derived from a desired individual (in need of therapy, prophylaxis, treatment, etc.), and is not an ES cell or egg cell of the desired individual. Since there is no need for an ES cell or egg cell of the desired individual, the present invention has a significant effect of overcoming ethical problems in this embodiment. In a preferred embodiment, the pluripotent stem cell of the present invention has chromosomes derived from a somatic cell of the desired individual.

In a preferred embodiment, the pluripotent stem cell of the present invention is not directly derived from embryos. Therefore, it is possible to avoid the socially and ethically problematic act of extracting an embryo from a host. Preferably, the pluripotent stem cell may be derived from a somatic cell. Since the pluripotent stem cell is derived from a somatic cell and has pluripotency, the pluripotent stem cell can be easily obtained and the breadth of applications is infinite.

In a preferred embodiment, the pluripotent stem cell of the present invention has reduced transplantation antigens other than those of the desired individual. More preferably, the pluripotent stem cell of the present invention has no transplantation antigen other than those of the desired individual. In one embodiment, the pluripotent stem cell of the present invention may be derived from cells other than the egg cell of the desired individual.

In one embodiment, the pluripotent stem cell of the present invent ion may be preferably a nonnaturally-occurring cell. Preferably, in the pluripotent stem cell of the present invention, the desired genome is of an individual other than early embryos. The tailor-made pluripotent stem cell has the same genome as that of a somatic cell of an individual of interest, and has pluripotency (preferably totipotency). The cell having both of such properties cannot be said to be naturally-occurring. ES cells are derived from undifferentiated cells of early embryos. Therefore, in principle, ES cells cannot be established from a host in states (e.g., adult) other than early embryo. Since no adult early embryo exists, the tailor-made pluripotent stem cell of the present invention cannot be achieved by conventional techniques.

In a preferred embodiment, the pluripotent stem cell of the present invention is an undifferentiated somatic cell fusion cell of an ES cell, in which a part or the whole of a transplantation antigen is deleted, and a somatic cell. More preferably, the pluripotent stem cell of the present invention is an undifferentiated somatic cell fusion cell of an ES cell, in which the whole of a transplantation antigen is deleted, and a somatic cell. Preferably, the transplantation antigen may be a major histocompatibility antigen. Preferably, the major histocompatibility antigen may be a class I antigen. In a preferred embodiment, the somatic cell may be, without limitation, a transplantation individual-derived lymphocyte, spleen cell or testis-derived cell. Preferably, at least one of the ES cell and the somatic cell may be a human-derived cell. The somatic cell is preferably of the same species (preferably, a line) as that of a host of interest. Therefore, for example, when a human is a target of therapy, the somatic cell is preferably a human cell, and more preferably a somatic cell of the human individual targeted by the therapy. The ES cell may be preferably of the same species (preferably the same line) as that of the somatic cell. Therefore, when a human is targeted by therapy and the somatic cell is a human cell, the ES cell is also preferably a human cell. Note that, in this case, the ES cell may be of any line. Preferably, previously established ES cells (or other pluripotent stem cells) can be used. Therefore, the pluripotent stem cell of the present invention may also be used as a stem cell as a supply source.

In a certain embodiment, at least one of the somatic cell and the stem cell may be genetically modified. Genetic modification may be performed as described herein. Therefore, the pluripotent stem cell of the present invention may be used in combination with gene therapy. A well known gene therapy may be appropriately used depending on the condition of a patient targeted by therapy or prophylaxis.

### (Genome reprogramming agent)

In a preferred embodiment of the present invention, the somatic cell may be treated with a genome reprogramming agent. The present inventors revealed how the somatic cell genome is epigenetically modified by cell fusion with the ES cell and obtained a reprogramming agent and a clue to analysis of the mechanism thereof. Eventually, by forceing the somatic cell to express a reprogramming agent, the somatic cell can be altered directly into a pluripotent stem cell.

The present inventors expected that cell fusion with ES cells causes a dramatic change in the chromatin structure of somatic cell genomes, and analyzed the histone acetylation of somatic cell nuclei in inter-subspecific fusion cells (domesticusxmolossinus).

An anti-acetylated histone H3 antibody, an anti-acetylated histone H4 antibody, an anti-methylated histone H3-Lys4 antibody, and an anti-methylated histone H3-Lys9 antibody were used to investigate modification of the nuclear histone of somatic cells, ES cells, and fusion cell. Next, these four antibodies were used to perform chromatin immunoprecipitation in order to analyze the interaction between histone and DNA. DNA-histone protein complexes were recovered by reaction with the respective antibodies. By PCR amplification of DNA contained in the recovered DNA-histone protein complex, it was revealed how histone was modified in what DNA region. Based on the polymorphism of the base sequence of inter-subspecific genomic DNA, it is possible to determine whether the genome derived from a somatic cell nucleus is modified. As a result of this example, the somatic cell genome is entirely acetylated due to cell fusion to have loose chromatin structure. Importantly, histone H3-Lys4 is specifically methylated in the reprogrammed genome. It is known that methylation of histone H3-Lys4 is associatedwith acetylation of histone H3. Methylation has more stable epigenetics than that of acetylation. Therefore, it is inferred that methylation of histone H3-Lys4 is a characteristic modification of the reprogrammed genome. An enzyme methylating histone H3-Lys4 or an agent involved in methylation is considered to be one of the the reprogramming agents (Figure 11).

An exemplary technique for confirming a reprogramming agent will be described below.
1. To distinguish the genome of a somatic cell from the genome of a somatic cell, an ES cell is established as described in Example 1 from subspecies M. m. molossinus (mol) which has a DNA base sequence having a higher degree of polymorphism as compared to Mus musculus domesticus(dom) mouse. An inter-subspecific fusion cell of an ES cell (dom) × a somatic cell (mol) or an ES cell (mol) × a somatic cell (dom) is produced.
2. The somatic cell, ES cell and fusion cell are fixed with 1% formaldehyde solution for 10 minutes to cross-link the histone protein with DNA (histone-DNA complex). Thereafter, the nuclear protein is extracted as described above. The nuclear protein is reacted with an anti-acetylated histone H3 antibody, an anti-acetylated histone H4 antibody, an anti-methylated histone H3-Lys4 antibody, and an anti-methylated histone H3-Lys9 antibody overnight as described above.
3. The reaction solution is passed through a protein A column to separate the histone-DNA complex reacts with the antibody as described above. DNA is extracted from the histone-DNA complex reacted with each antibody as described above.
4. The extracted DNA is blotted and adsorbed onto a membrane. The DNA, the repeat sequence B2 repeat scattered on the genome, IAP, and mouse genomic DNA are used as probes to perform hybridization. As a result, all of the probe DNAs used reacted with acetylated histone H3-Lys9 on the genomes of somatic cells, while they reacted with acetylated histone H3-Lys4, acetylated histone H3, and acetylated histone H4 on the genomes of ES cells and fusion cells.
5. The extracted DNA was amplified using genomic PCR-primer sets respectively specific to the Oct4 gene which is expressed in undifferentiated cells, but not in somatic cells, the Neurofilament-M and -L genes which are not expressed in somatic cells or undifferentiated cells, the Thy-1 gene which is expressed in somatic cells, but not in undifferentiated cells. The difference in recognition of restriction enzymes for polymorphic sites of DNA base sequences was utilized to determine wheter DNA amplified in a fusion cell was derived from an ES cell genome or a somatic cell genome. As a result, somatic cell-derived genomes were reacted with acetylated histone H3-Lys4, acetylated histone H3, and acetylated histone H4 in fusion cells irrespective of the presence or absence of genes in somatic cells or irrespective of presence or absence of genes in fusion cells. Although an ES cell is used as an exemplary stem cell in the above description, a reprogramming agent can be confirmed in any stem cell exhibiting pluripotency.

Acetylated histone is known to form loose chromatin structure. On the other hand, it is known that the methylation of histone H3-Lys4 and histone H3-Lys9 are complementary modifications, and that histone H3-Lys9 is methylated in tight chromatin, while histone H3-Lys4 is methylated in loose chromatin. Analysis of repeat sequences scattered throughout the genome and each gene in fusion cells suggests that the reprogrammed somatic cell genome forms loose chromatin structure. Particularly, it seems that methylation of histone H3-Lys4 plays an important role in reprogramming.

Therefore, in another aspect of the present invention, the present invention provides a method for producing a pluripotent stem cell having a desired genome, comprising the steps of: 1) providing a cell having the desired genome; and 2) exposing the cell to a reprogramming agent. Preferably, the cell may be a somatic cell. The reprogramming agent may be prepared as an intranuclear agent within the cytoplasm of the pluripotent stem cell. Examples of the reprogramming agent include, but are not limited to, an enzyme methylating histone H3-Lys4 or an agent involved in methylation of histone H3-Lys4, a cell cycle agent, DNA helicase, a histone acetylating agent, and a transcription agent and the like.

### (Fusion cell of transplantation antigen (e.g., MHC)-deficient ES cell with somatic cell)

In one aspect of the present invention, the present invention is a method for producing a pluripotent stem cell having a desired genome, comprising the steps of: 1) deleting a part or the whole of a transplantation antigen of a stem cell: and 2) fusing the stem cell with a somatic cell having the desired genome. Preferably, the stem cell may be an ES cell, and more preferably a previously established ES cell.

In a preferred embodiment, the transplantation antigen may be a major histocompatibility antigen. More preferably, the major histocompatibility antigen may be a class I antigen. The somatic cell may be a transplantation individual-derived lymphocyte, spleen cell, or testis-derived cell.

In the method for producing the pluripotent stem cell of the present invention, an undifferentiated somatic cell fusion cell of a stem cell (e.g., an ES cell), in which a part or the whole of a transplantation antigen is deleted, with a somatic cell may be used as a supply source. More preferably, an undifferentiated somatic cell fusion cell of a stem cell (e.g., an ES cell), in which the whole of a transplantation antigen is deleted, with a somatic cell may be used as a supply source.

In a preferred embodiment, the somatic cell may be, without limitation, a transplantation individual-derived lymphocyte, spleen cell or testis-derived cell. Preferably, at least one of the ES cell and the somatic cell may be a human-derived cell. The somatic cell is preferably of the same species (preferably, a line) as that of a host of interest. Therefore, for example, when a human is a target of therapy, the somatic cell is preferably a human cell, and more preferably a somatic cell of the human individual targeted by the therapy. The ES cell may be preferably of the same species (preferably the same line) as that of the somatic cell. Therefore, when a human is targeted by therapy and the somatic cell is a human cell, the ES cell is also preferably a human cell. Note that, in this case, the ES cell may be of any line. Preferably, previously established ES cells (or other pluripotent stem cells) can be used. Therefore, the pluripotent stem cell of the present invention may also be used as a stem cell as a supply source.

In a preferred embodiment, the method for producing the pluripotent stem cell of the present invention may comprise deleting the whole of the transplantation antigen. A technique for deleting the whole of the transplantation antigen includes irradiation, chemical treatment, a method using genetic manipulation, and the like. For example, by treating stem cells (e.g., ES cells) with irradiation or chemicals before fusion with somatic cells, it is possible to destroy only chromosomes derived from the stem cell after fusion. An exemplary chemical used in removal of chromosomes may be bromodeoxyuridine (BrdU). Chromosomes are treated with BrdU as follows: initially, ES cells are treated with this chemical; and UV irradiation is performed after fusing the ES cells with somatic cells. By irradiation, only chromosomes derived from the stem cell (e.g., an ES cell) treated with BrdU are removed. A technique for removing chromosomes derived from a stem cell (e.g., an ES cell) from a fusion cell by genetic manipulation may be conceived as follows. Initially, a LoxP sequence is randomly introduced into the genome of a stem cell (e.g., an ES cell). After fusing the stem cell with a somatic cell, the Cre protein is induced to express so that only chromosomes derived from the stem cell (e.g., an ES cell) are removed. Therefore, the above-described techniques can be used to remove a part or the whole of the genome derived from a stem cell (e.g., an ES cell).

In a preferred embodiment of the present invention, it is intended to produce a fusion cell of an ES cell deficient in a major histocompatibility complex (MHC) with a somatic cell. The major histocompatibility complex (MHC) is known as a molecule involved in a rejection reaction of tissue transplanted into other individuals of the same species. Mouse MHC corresponds to the H-2 antigen. MHC is divided into three clusters: class I, class II, and class III. It is known that class I genes which undergo antigen presentation to CD4 T cells, and class II genes, which undergo antigen presentation to CD8 T cells, are involved in rejection reactions to non-self transplanted cells. The present inventors produced an ES cell from which MHC class I and class II genes were removed by genetic manipulation and fused the ES cell with a somatic cell obtained from an individual. The resultant somatic cell-ES (MHC-) fusion cell presents only somatic cell genome-derived self MHC class I and class II antigens on the cell surface, so that the fusion cell is no longer recognized as non-self. The somatic cell-ES (MHC-) fusion cell, which inherits antigens for self recogniztion from the somatic cell genome and the reprogramming activity from the ES (MHC-) cell, can be said to be an MHC tailor-made fusion cell. Since the somatic cell-derived MHC is expressed in the MHC tailor-made ES cell, the cell is not attacked by natural killer cells. MHC class I deficient mice and MHC class II deficient mice have already been produced. An approach of producing MHC class I/class II deficient mice by mating these mice, and MHC class II deficient mouse-derived ES cells were established, and class I genes were deleted by homologous recombination. ES (MHC- ) cells are obtained and fused with somatic cells of different mouse lines to produce somatic cell-ES(MHC-) fusion cells. By transplanting the fusion cells into the ES cell-derived mice and the somatic cell-derived mice, the presence or absence of rejection reactions can be examined.

The above-described method will be, for example, described below (Figure 12).
1. H-2 class I deficient mice and H-2 class II deficient mice are used to produce H-2 class I and II deficient mice. Three approaches are considered for production. 1) H-2 class I deficient mice are mated with H-2 class II deficient mice to produce mice deficient in both class I class II. 2) H-2 class II (-/-) ES cells are produced from H-2 class II deficient miceandH-2 class I is removed therefrom by homologous recombination. From the resultant ES cells, mice deficient in both class I and class II are produced. 3) H-2 class I is removed from H-2 class II deficient mice somatic cell-derived cultured cells by homologous recombination. By somatic cell nuclear transplantation, mice deficient in both class I and class II are produced.
2. The H-2 class I (-/-) class II (-/-) ES cell is fused with a somatic cell or a tissue stem cell to produce an MHC tailor-made ES cell.
3. The MHC tailor-made ES cell or a tissue cell differentiated therefrom is implanted into an individual which has supplied the somatic cell. The presence or absence of a rejection reaction is determined.
4. Once a master cell for the H-2 class I (-/-) class II (-/-) ES cell is obtained, an MHC tailor-made ES cell suited to an individual can be produced by changing the somatic cell to be combined.

### (Removal of ES cell genome from somatic cell-ES fusion cell)

It is more preferably to completely avoid agents inducing rejection reactions. To completely avoid rejection reactions, it is necessary to produce a tailor-made stem cell derived from a somatic cell of an individual of interest. In somatic cell-stem cell (e.g., an ES cell) fusion cells, the genome of the reprogrammed somatic cell has differentiation ability similar to that of the genome of the original stem cell (e.g., an ES cell). Therefore, by removing the genome of the stem cell (e.g., an ES cell) from the fusion cell using genetic manipulation, a tailor-made ES cell can be obtained. According to the present inventors experiment of reactivating the somatic cell-derived Oct4 gene in cell fusion (Tada et al., Curr. Biol., 2001), it has been revealed that it takes about 2 days for the somatic cell genome to be reprogrammed. Therefore, it is necessary to selectively remove the ES cell genome after cell fusion.

The present inventors produced a transgenic ES cell in which at least one LoxP sequence was introduced into each chromosome thereof (Figure 13). A construct of Insulator-Polymerase II promoter-GFP-LoxP-Insulator was produced using a retrovirus vector (Figure 14). ES cells are infected with the retrovirus, followed by sorting with a cell sorter using GFP as a marker, so that the resultant transgenic ES cells are concentrated. The inserted site is detected by DNA FISH. Aplasmid, which transiently expresses the Cre enzyme, is introduced into the fusion cell of the transgenic ES cell and the somatic cell. Due to the action of the Cre enzyme, the LoxP sequences undergo homologous recombination, so that only the chromosomes derived from the ES cell genome are modified to dicentric or acentric chromosomes, and are removed by cell division over the cell cycle. Only diploid genome derived from the reprogrammed somatic cell remains. Thus, the individual somatic cell-derived tailor-made pluripotent stem cell is produced. Once a transgenic ES cell is established, it is possible to easily establish a tailor-made ES cell by fusion using a somatic cell derived individual patients. Therefore, if tailor-made ES cells are successfully established in the mouse model experimental system, an attempt will be made to apply the present invention to human ES cells to produce human tailor-made ES cells derived from somatic cells of individuals. Unlike nuclear transplantation clones, reprogramming of somatic cell genomes by cell fusion without use of human unfertilized eggs is within the scope of ES cell application and complies with guidelines. This is an innovative genome engineering technique which provides a maximum effect on regenerative medicine while minimizing ethical problems.

Such a technique will be described below.
1. In order to introduction efficiency of genes, a retrovirus is used for gene introduction. A construct of Insulator-Polymerase II promoter-GFP-LoxP-Insulator is produced using a retrovirus vector (Figure 14). The Insulator was used to separate LoxP from the influence of surrounding genes, and the Polymerase II promoter was used to cause the GFP to be properly expressed so that the number of gene copies can be linearly identified using a cell sorter. GFP, which has the lowest toxicity at present, is used to screening ES cells having the introduced gene. The number of LoxP sequence copies is correlated with the expression level of GFP.
2. The Insulator-Polymerase II promoter -GFP-LoxP-Insulator gene is introduced into ES cells. Thereafter, transgenic ES cells are collected using the cell sorter where the expression level of the GFP gene is used as a reference. This manipulation is performed several times.
3. ES cells, for which gene introduction is performed several times, are cloned. Insulator-Polymerase II promoter-GFP-LoxP-Insulator is used as a probe and mapped onto chromosomes. Transgenic ES cells, which have at least one gene per chromosome, are selected.
4. A plasmid expressing the Cre enzyme is introduced into the fusion cell obtained by fusing the transgenic ES cell with the somatic cell, causing the Cre enzyme to be temporarily expressed. Due to the action of the Cre enzyme, the LoxP sequences undergo homologous recombination, so that only the chromosomes derived from the ES cell genome are modified to dicentric or acentric chromosomes, and are removed by cell division.
5. After several cell division, only the reprogrammed somatic cell genome remains, so hat a tailor-made ES cell is completed.

In the case where the cells, tissues, or organs of the present invention are used for transplantation, the cells, tissues, or organs may be used alone or may be used in combination with existing immunosuppression methods, such as immunosuppressants, surgical operations, or irradiation. Major immunosuppressants are adrenocorticosteroid, cyclosporine, FK506 and the like. Surgical operations may be, for example, extraction of lymph node, extraction of spleen, extraction of thymus, thoracic duct drainage, and the like. Irradiation may be total body irradiation and transplantation graft irradiation. By combining these methods appropriately, the rejection reaction in the recipient against the transplantation graft can be more efficiently suppressed.

In another aspect, the present invention provides cells, tissues or organs differentiated from a pluripotent stem cell having a desired genome. The cells may be epidermic cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, and chondrocytes. Preferably, the cells may be myocytes, chondrocytes, epithelial cells, or neurons. Techniques for differentiation are well known in the art and are well described in the examples described herein and the documents mentioned herein.

In another preferred embodiment, the tissue maybe, without limitation, muscle, cartilage, epithelium or nerve. In a preferred embodiment, the organ is selected from the group consisting of brain, spinal cord, heart, liver, kidney, stomach, intestine, and pancreas. Techniques for differentiation of tissues and organs are well known in the art and are well described in the examples described herein and the documents mentioned herein.

In a preferred embodiment, the cell, tissue or organ of the present invention is used for transplantation. More preferably, the desired genome is substantially the same as that of a transplanted host. When the cell, tissue or organ of the present invention is used for transplantation, a desired effect can be achieved because of the desired genome. In addition, there is advantageously a reduced level of or no immune rejection reaction.

### (Medicament, and therapy, prophylaxis, and the like using the same)

In another aspect, the present invention provides a medicament comprising a cell, tissue or organ differentiated from a pluripotent stem cell having a desired genome. The medicament can be used for patients having a disease, disorder or condition in need of such a cell (preferably, a differentiated cell), tissue or organ. Such a disease, disorder or condition includes defects/injuries in cells, tissues or organs.

In another aspect, the present invention provides a medicament for treatment or prophylaxis of a disease or disorder due to a defect in a cell, tissue or organ of a subject, comprising a pluripotent stem cell having substantially the same genome as that of the subject. In this case, the pluripotent stem cell itself is used as the medicament and the pluripotent stem cell is differentiated as desired, depending on the transplanted environment. As a result, therapy may be promoted. In order to achieve the desired differentiation at the transplanted site, an agent involved in differentiation (e.g., SCF) or the like may be previously or simultaneously administered.

The above-described medicament may further comprise a carrier and the like as described herein.

In another aspect, the present invention provides a method for treatment or prophylaxis of a disease or disorder due to a defect in a cell, tissue or organ of a subject, comprising the steps of: preparing a pluripotent stem cell having substantially the same genome as that of the subject; differentiating the pluripotent stem cell into the cell, tissue or organ; and administering the cell, tissue or organ into the subject. The disease or disorder may be any one that requires a fresh differentiated cell, tissue or organ. Specific examples of the disease or disorder will be described below. Substantially the same genome refers to a genome having a level of homology which does not impair identity (i.e., does not elicit an immune response). Note that when a step of avoiding a rejection reaction is used, the genome may not be necessarily the same as that of the subject.

In another aspect, the present invention provides a method for treatment or prophylaxis of a disease or disorder due to a defect in a cell, tissue or organ of a subject, comprising the steps of: administering a pluripotent stem cell having substantially the same genome as that of the subject into the subject. To administer the pluripotent stem cell, techniques well known in the art are used. Administration methods may be herein oral, parenteral administration (e.g., intravenous, intramuscular, subcutaneous, intradermal, tomucosa, intrarectal, vaginal, topical to an affected site, to the skin, etc.). A prescription for such administration may be provided in any formulation form. Such a formulation form includes liquid formulations, injections, sustained preparations, and the like.

In another aspect, the present invention provides a method for treatment or prophylaxis of a disease or disorder due to a defect in a cell, tissue or organ of a subject, comprising the steps of: administering a medicament comprising a cell, tissue or organ differentiated from a pluripotent stem cell having substantially the same genome as that of the subject. To administer the medicament, techniques well known in the art are used and may be herein oral, parenteral administration (e.g., intravenous, intramuscular, subcutaneous, intradermal, to mucosa, intrarectal, vaginal, topical to an affected site, to the skin, etc.). A prescription for such administration may be provided in any formulation form. Such a formulation form includes liquid formulations, injections, sustained preparations, and the like. Alternatively, when the medicament is an organ itself, administration is achieved by transplantation.

In another aspect, the present invention provides use of a pluripotent stem cell for treatment or prophylaxis of a disease or disorder due to a defect in a cell, tissue or organ of a subject. The medicament comprises a cell, tissue or organ differentiated from a pluripotent stem cell having substantially the same genome as that of the subject.

In another aspect, the present invention use of a pluripotent stem cell comprising a desired genome for producing producing a medicament comprising the pluripotent stem cell. Techniques for producing the medicament (e.g., biotechnology formulations) are well known in the art. Those skilled in the art can produce the medicament in accordance with the regulation of the authority.

In another aspect, the present invention use of a pluripotent stem cell having a desired genome for producing a medicament comprising a cell, tissue or organ differentiated from the pluripotent stem cell.

Diseases or disorders, which may be treated by the present invention, may be associated with defects in cells, tissues or organs differentiated from the stem cell of the present invention.

In one embodiment, the above-described differentiated cells, tissues, or organs may be of the circulatory system (blood cells, etc.). Examples of the diseases or disorders include, but are not limited to, anemia (e.g., aplastic anemia (particularly, severe aplastic anemia), renal anemia, cancerous anemia, secondary anemia, refractory anemia, etc.), cancer or tumors (e.g., leukemia); and after chemotherapy therefor, hematopoietic failure, thrombocytopenia, acute myelocytic leukemia (particularly, a first remission (high-risk group), a second remission and thereafter), acute lymphocytic leukemia (particularly, a first remission, a second remission and thereafter), chronic myelocytic leukemia (particularly, chronic period, transmigration period), malignant lymphoma (particularly, a first remission (high-risk group), a second remission and thereafter), multiple myeloma (particularly, an early period after the onset), and the like.

In another embodiment, the above-described differentiated cells, tissues, or organs may be of the nervous system. Examples of such diseases or disorders include, but are not limited to, dementia, cerebral stroke and sequela thereof, cerebral tumor, spinal injury, and the like.

In another embodiment, the above-described differentiated cells, tissues, or organs may be of the immune system. Examples of such diseases or disorders include, but are not limited to, T-cell deficiency syndrome, leukemia, and the like.

In another embodiment, the above-described differentiated cells, tissues, or organs may be of the motor organ and the skeletal system. Examples of such diseases or disorders include, but are not limited to, fracture, osteoporosis, luxation of joints, subluxation, sprain, ligament injury, osteoarthritis, osteosarcoma, Ewing's sarcoma, osteogenesis imperfecta, osteochondrodysplasia, and the like.

In another embodiment, the above-described differentiated cells, tissues, or organs may be of the skin system. Examples of such diseases or disorders include, but are not limited to, atrichia, melanoma, cutis matignant lympoma, hemangiosarcoma, histiocytosis, hydroa, pustulosis, dermatitis, eczema, and the like.

In another embodiment, the above-described differentiated cells, tissues, or organs may be of the endocrine system. Examples of such diseases or disorders include, but are not limited to, hypothalamus/hypophysis diseases, thyroid gland diseases, accessory thyroid gland (parathyroid) diseases, adrenal cortex/medulla diseases, saccharometabolism abnormality, lipid metabolism abnormality, protein metabolism abnormality, nucleic acid metabolism abnormality, inborn error of metabolism (phenylketonuria, galactosemia, homocystinuria, maple syrup urine disease), analbuminemia, lack of ascorbic acid systhetic ability, hyperbilirubinemia, hyperbilirubinuria, kallikrein deficiency, mast cell deficiency, diabetes insipidus, vasopressin secretion abnormality, dwarfism, Wolman's disease (acid lipase deficiency)), mucopolysaccharidosis VI, and the like.

In another embodiment, the above-described differentiated cells, tissues, or organs may be of the respiratory system. Examples of such diseases or disorders include, but are not limited to, pulmonary diseases (e.g., pneumonia, lung cancer, etc.), bronchial diseases, and the like.

In another embodiment, the above-described differentiated cells, tissues, or organs may be of the digestive system. Examples of such diseases or disorders include, but are not limited to, esophagial diseases (e.g., esophagial cancer, etc.), stomach/duodenum diseases (e.g., stomach cancer, duodenum cancer, etc.), small intestine diseases/large intestine diseases (e.g., polyps of the colon, colon cancer, rectal cancer, etc.), bile duct diseases, liver diseases (e.g., liver cirrhosis, hepatitis (A, B, C, D, E, etc.), fulminant hepatitis, chronic hepatitis,primary liver cancer, alcoholic liver disorders, drug induced liver disorders, etc.), pancreatic diseases (acute pancreatitis, chronic pancreatitis, pancreas cancer, cystic pancreas diseases, etc.), peritoneum/abdominal wall/diaphragm diseases (hernia, etc.), Hirschsprung's disease, and the like.

In another embodiment, the above-described differentiated cells, tissues, or organs may be of the urinary system. Examples of such diseases or disorders include, but are not limited to, kidney diseases (e.g., renal failure, primary glomerulus diseases, renovascular disorders, tubular function abnormality, interstitial kidney diseases, kidney disorders due to systemic diseases, kidney cancer, etc.), bladder diseases (e.g., cystitis, bladder cancer, etc.), and the like.

In another embodiment, the above-described differentiated cells, tissues, or organs maybe of the genital system. Examples of such diseases or disorders include, but are not limited to, male genital organ diseases (e.g., male sterility, prostatomegaly, prostate cancer, testicular cancer, etc.), female genital organ diseases (e.g., female sterility, ovary function disorders, hysteromyoma, adenomyosis uteri, uterine cancer, endometriosis, ovarian cancer, villosity diseases, etc.), and the like.

In another embodiment, the above-described differentiated cells, tissues, or organs may be of the circulatory system. Examples of such diseases or disorders include, but are not limited to, heart failure, angina pectoris, myocardial infarct, arrhythmia, valvulitis, cardiac muscle/pericardium diseases, congenital heart diseases (e.g., atrial septal defect, arterial canal patency, tetralogy of Fallot, etc.), artery diseases (e.g., arteriosclerosis, aneurysm), vein diseases (e.g., phlebeurysm, etc.), lymphoduct diseases (e.g., lymphedema, etc.), and the like.

With the stem cell of the present invention, the above-described diseases could be treated while avoiding conventional side effects of transplantation therapy of naturally-occurring stem cells or differentiation cells (particularly, caused by foreign matter or heterogenous cells, (e.g., infection, graft-versus-host diseases, etc.)). This effect is efficiently achieved only after a method is provided which can maintain the pluripotency and self-replication of a stem cell. The effect cannot be conventionally achieved or is difficlut.

In another embodiment, the pluripotent stem cell of the present invention may be genetically modified, or alternatively, may be used in combination with gene therapy when a cell, tissue or organ derived from the pluripotent stem cell of the present invention is used. Gene therapy is well known in the art as described in, for example, review articles in Curr. Gene Ther., 2002 May, 2(2). Examples of such gene therapy include, but are not limited to, those using adeno-associated virus, Sendai virus, Epstein-Barr virus (EBV), herpes simplex virus (HSV), Alpha virus, Lentivirus, and the like.

In another embodiment, the treatment method of the present invention may further comprise administering other medicament(s). Such a medicament may be any medicament known in the art. For example, such a medicament may be any medicament known in the field of pharmacy (e.g., antibiotics, etc.). The treatment method of the present invention may comprise two or more other medicaments. Examples of such medicaments include those described in, for example, the latest Japanese Pharmacopeia, the latest US Pharmacopeia, the latest pharmacopeias in other countries, and the like. The medicament may have an effect on a disease or treatment of interest, or may have an effect on other diseases or treatments.

In another embodiment, the present invention may comprise two or more types of somatic cells. When two or more types of cells are used, the cells may have similar properties or may be derived from similar cells, or may have different properties or may be derived from different cells.

The amount of cells used in the treatment method of the present invention can be easily determined by those skilled in the art with reference to the purpose of use, a target disease (type, severity, and the like), the patient's age, weight, sex, and case history, the form or type of the cell, and the like.

The frequency of the treatment method of the present invention applied to a subject (or a patient) can also be easily determined by those skilled in the art with reference to the purpose of use, a target disease (type, severity, and the like), the patient's age, weight, sex, and case history, the form or type of the cell, and the like. Examples of the frequency include once per day to several months (e.g., once per week to once per month). Preferably, administration is performed once per week to month with reference to the progression.

Any pharmaceutically acceptable carrier known in the art may be used in the medicament of the present invention.

Examples of a suitable formulation material or a pharmaceutical acceptable agent include, but are not limited to, antioxidants, preservatives, colorants, flavoring agents, diluents, emulsifiers, suspending agents, solvents, fillers, bulky agents, buffers, delivery vehicles, and/or pharmaceutical adjuvants. Represetatively, a medicament of the present invention is administered in the form of a composition comprising an isolated pluripotent stem cell, or a variant or derivative thereof, with at least one physiologically acceptable carrier, exipient or diluent. For example, an appropriate vehicle may be injection solution, physiological solution, or artificial cerebrospinal fluid, which can be supplemented with other substances which are commonly used for compositions for parenteral delivery.

Examples of appropriate carriers include neutral buffered saline or saline mixed with serum albumin. Preferably, the product is formulated as a lyophilizate using appropriate excipients (e.g., sucrose). Other standard carriers, diluents, and excipients maybe included as desired. Other exemplary compositions comprise Tris buffer of about pH 7.0 to 8.5, or acetate buffer of about pH 4.0 to 5.5, which may further include sorbitol or a suitable substitute therefor.

The medicament of the present invention may be administered orally or parenterally. Alternatively, the medicament of the present invention may be administered intravenously or subcutaneously. When systemically administered, the medicament for use in the present invention may be in the form of a pyrogen-free, pharmaceutically acceptable aqueous solution. The preparation of such pharmaceutically acceptable compositions, with due regard to the survival of cells, tissues or organs, pH, isotonicity, stability and the like, is within the skill of the art.

The medicament of the present invention may be prepared for storage by mixing a sugar chain composition having the desired degree of purity with optional physiologically acceptable carriers, excipients, or stabilizers (Japanese Pharmacopeia ver. 14, or a supplement thereto or the latest version; Remington's Pharmaceutical Sciences, 18th Edition, A. R. Gennaro, ed., Mack Publishing Company, 1990; and the like), in the form of lyophilized cake or aqueous solutions.

Acceptable carriers, excipients or stabilizers used herein preferably are nontoxic to recipients and are preferably inert at the dosages and concentrations employed, and preferably include phosphate, citrate, or other organic acids; antioxidants, such as ascorbic acid; low molecular weight polypeptides; proteins (e.g., serum albumin, gelatin, or immunoglobulins); hydrophilic polymers (e.g., polyvinylpyrrolidone); amino acids (e.g., glycine, glutamine, asparagine, arginine or lysine); monosaccharides, disaccharides, and other carbohydrates (glucose, mannose, or dextrins); chelating agents (e.g., EDTA); sugar alcohols (e.g., mannitol or sorbitol); salt-formingcounterions (e.g., sodium); and/or nonionic surfactants (e.g., Tween, pluronics or polyethylene glycol (PEG)).

In a preferred embodiment of the present invention, by exposing a cell (e.g., a somatic cell) having a desired genome (e.g., substantially the same genome as that of an individual targeted by therapy) to a reprogramming agent, an aquired pluripotent stem cell having the desired genome can be obtained.

When the undifferentiated somatic cell fusion cell and the fusion cell-derived cell, tissue , or organ obtained by the method of the present invention are used for transplantation, the rejection reaction of the recipient is reduced as convetional ES cell only-derived cells, tissues or organs since a part or the whole of an ES cell-derived transplantation antigen is deleted. Therefore, the present invention can infinitely supply materials for transplantationfor a number of diseases including myocardiac infarct, Parkinson's disease, diabetes, and leukemia. In addition, since the infinite proliferative ability and pluripotency of the ES cell are maintained, the cell, tissue or organ of the present invention can be used for examination and production of pharmaceutical products, cosmetic products, and the like, and are useful for functional analysis of genes whose sequences are revealed by the Genome Project or the like. In addition, the pluripotent stem cell of the present invention can be used for screening for cell growth factors, growth factors, and the like required for inducing differentiation of specific cells, tissues or organs.

Further, the present invention provides an experimental system which can manipulate and study a molecular mechanism associated with reprogramming. In such an experimental system, the ability of an ES cell to reprogram at least a portion of the nucleus of a somatic cell, which was achieved only after a fusion cell of an ES cell with a somatic cell had been produced, is utilized in vitro. After a thymocytewas fusedwith an ES cell, the specific methylation pattern of H19 and Igf 2r of somatic cells was not altered. Typically, this allelic gene specific methylation is maintained during development after fertilization, but not during the development of germ cells [Tremblay K.D. et al., Nature Ganet., 9:407-413(1995); Stroger R. et al., Cell, 73:61-71(1993)]. In fact, the somatic cell methylation pattern of several imprinted genes (including Igf2r) were interrupted in the EG-thymocyte fusion cell, and none of alleles were methylated [Tada M. et al., EMBO J., 16:6510-6520(1997)]. According to this finding, it is considered that both ES cells and EG cells retain similar cellular agents which can reprogram the epigenetic state of the somatic cell nucleus capable of the development of germ cells. However, unlike EG cells, imprinting is not reprogrammed in ES cells. Methylation analysis of Igf2r in ES×EG fusion cells suggested that EG cells have an additional dominant agent involved in more potent reprogramming of epigenetics. In fact, it seems that ES cells and EG cells exhibit characteristics of the respective origins thereof. Thus, both ES cells and EG cells are useful materials for reprogramming epigenetics and identifying agents involved in demethylation in early germ cells and gonad PGC.

In the case of production of clones using somatic cell nuclei, the proportion of clones which survive to become adults is very low. The loss of embryos before transplantation may be caused in part by lack of nucleus-cytoplasm interaction [Kato Y.et al., Science 282:2095-2098 (1998); Wakayama T. et al., Nature 394:369-374(1998). Further, many cloned fetuses are lost during pregnancy and immediately after birth. One reason for failure during the development stage is considered to be the lack of effective reprogramming of the somatic cell nucleus. In almost all ES hybrid clones examined, stable expression of GFP was observed, indicating that this system had normal reprogramming of nuclei. It was revealed that primordial methylation imprints from the somatic cell are maintained in ES hybrids for H19 and IGF2r genes, and the epigenetics profile of cells in a certain species are not affected by cell fusion. This is also supported by the finding that the somatic cell-derived inactive X chromosome "memorizes" the origin thereof and is non-randomly selected by inactivation in trophectodermal cells of cloned embryos [Eggan K. et al., Science, 290:1578-1581(2000)].

The mechanism of somatic cell nuclei involved in reprogramming of epigenetics, which leads to the ability of normal embryonic development, has yet to be fully investigated. Recently, it has been indicated that the mutation of the ATRX gene, which is a member of the SWI2/SNF2 helicase/ATPase family, alters the methylation profile of a sequence, which is repeated many times in mammals [Gibbons R.J. et al., Nat. Genet., 24:368-371(2000)]. As a result, the possibility that demethylation takes place as a result of reconstruction of chromosomes, was suggested. It has been reported that the activity of maternal ISWI, which is an ATPase-dependent DNA helicase, may function as a chromosome remodeler during reprogramming of the nuclei of cloned frog somatic cells [Kikyo N. et al., Science, 289:2360-2362 (2000)]. Nuclei, which are extracted from Xenpus XTC-2 epithelial cells and are incubated in Xenopus eggs for a short time, are reconstructed and lose component TBP which is a key component in the basic transcription complex. Therefore, the reprogramming activity of ES cells seems to facilitate the formation of chromosomes having loose structure which causes loss of the memory of epigenetics in somatic cells. In the undifferentiated somatic cell fusion cell of the present invention, somatic cell-derived imprints are maintained, so that normal reprogramming is performed. As compared to when EG cells are used, the present invention is advantageous not only in production of animal clones but also in production of cells, tissues or organs for transplantation. It has been indicated that because of the instability of epigenetics of several genes imprinted in mouse ES cells, the state of the epigenetics has to be evaluated before clinically applying human ES cells [Humpherys D. et al., Science, 293:95-97(2000)]. When chromosomes of a host ES cell can be successfully removed, ES fusion cells can be a particularly useful therapeutic means. It is considered that once reprogramming agents are identified, epigenetics can be manipulated using these reprogramming agents. Identification of such an agent makes it possible to produce clones from adult somatic cells or tissue specific stem cells without mammalian embryos. Such a technique is expected to make it possible to produce donor cells for a number of clinical applications, where cell or tissue transplantation is required.

Hereinafter, the present invention will be described by way of examples. Examples described below are provided only for illustrative purposes. Accordingly, the scope of the present invention is not limited except as by the appended claims.

### EXAMPLES

Hereinafter, the present invention will be described by way of examples. The present invention is not limited to the examples.

### (Example 1: Preparation of fusion cells of somatic cells)

### 1. Preparation of chimeric embryos

### (1) Establishment of ES cell lines and EG cell lines

As ES cell lines, ES cell line TMAS-5 [Isolation, Culture, and Manipulation of embryonic stem cells (pp. 254-290), in "Manipulating the mouse embryo: A Laboratory Manual 2nd Edition" edited by Hogan, Beddington, Castantini and Lacy (Cold Spring Harbor Laboratory Press, USA) (1994)], and G418-resistant ES cell line NR-2 carrying a neo/lacZ reporter gene, which was derived from Rosa26 blastocyst [Friedrich G. and Soriano P., GenESDev. 5 :1513-1523 (1991)], which were established from E3.5 male 129/Sv blastocysts, were used. As EG cell lines, EG cell line TMA-58G [Tada M. et al. , EMBO J., 16:6510-6520 (1997)], which as established from E12.5 female PGC [Tada T. et al., Dev. Gene. Evol. 207:551-561 (1998)], and blstoydine hydrochloride (BS)-resistant EG cell line (TMA-58G^{bsr}), which was produced by transfecting a drug-resistant gene pSV2bsr into TMA-58G cells, were used. These cells were maintained on mouse G418-resistant primordial embryonic fibroblast (PEF) feeder cells (prepared using a typical technique from primary cultured fibroblasts in 12.5 day-old embryos of Rosa26), which were inactivated with mitomycin C in Dulbecco's Modified Eagle's medium (DMEM)) supplemented with ES medium (15% fetal bovine serum, 10⁻⁴ M2-mercaptoethanol, and 1000 units /mL recombinant leukemia inhibiting agent (LIF; ESGRO)). TMA-58G^{bsr} cells were cultured in ES medium containing 3 to 4 µG/mL BS. ES cell lines and EG cell lines, which were used in the cell fusion experiments below, were within passage number 10.

### (2) Preparation of hybrid clones by cell fusion

### (2)-1. ES fusion cells

Thymus cells derived from the following 3 types of 6 to 8 week-old mice:
(A) 129/Sv-TgR (Rosa26) 26Sor (referred to as Rosa26) [Friedrich G. and Soriano P., GenESDev. 5:1513-1523 (1991)], which expresses a neo/lacZ reporter gene in cells of the whole the body;
(B) GOF-18/GFP (referred to as Oct4-GFP) [Yoshimizu T. et al. , Develp. Growth Differ. , 41:675-684 (1999)], whose totipotent and pluripotent cells specifically express GFP; and
(C) (Rosa26×Oct4-GFP) F1 transgenic mouse (hybrid mouse obtained by mating a female Rosa26 mouse with a male Oct4-GFP transgenic mouse [Yoshimizu T. et al., Develop. Growth Differ., 41:675-684 (1999)]), which contains both a neo/lacZ gene and an Oct4-GFP gene.

The Rosa26 mouse was identified by X-gal staining the tip of the tails thereof. The Oct4-GFP mouse was confirmed by PCR analysis of DNA from the tail thereof using the following primer: OCGOFU35, 5'-CTAGGTGAGCCGTCTTTCCA-3' (SEQ ID NO.: 1), and EGFPUS23, 5'-TTCAGGGTCAGCTTGCC GTA-3' (SEQ ID NO. : 2). A thymus obtained from the transgenic mouse was passed through a 18-gauge needle several times to obtain a suspension of single cells. As an ES cell, a TMAS-5 cell was used and mixed with the above-described 3 types of thymocytes at a ratio of 1:5 (ES cell: thymocyte), followed by washing in PBS three times. The cells were suspended in 0.3 M mannitol buffered solution at a concentration of 1×10⁶ cells/mL. A glass slide having a 1-mm electrode gap and Electro Cell Manipulator 2000 (BTX) were used to conduct electric fusion (E=2. 5 to 3.0 KV/cm) to prepare fusion cells. The fusion cells were cultured in ES medium for a day. Inactivated G418-resistant PEFs were selected in ES medium containing 250 µg/mL G418 in 7 to 10 days. Fusion cell clones were collected and spread (passage number 1) in ES medium supplemented with G418, and were cultured for 3 to 4 days. ES hybrid clones were subcultured in new medium every two days.

### (2)-2, ES×EG fusion cell

ES×EG fusion cells were produced as follows. NR2 ES cells and TMA-58G^{bsr} EG cells were mixed at a ratio of 1:1, and were suspended in 0.3 M mannitol buffered solution at a concentration of 1×10⁶ cells/mL. ES×EG fusion cells. were selected in ES medium containing 250 µg/mL G418 and 3 to 4 µg/mL BS in 7 to 10 days.

ES hybrid clones could be maintained at a proportion (2.8×10⁻⁴) similar to that of EG hybrid clones produced by the present inventors in previous research [Tada M. et al. , EMBO J. 16:6510-6520(1997)]. All types of fusion cells were similar to that of the parent ES cell, and no morphological change was found. Cytogenetic analysis using G-banding demonstrated a complete set of chromosomes including three X chromosomes and one Y chromosome in all of the 13 ES fusion cells and 2 ES×EG hybrid clones which were used in experimentation. Further, ES hybrid and ES×EG hybrid cell lines at passage number 2 to 4 were used in molecular analysis.

### (3) Confirmation of fusion

In order to confirm the fusion between ES cells and differentiated cells, 4 primer sets specific, respectively, to the D-J region of T cell receptor (Tcr) β, the D-J region of immunoglobulin (Ig) H, and the V-J regions of Tcrδ and Torγ were used to perform PCR amplification using DNA extracted from thymocytes and ES fusion cells as templates. For genomic DNA (0.5 µg) from an adult thymus, an ES cell and an ES hybrid clone, PCR amplification was performed to detect the rearrangement of each gene using the following primer sets:
(A) Dβ2-Jβ2 rearrangement of Tcrβ gene: Dβ2,5'-GTAGGCACCTGTGGGGAAGAAACT-3' (SEQ ID NO.: 3); Jβ2,5'-TGAGAGCTGTCTCCTACTATCGATT-3' (SEQ ID NO.: 4) [Levin S.D. et al., EMBO J. 12:1671-1680(1993)];
(B) D-J rearrangement of IgH gene: D µ,5'-ACAAGCTTCAAAGCACAATGCCTGGCT-3' (SEQ ID NO.: 5); J µ,5'-GGGTCTAGACTCTCAGCCGGCTCCCTCAGG-3' (SEQ ID NO.: 6) [Gu H. et al., Cell 65:47-54(1991)];
(C) Vγ7-Jγ1 rearrangement of Tcrγ gene: Vγ7,5'-CTCGGATCCTACTTCTAGCTTTCT-3' (SEQ ID NO.: 7); Jγ1,5'-AAATACCTTGTGAAAACCTG-3' (SEQ ID NO.: 8) [Livak F. et al., J. Immunol. 162:2575-2580(1999)]; and
(D) Vδ5-Jδ1 rearrangement of Tcrδ gene: Vδ5,5'-CAGATCCTTGCAGTTCATCC-3' (SEQ ID NO.: 9); Jδ1,5'-TCCACAGTCACTTGGGTTCC-3' (SEQ ID NO.: 10) [Wilson A. et al., Immunity 4:37-45(1996)].

PCR products were subjected to electrophoresis on 1.2% agarose gel, f ollowed by staining with ethidium bromide. The specificity of the PCR product was confirmed by Southern hybridization using: biotinylated Jβ2-specific oligoprobe (5'-TTTCCCTCCCGGAGATTCCCTAA-3' (SEQ ID NO.: 11) [Levin S.D. et al., EMBO J. 12:1671-1680(1993)]) for Tcrβ; and biotynylated JH4 oligoprobe (5'-CCTGAGGAGACGGTGACTGAGGTTCCTTG-3' (SEQ ID NO.: 12) [Ehlich A. et al., Cell 72:695-704(1993)]) for IgH.

The rearrangement of DNA is clear evidence indicating that a thymocyte has differentiated into a lymphoid cell [Fowlkes, B.J. and Pardoll D.M., Adv. Immunol. 44:207-264(1989)]. Rearrangement specific to TcrβD-Jβ2.1, 2.2, 2.3, 2.4, 2.5 or 2.6 was observed in 45% of the hybrid clones (Figure **1a**). Further, in several clones, similar rearrangement was observed in the D-J region of IgH (Figure **1b**), and the V-J regions of Tcrδ and Tcrγ (Figures **1c** and **1d**, respectively). Of the 31 ES hybrid clones studied, a total of 17 clones (55%) underwent rearrangement, which was, at least, researched. In these cases, it is considered that the ES cell were fused after differentiation of a thymocyte nucleus into a lymphoid cell.

### (4) X chromosome activity

In female somatic cells, one of the two X chromosomes is randomly inactivated due to dosage compensation of an X-linked gene. Inactivation of the X chromosome occurs in early cell division to induce the delay of transition of DNA replication into the late S phase, epigenetic changes including hypermethylation of DNA and low acetylation of histone H4 are known to occur. In cloned embryos obtained by nuclear transplantation of a somatic cell nucleus into an oocyte, the inactivated X chromosome of female somatic cells is reactivated [Eggan K. et al., Science 290: 1578-1581(2000)]. Therefore, activation of both X chromosomes serves as an indicator of the occurrence of reprogramming of a nucleus. In order to analyze the activity of X chromosomes, the present inventors studied using replication differential staining method [Sugawara O. et al., Chromosoma 88:133-138(1983)] (Figure **2a**).

### (4)-1. Timing of replication of X chromosome

Chromosome preparations of ES fusion cells and ES×EG fusion cells described in the above-described section (2) were produced by culturing the cells along with 150 µg/mL 5-bromo-2-deoxy uridine (BrdU) for 7 hours, where the cells were cultured for the last hour in the presence of 0.3 µg/mL colcemide. The cells were then subjected to hyposmotic treatment with 0. 075M KCl at room temperature for 8 minutes. Thereafter, the cells were fixed by immersing in methanol:acetic acid (3:1) solution three times, followed by air drying. The cells were stained by freshly prepared acridine orange solution. The slide was observed under a fluorescence microscope with a standard B filter. After continued incorporation of BrdU at the late stage of the S period and acridine orange staining, active X chromosomes and autosomes were observed as red and green banded factors. Inactive X chromosomes were uniformly dark-red stained in female somatic cells due to the delay of replication (Figure **2b**). Among all 32 cells (4n=80) whose karyotypes were determined, 6 fusion cell clones of an XY female ES cell and an XX female thymocyte carried three X chromosomes which were simultaneously replicated (Figure **2c**).

### (4)-2. Xist RNA FISH

Probes prepared by nick translation of a mixture of Xist cDNA clones, which contained a series of exons 1 to 7, using cy3-dUTP (Amersham Pharmacia) [Sado T. et al., Development 128:1275-1286(2001)] were used and hybridized with chromosome preparations obtained as in 4-1. Hybridization and subsequent washing were performed as previously described [Lawrence J.B. et al., Cell 57: 493-502(1989)]. The result was in agreement with the result obtained in (4)-1, i.e., Xist (inactive X specific transcript) RNAwas not stably accumulated (spotted) on three X chromosomes in two ES hybrid cell lines tested by RNA FISH (fluorescence in situ hybridization) (Figure **2d**). Xist accumulation was also instable on active X chromosomes of male ES cells, and was stable on inactive X chromosomes of female thymocytes (colored signal). Somatic cell nucleus-derived inactive X chromosomes acquired several properties of active X chromosomes after hybridization and had replication andXist expression patterns similar to those observed in undifferentiated cells. Changes in replication timing and Xist RNA accumulation of X chromosomes of somatic cells in ES fusion cells shown in the above-described (4)-1 and (4)-2 suggest that somatic cell nuclei were reprogrammed after cell fusion.

### (5) Reprogramming of somatic cell nucleus

A mouse line having an Oct4-GFP transgene was used to visualize the reprogramming of somatic cell nuclei (Figure **3**). Expression of Oct4 was specifically observed in germ cells, embryos before implantation, and ectoblasts of early embryos before implantation. Therefore, the activity of Oct4 can be used as an ideal marker for identification of totipotency and/or pluripotent cells. The expression pattern of Oct4-GFP is known to be comparable to the expression pattern of endogenous Oct4 [Yoshimizu T. et al., Develop Growth Differ. 41:675-684(1999)]. Expression of Oct4-GFP was examined for the thymus and ovary of Oct4-GFP transgenic mice. GFP was detected in the growing ovary, but not in the thymus (Figures **3a** to **d**).

Thymus cells of Oct4-GFP transgenic mice were fused with ES cells, followed by culturing without screening. Expression of GFP was examined every 12 hours. Expression of GFP in living ES fusion cells on a culture dish was investigated under a dissecting microscope (Leica) with a GFP exciting source and a GFP filter. After 48 hours, a GFP positive colony consisting of 16 cells was observed at the periphery of a larger non-expressing colony (Figures 3e, f ). Subsequently, several other GFP positive colonies were observed on the same culture plate before reaching confluency. No GFP positive cells were observed among non-fusion thymocytes cultured under the same conditions. In order to investigate whether or not somatic cell nuclei can be reprogrammed in all ES fusion cells, thymocytes of G418 selection-resistant (Rosa26×Oct4-GFP) F1 mouse were used. After screening, 36 of the resultant 37 clones expressed GFP (97%). The expression was stably maintained even after subculturing over some passages (Figures **3g, h**), indicating that the thymocyte nucleus was reprogrammed in most of the ES fusion cells. The Oct4-GFP transgene, which was suppressed in thymocytes before cell fusion, was reactivated in the ES fusion cells. This further supports the result of (4) that after cell fusion, somatic cell nuclei were reprogrammed.

### (6) Introduction into blastocysts

Normal diploid blastocysts were used to produce chimeric embryos with ES fusion cells. Diploid blastocysts were obtained from the uteruses of Day 3.5 pregnancy ICR females mated with ICR males. Hybrid clones with the above-described (Rosa26×Oct4-GFP) F1 mouse-derived (differentiated) thymocytes and hybrid clones with Rosa26 mouse-derived thymocytes were used as tetraploid fusion cells. The tetraploid fusion cells were microinjected into the blastocoele pores of well expanded blastocysts (Figure **4a**). These blastocysts were transferred into the uteruses of pseudopregnant ICR females. Chimeric embryos were removed from the E7.5 uteruses, followed by removal of the Reichert membrane, and β-galactosidase staining and histological analysis.

### (6)-1. β-galactosidase active staining

By β-galactosidase active staining, the relative contribution of a fusion cell to each chimera was confirmed. Cultured cells were washed with PBS, and fixed using PBS containing 1% formaldehyde, 0.2% glutaraldehyde, 0.02% NP40 and 1 mM MgCl₂, at 4°C for 5 minutes. The same fixing solution was used to fix embryos and mouse tails at 4°C for 3 to 4 hours. The samples were washed with PBS, followedbystaining with a reaction mixture containing 1 mg/mL 4-Cl-5-Br-Indolyl-β-galactosidase (X-gal) with dimethyl formamide, 5 mM potassium ferricyanide, 5 mM potassium ferricyanide, and 2 mM MgCl₂ in PBS at room temperature for 24 to 48 hours.

### (6)-2. Histological analysis

E7.5 embryos stained with X-gal were dehydrated with an ascending alcohol series, and embedded in JB-4 plastic resin (polyscience, Warrington, PA). Ultrathin sections (2-4 µm thick) were negative stained with 0.25% eosin Y. Four week old teratoma fixed and embedded in paraffin were cut into 8-µm thick sections. The serial sections were stained with hematoxylin and eosin.

As a result, 8 of 20 E7.5 embryos were positive, indicating the limited contribution of the fusion cell (Figures **4b, c**). Detailed analysis revealed derivatives from the fusion cell in the embryonic ectoderm, embryonic mesoderm, and the internal organ endoderm (Figures **4d, e**). As described above, the ES fusion cell had a developmental capability of differentiating into three primordial germinative layers (ectoderm, mesoderm, and endoderm) in early embryos before implantation.

### (7) Methylation of DNA

Next, DNA of thymocytes, ES cells and prepared hybrid clones was investigated by Southern blot hybridization as to whether or not the reprogramming of a thymocyte nucleus has an influence on the methylation of an imprinted genetic locus. Southern blot hybridization was performed by separating genomic DNA digested with a restriction enzyme into fractions using 0.8% agarose, transferring the fractions onto a Hybond N+membrane (Amersham) by alkali blotting, and hybridizing DNA with a ³²P-dCTP-labeled probe.

### (7)-1. H19 genetic locus

It is considered that a paternal methylated region is incorporated upstream of the maternal H19 genetic locus to be expressed, so that primordial methylation imprinting is maintained [Tremblay K.D. et al., Nature Genet. 9:407-413(1995)]. DNA samples of thymocytes, ES cells, and prepared hybrid clones were digested with BamHI and a methylation sensitive restriction enzyme HhaI. A 3.8-kb SacI probe and a 2. 7-kb BamHI probe were used to detect 10-kb and 2.7-kb paternal methylated fragments and 7.0-kb and 1.8-kb maternal unmethylated fragments in DNAs from both the thymocyte and the ES cell. The same pattern was found in the hybrid clone. There was no difference in the relative intensity of bands between the methylated (RI=0.60) and unmethylated fragment (RI=0.40) bands (Figure **5a**). Similar results were observed using BamHI probes, in which 2.7-kb paternal methylated fragments and 1. 8-kb and 0.8-kb maternal unmethylated fragments were identified. For all samples, methylated (RI=0.55) and unmethylated (RI=0.45) bands were similarly detected (Figure **5a**).

### (7)-2. Igf2r genetic locus

Probes for analysis of methylation of Igf2r region 2 were produced by PCR using the following primers: 5'-AATCGCATTAAAACCCTCCGAACCT-3' (SEQ ID NO.: 13) and 5'-TAGCACAAGTGGAATTGTGCTGCG-3' (SEQ ID NO.: 14) [Stoger R. et al., Cell 73:61-71(1993)]. A CpG island, which is an intron of the Ifg2r gene and is known to be imprinted with methylation, is methylated only on an allele [Stoger R. et al., Cell 73:61-71(1993)]. As in the above-described section (7)-1, each DNA sample was digested with PvuII and a methylation sensitive restriction enzyme MluI. With the 330-bp Igf2r CpG island probe, a 2. 9-kb maternal methylated fragment and a 2.0-kb paternal unmethylated fragment were detected in DNAs from both the thymocyte and the ES cell (Figure **5b**). The same pattern was also found in the hybrid clone. There was no difference in relative intensity (RI) between methylated (RI=0.55) and unmethylated (RI=0.45) bands (Figure **5a**).

According to sections (7)-1 and (7)-2, it was demonstrated that the primordial methylation of the H19 upstream region and the Igf2r intron region of the genome of a thymocyte are not affected by fusion with an ES cell. This results differs from previous observation of a hybrid clone of a thymocyte and an EG cell derived from a germ cell PGC of an E12.5 mouse in which the maternal specific methylation of Igf2r disappeared [Tada M. et al., EMBO J. 16: 6510-6520(1997)]. The maintenance of the somatic cell methylation pattern in ES fusion cells suggests that ES cells and EG cells have different control mechanism for regulating the DNA methylation of imprinted genes. While the maternal allele specific methylation of Igf2rwas observed in ES cells, it was not observed in EG cells and a control 1:1 mixture of ES cell DNA and EG cell DNA at a ratio of about 1:3 (methylation/RI=0.27, unmethylation/RI=0.73). In the ES×EG hybrids, methylation bands disappeared (Figure **5c**), indicating that demethylation activity in EG cells is dominant over the maintenance of methylation imprinting in ES cells.

### 2. Production of teratoma

Fusion cells of TMAS-5ES cells and Rosa26-derived thymocytes, which were produced with a method similar to that for production of chimeric embryos in section 1, were used as tetraploid fusion cells. About 100 million to 500 million tetraploid fusion cells were subcutaneously injected into the posterior limb inguinal region of a SCID mouse (CLEA Japan KK). Four weeks after subcutaneous injection, teratoma was collected. By X-gal staining, it was determined that the teratoma was derived from the fusion cell. Thereafter, the teratoma was fixed in Bouin's fixative, followed by Haematoxyline-Eosin (HE) staining to stain both the nucleus and cytoplasm. As a result of HE staining, muscles, cartilages, epithelial cells, and neurons were observed (Figure 6).

### (Example 2: Identification and use of reprogramming agent)

### (Fusion cell)

Electric fusion, culture of ES cells and fusion cells, and analysis of the chromosomes were conducted in accordance with standard procedures well known in the art (Tada, M., Tada, T., Lefebvre, L., Barton, S.C. & Surani, M.A., EMBO J., 16, 6510-6520 (1997)), specifically, as follows.

To make two types of fusion cells, the present inventors used two types of ES cell lines, the domesticus XY ES cell line deficient for the Hprt gene on the X chromosome and the molossinus XY ES cell line deficient for MP4, which were established in accordance with "Manipulating the Mouse Embryo: A Laboratory Manual 2nd Edition" edited by Brigid Hogan, Rosa Beddington, Frank Castantini and Elizabeth Lacy, pp 253-290, Cold Spring Harbor (USA). Specifically, blastocysts were washed away from the uterus of a 3.5 day old female mouse after mating. The resultant blastocysts were cultured on mouse primary fibroblasts (PEF) inactivated with mitomycin C. ES culture medium was used, which was MEM+F12 medium (Sigma) supplemented with 15% fetal bovine serum, antibiotics, L-glutamine, sodium bicarbonate, sodium pyruvate, mercaptoethanol, leukemia inhibitory factor (LIF) ("Manipulating the Mouse Embryo: A Laboratory Manual 2nd Edition" edited by Brigid Hogan, Rosa Beddington, Frank Castantini andElizabethLacy, pp 253-290, Cold Spring Harbor (USA)). After 5 days of culture, proliferative cells derived from the internal cell mass of blastocysts were separated by trypsin treatment, and were cultured on new PEF, thereby purifying ES cells.

The fusion cell lines, HxJ-17 and 18 were obtained by electric fusion between the domesticus Hm1 ES cells and thymocytes from the molossinus JF1 mice as follows. Mannitol buffer (0.3 M) suspension containing the ES cells and the somatic cells was prepared. The suspension was placed on a fusion slide having an electrode gap of 1 mm. Alternating current was applied to the suspension at 10 V for 60 seconds, followed by applying direct current at 250 V for 10 microseconds. Thereafter, the processed mixture was cultured on PEF feeder cells in ES culture medium ( "Takinokansaibo no Saipuroguramukakassei, In Jikken Igaku Bessatsu Posutogenomujidai no Jikkenkoza 4; Kansaibo · Kuron Kenkyu Purotokoru [Reprogramming Activity of Pluripotent Stem Cell, In Experimental Medicine, Special Issue, Experimental Lecture 4 in Postgenome era; Stem cell · Clone Research Protocol]", pp 191-198, Yodo-sha (Tokyo), etc.). The above-described ES culture medium was supplemented with hypoxanthine, aminopterin, and thymidine (HAT) selective reagents to obtain HAT selective culture medium. The above-described fusion cell was obtained after 8 days of HAT selective culture.

Other fusion cell lines, MxR-2 and Mxr-3, were obtained by electric fusion of molossinus MP4 ES cells and thymocytes from domesticus 129/Sv-Rosa26 transgenic mice (lacZ/neo is globally expressed in the mouse) as follows. Mannitol (0.3 M) buffer suspension containing the ES cells and the somatic cells was prepared. The suspension was placed on a fusion slide having an electrode gap of 1 mm. Alternating current was applied to the suspension at 10 V for 60 seconds, followed by applying direct current at 250 V for 10 microseconds. Thereafter, the processed mixture was cultured on PEF feeder cells in ES culture medium ("Takinokansaibo no Saipuroguramukakassei, In Jikken Igaku Bessatsu Posutogenomujidai no Jikkenkoza 4; Kansaibo · Kuron Kenkyu Purotokoru [Reprogramming Activity of Pluripotent Stem Cell, In Experimental Medicine, Special Issue, Experimental Lecture 4 in Postgenome era; Stem cell · Clone Research Protocol]", pp 191-198, Yodo-sha (Tokyo), etc.). The above-described ES culture medium was supplemented with Geneticin (Sigma) selective reagent to obtain G418 selective culture medium, in which the cells were in turn cultured. After 8 days of G418 selective culture, the above-described fusion cell was obtained (Tada, M., Takahama, Y., Abe, K., Nakatsuji, N., and Tada, T. (2001), Curr. Biol., 11, 1553-1558).

### (Immunohistochemistry)

X-gal staining of cells and tissues was conducted by standard procedures (Tada, M., Tada, T., Lefebvre, L., Barton, S.C. & Surani, M.A. , EMBO J., 16, 6510-6520 (1997)), specifically, as follows.

To form teratomas from four fusion cell clones (HxJ-17, HxJ-18, MxJ-2, and MxJ-3), about 1×10⁶ cells of each clone were subcutaneously injected into the inguinal region of the immunodeficient SCID mice. Teratoma formation was found in all sites 4-5 weeks after injection. Teratomas, culture cells and graft tissues of mouse brains fixed with 4% PFA were used for the following immunoreaction with antibodies: rabbit anti-TuJ (Babco), mouse anti-Nestin (BD PharMingen), rabbit anti-TH (CHEMICON), mouse anti-NF-M (CHEMICON), rat anti-Ecad (TAKARA), goat anti-β -Gal (Biogenesis) and mouse anti-Desmin (DACO). X-gal staining of cells and tissues was performed by standard procedures.

### (Genomic PCR, RT-PCR and sequencing)

To detect D-J DNA rearrangements of Tcrβ and IgH in fusion clones, genomic DNAs were amplified at the 65°C annealing temperature for 30 cycles of PCR reactions with the primer sets described below. One cycle included: 95°C for 30 seconds (denaturing); 65°C for 30 seconds (primer annealing); and 72°C for 30 seconds (elongation using Taq enzyme). Thirty cycles of DNA PCR amplification were conducted.

Tcrβ, Dβ2 (5'-GTAGGCACCTGTGGGGAAGAAACT) (SEQ ID NO.: 15) and
Jβ2 (5'-TGAGAGCTGTCTCCTACTATCGATT) (SEQ ID NO.: 16);

IgH, Dμ (5'-ACAAGCTTCAAAGCACAATGCCTGGCT) (SEQ ID NO.: 17)
and Jμ (5'-GGGTCTAGACTCTCAGCCGGCTCCCTCAGGG) (SEQ ID NO.:
18).

To analyze gene expression, cDNA was synthesized from total RNA with oligo-dT primers. In the case of Pitx and Nestin cDNA detection, LA Tag polymerase in GC buffer 2 (TAKARA) was used to counter the high GC content. Primer sequences, the annealing temperature for 30 cycles of PCR reactions and the length of amplified products were as follows:

Albumin, 55°C, 567 bp, 5'-AAGGAGTGCTGCCATGGTGA (SEQ ID
NO.: 19), 5'-CCTAGGTTTCTTGCAGCCTC (SEQ ID NO.: 20);

α-Fetoprotein, 55°C, 342 bp, 5'-TCGTATTCCAACAGGAGG (SEQ ID
NO.: 21), 5'-CACTCTTCCTTCTGGAGATG (SEQ ID NO.: 22);

Desmin, 55°C, 361 bp, 5'-TTGGGGTCGCTGCGGTCTAGCC (SEQ ID

NO.: 23), 5'-GGTCGTCTATCAGGTTGTCACG (SEQ ID NO.: 24);

TH, 60°C, 412 bp, 5'-TGTCAGAGGAGCCCGAGGTC (SEQ ID NO.: 25),
5'-CCAAGAGCAGCCCATCAAAG (SEQ ID NO.: 26);

Nestin, 55°C, 327 bp, 5'-GGAGTGTCGCTTAGAGGTGC (SEQ ID
NO.: 27), 5'-TCCAGAAAGCCAAGAGAAGC (SEQ ID NO.: 28);

Nurr1, 55°C, 253 bp, 5'-TGAAGAGAGCGGACAAGGAGATC (SEQ ID
NO.: 29), 5'-TCTGGAGTTAAGAAATCGGAGCTG (SEQ ID NO.: 30);

NF-M, 55°C, 186 bp, 5'-GCCGAGCAGACCAAGGAGGCCATT (SEQ ID
NO.: 31), 5'-CTGGATGGTGTCCTGGTAGCTGCT (SEQ ID NO.: 32);

Pitx3, 55°C, 373 bp, 5'-AGGACGGCTCTCTGAAGAA (SEQ ID NO.: 33),
5'-TTGACCGAGTTGAAGGCGAA (SEQ ID NO.: 34);

G3pdh, 55°C, 983 bp, 5'-TGAAGGTCGGTGTGAACGGATTTGGC (SEQ ID
NO.: 35), 5'- CATGTAGGCCATGAGGTCCAC (SEQ ID NO.: 36);

MyoD, 60°C, 397 bp, 5'-GCCCGCGCTCCAACTGCTCTGAT (SEQ ID
NO.: 37), 5'-CCTACGGTGGTGCGCCCTCTGC (SEQ ID NO.: 38);

Myf-5, 60°C, 353 bp, 5'-TGCCATCCGCTACATTGAGAG (SEQ ID
NO.: 39), 5'-CCGGGTAGCAGGCTGTGAGTTG (SEQ ID NO.: 40).

For the direct cloning of PCR products into a plasmid vector, the TA cloning Kit (Invitrogen) was used. The cDNA sequence of a single plasmid clone was independently analyzed by using M13 reverse and forward primers.

### (Neural differentiation and cell graft)

To produce TH-positive neuron at high efficiency, ES and fusion cells were cultured for 8 to 11 days on PA6 stromal cells (Kawasaki, H., Mizuseki, K., Nishikawa, S., Kaneko, S., Kuwana, Y., Nakanishi, S., Nishikawa, S.I., and Sasai, Y. (2000), Neuron, 28,31-40), threafter the cells were washed three times with MEM medium to remove serum and LIF. For transplantation of the differentiated TH-positive colonies, PA6 feeder layer was isolated by treatment with papain, and then slowly injected into the mouse striatum by using a blunt-ended 26G Hamilton syringe (Kawasaki, H., Mizuseki, K., Nishikawa, S., Kaneko, S., Kuwana, Y., Nakanishi, S., Nishikawa, S.I., and Sasai, Y. (2000), Neuron, 28,31-40). About 5×10⁵ fusion cell-derived TH-positive cell suspension was grafted to each injected site. Two weeks later, the whole brains were frozen for making frozen sections after 4% PFA fixation.

### (Results)

The acquired capability of reprogramming a somatic cell nucleus by cell fusion is a significant feature possessed by pluripotent ES cells (Tada, M., Takahama, Y., Abe, K., Nakatsuji, N., and Tada, T. (2001), "Nuclear reprogramming of somatic cells by in vitro hybridization with ES cells", Curr. Biol., 11, 1553-1558). Similarly, nerve spherocytes and bone marrow cells spontaneously undergo cell fusion with ES cells by culturing together, so that the nuclei thereof are reprogrammed. The pluripotent cell-specific marker gene Oct4-GFP and the reactivation of an inactive X chromosome are indicators at least partially showing that the somatic cell of a fusion cell is reprogrammed into an undifferentiated state.

The result that teratomas and chimeras were successfully formed demonstrated that the fusion cells have pluripotency. Reprogrammed somatic cell genomes may have pluripotency or may be genetically dormant in the course of redifferentiation.

If reprogrammed somatic cell genomes acquire pluripotency as in ES cell genomes, tailor-made ES cells suitable for individuals can be obtained by cell fusion without therapy cloning.

Inter-subspecific fusion cells of Mus musuculus domesticus Hm1 ES cells and M.m.molossinus JF1 thymocytes (HxJ), and also of molossinus MP4 ES cells and domesticus Rosa26 thymocytes (MxR) were made for the following experiments (Figure **7A**). The reporter gene, lacZ/neo in somatic genomes derived from the Rosa26 transgenic mouse is ubiquitously expressed (Friedrich, G. & Soriano, P., GenES & Dev. , 5, 1513-1523(1991)). The Hm1 ES cells deficient for the X chromosome-linked Hprt gene and the MP4 ES cells were newly established from molossinus blastocysts. DNA sequence polymorphism was easily found in the molossinus genomes when compared with the domesticus genomes (ref5). Full sets of domesticus and molossinus-derived chromosomes were maintained in the fusion clones the present inventors examined (Figure **7B**). To test differentiation capability of the ES fusion cells with adult thymocytes, the HxJ and MxR fusion cells were subcutaneously injected into the inguinal region of immunodeficient SCID mice. A half piece of teratomas 4 to 5 weeks after injection of the MxR-2 and 3 fusion cell lines, in which the fusion protein of lacZ/neo was ubiquitously expressed, was positive for X-gal staining. Thus, tissues containing the teratomas were derivatives of the fusion cells. Immunochemical analysis of the sections revealed expression of the Class III β-Tublin (TuJ), Neurofilament-M (NF-M), Desmin and Albumin proteins (Figure **7D**), indicating that the fusion cells retain the capability to differentiate into ectodermal, mesodermal and endodermal lineages in vivo, even if the somatic cells were mesodermal progeny. DNA rearrangements of T cell receptors and/or Immunoglobulin H genes specific to lymphoid cells in all fusion clones the present inventors used revealed that the somatic cells were mesodermal derivatives (Figure **7C**). Further histological analysis with hemotoxylin-eosin staining showed that the teratomas contained other tissues including cartilage, ciliated epithelium and gland. This multi-lineage differentiation of the fusion cells was confirmed by the tissue-specific mRNA of the paired-like homeodomain transcription factor 3 (Pitx3), Albumin, α-fetoprotein, MyoD, Myf-5 and desmin genes (Figure **8A**). The lack of expression of these genes in undifferentiated ES cells and fusion cells suggests that their expression was induced by cell type-specific differentiation in teratomas.

To address whether mRNA of tissue-specific genes was transcribed from the reprogrammed somatic genomes in the tissues, RT-PCR products of Pitx3, Albumin andMyoD amplified with the domesticus and molossinus cells and their inter-subspecific fusion cell-derived teratoma cDNAs were sequenced and compared. In Pitx3, a single base replacement of the guanidine (G) residue in domesticus genomes to the adenine (A) residue in molossinus genomes was found at the position 322 of mRNA (accession no. 008852). Out of 12 clones sequenced, 5 and 7 were domesticus and molossinus type sequences, respectively. A roughly equal amount of products was amplified from the somatic genome RNAs (Figure **8B**). A similar pattern was detected in transcription of the endodermal cell-specific gene, Albumin. RT-PCR products at 567 bp from the domesticus mRNA were digested with the restriction enzyme NcoI and detected at 554 bp and 13 bp, whereas RT-PCR products from the molossinus mRNA were detected as three bands at 381, 173 and 13 bp after digestion with NcoI (Figure **8C**). An equal level of Albumin expression from ES genomes and somatic genomes was recognized by the similar intensity of bands in the teratomas differentiated from the HxJ and MxR inter-specific fusion cell lines. In themuscle-specificregulatoryfactorMyoD, transcripts from ES genomes and somatic genomes were distinguished by the sequence polymorphism sensitive to the BssHI digestion. RT-PCR products at 395 bp from the domesticus mRNA was separated to two bands at 293 and 102 bp, whereas that from the molossinus mRNA was resistant to the digestion and was detected as an intact 395 bp band (Figure **8D**). In the HxJ-17 and 18 and MxR-2 and 3 inter-subspecific fusion cell lines, somatic genome-derived transcripts were found to be similar to ES genome-derived transcripts. These data indicated that similarly to ES genomes, reprogrammed somatic genomes gained nuclear competency capable of transcribing tissue-specific mRNAs in teratomas differentiated in vivo.

The next question is whether the somatic genomes in the fusion cells could differentiate to a specific cell type under in vitro differentiation-inducing conditions. To do this, the present inventors used the system of neural differentiation induced by co-culture on PA6 stromal cells in serum-free conditions (Kawasaki, H. et al., Neuron, 28, 31-40 (2000)). Host MP4 ES cells andMxR-3 fusion cell clones were cultured to promote neural differentiation for 8 to 11 days (Figures **9A, B**). By this induction, most colonies from the fusion cells and the host ES cells were positively immunoreactive for neuroepithelial stem cell-specific Nestin and postmitotic neuron-specific TuJ. The colonies positive for stem cell-specific E-Cadherin were rarely found. These data indicate that pluripotency of the fusion cells was recapitulated and the fusion cells were controlled to differentiate effectively into the neural lineage. Thus, to improve the efficiency of producing dopaminergic neurons, the period of induction culture was prolonged to 11 days. The majority of cells were positively stained with antibodies against TuJ and NF-M. Cells immunoreactive to tyrosin hydroxylase (TH), which is required for the production of catecholamine neurotransmitters, were mainly detected in the inside of all surviving colonies. Roughly 20 to 50% of cells per colony were detected as TH-positive (Figure **9C**). A similar pattern was found when host ES cells were used. This effective neuronal differentiation succeeded in 5 repeated experiments. Production of the mesencephalic dopaminergic neurons in vitro was reconfirmed by transcription of TH, Nurr1 and Pitx3 amplified by RT-PCR with mRNA extracted from fusion clones 11 days after differentiation induction (Figure 9D). To examine whether tissue-specific genes were expressed by the reprogrammed somatic genomes was similar to the ES genomes, sequencing was performed with RT-PCR products of Pitx3, which is a transcriptional activator of TH. In a single base replacement of the domesticus guanidine (G) residue to the molossinus adenine (A) residue of Pitx3 mRNA, out of 12 clones sequenced, 8 clones were found as the molossinus type while 4 clones were the domesticus type in the dopaminergic neurons differentiated from the MxR inter-subspecific fusion clone (Figure **9E**). Similar results were obtained with the dopaminergic neurons differentiated from HxJ fusion clones. Thus, somatic genomes, which were reprogrammed in the fusion cells, acquired pluripotential competency capable of expessing the mesencephalic dopaminergic neuron-specific transcripts by in vitro differentiation.

The present inventors next examined whether the fusion cell-derived dopaminergic neurons, which were induced to be differentiated in vitro for 11 days have potential to integrate into the striatum of mouse brain after transplantation. In this experiment, the MxR-3 fusion cells between molossinus ES cells and domesticus somatic cells obtained from Rosa26 carrying lacZ/neo. About 5×10⁵ cells per site of fusion cell-derivatives were injected to the striatum at A=+1.0 mm, L=+2.0 mm, V=+3.0 mm by the bregma as a reference in the two mouse brains (Figure **10A**). Survival of cells in the grafts, was detected by X-gal staining as blue-positive cells 15 days after injection. Immunohistochemical double staining analysis of frozen sections of the grafts with antibodies against TH and LacZ clearly demonstrated that the fusion cell-derived neural cells were expressing a dopaminergic neuron-specific TH protein at the injection of site (Figures **10B** and **C**). Thus, the somatic genomes were capable of expressing neuron-specific genes even after the fusion cells were induced to be differentiated in vitro to the specific type of cells. These data indicate the possibility that the fusion cells could be used for producing replacement tissues in therapeutic applications for diseases and aging.

ES cells are self-renewal and pluripotential cells, which are capable of differentiating to a variety of tissues including germ cells following their introduction to recipients. Thus, their pluripotential property is suitable as a cell source for making many types of replacement tissues by in vitro differentiation induction. However, tissues provided from an ES cell source would be mismatched to the majority of recipients, resulting in non-self transplantation rejection. Thus, a key issue of therapeutic transplantation is how to reduce immunological rejection against ES-derived grafts. To produce autologous transplants, personal tailored ES cells or personal adult tissue stem cells are appropriate as a cell source. Personal adult tissue stem cells are a strong candidate cell source (Jiang, Y., Jahagirdar, B.N., Reinhardt, R.L., Schwartz, R.E., Keene, C.D., Ortiz-Gonzalez, X.R., Reyes, M., Lenvik, T., Lund, T., Blackstad, M., Du, J., Aldrich, S., Lisberg, A., Low, W.C., Largaespada, D.A. , and Verfaillie, C.M. (2002), "Pluripotency of mesenchymal stem cells derived from adult marrow", Nature, 418, 41-49). Personal tailored ES cells may be made with cloned blastocysts produced by nuclear transplantation of somatic cells to enucleated unfertilized eggs (Rideout, W.M. , 3rd, Hochedlinger, K., Kyba, M., Daley, G.Q. & Jaenisch, R., Cell, 109, 17-27 (2002)). However, human therapeutic cloning encounters social issues of biomedical ethics (Weissman, I.L., N. Engl. J. Med., 346, 1576-1579 (2002)). To avoid the ethical issues, the pluripotency of reprogrammed somatic genomes opens up at least three possibilities: 1) fusion cells having personal somatic genomes and ES genomes deficient for MHC class I and class II could be semi-matched to individual patients; 2) genetically tailored ES-like cells (pluripotent cells) may be made by targeted elimination of the ES genomes following cell fusion with personal somatic cells; and 3) personal somatic cells can be reprogrammed by genetic manipulations of reprogramming agents identified from ES cells. The cell fusion technology might have potential to make an important contribution in personal therapeutic applications without raising the social problems which has occurred with cloning.

### (Example 3: Identification of reprogramming agents)

In Example 3, the present inventors investigated how somatic genomes are epigenetically modified when fused with ES cells, and analyzed reprogramming agents and their mechanism. The present inventors expected that cell fusion with ES cells causes a dramatic change in the chromatin structure of somatic cell genomes, and analyzed the histone acetylation of somatic cell nuclei in inter-subspecific fusion cells (domesticus×molossinus). Experiments below were conducted in accordance with Forsberg et al., Proc. Natl. Acad. Sci. USA, 97, 14494-99 (2000). Actual protocols were in accordance with Upstate biotechnology, Chromatin immunoprecipitation protocol, from which antibodies were obtained.

### (Modification assay for nuclear histone)

An anti-acetylated histone H3 antibody, an anti-acetylated histone H4 antibody, an anti-methylated histone H3-Lys4 antibody, and an anti-methylated histone H3-Lys9 antibody were used to grasp modification of the nuclear histone of somatic cells, ES cells, and fusion cell. Next, these four antibodies were used to perform chromatin immunoprecipitation in order to analyze the interaction between histone and DNA.

DNA-histone protein complexes were recovered by reaction with the respective antibodies. By PCR amplification of DNA contained in the recovered DNA-histone protein complexes, it was revealed how histone was modified in what DNA region.

2×10⁷ to 10⁸ cells were cultured per assay.

On Day 1, 270 µl of 37% formaldehyde was directly added per 10 mL of culture medium to a final concentration of 1%. Thereafter, the mixture was shaked at 37°C for 10 minutes at a rate of 60 strokes/min.

Next, glycine was added to a final concentration of 0.125 M to stop the cross-linking reaction, and allowed to stand for 5 minutes at room temperature.

For adherent cells, the medium was removed, and plates were rinsed with ice-cold PBS (containing 8 g NaCl, 0.2 g KCl, 1.44 g Na₂HPO₄ and 0.24 g KH₂PO₄ /liter (pH 7.4), and 1 mM PMSF). Two mL of trypsin was added per dish. Thereafter, trypsin was aspirated from the dish. The dish was incubated at 37°C for 10 minutes. Trypsinaization was stopped by adding 1% FCS-PBS. The cells were centrifuged and the resultant cell pellet was washed with ice-cold PBS twice. For suspension cells, the cells were centrifuged and the resultant cell pellet was washed with ice-cold PBS twice.

Next, the cell pellet was resuspended in 10mL of cell lysis buffer (10 mM Tris-HCl (pH8.0), 10 mM NaCl, 0.2% Nonidet P-40, 10 mM sodium lactate (to avoid deacetylation) and protease inhibitor (1 tablet of complete protease inhibitor (Roche; Cat No. 1697498) was dissolved in 1 mL of sterilized water, diluted 1/100 for use)). No clump of cells was confirmed, followed by incubation on ice for 10 minutes. The suspension was centrifuged at 5000 rpm at 4°C for 5 minutes.

The pellet of nuclei was resuspended in nucleus lysis buffer (50 mM Tris-HCl (pH 8.1), 10 mM EDTA, 1% SDS, 10 mM sodium lactate and protease inhibitor (1 tablet of complete protease inhibitor (Roche; Cat No. 1697498) was dissolved in 1 mL of sterilizedwater, diluted 1/100 for use)), followed by incubation on ice for 10 minutes.

Next, the sample was sonicated so that the length of chromatin was averaged to 500 bp. In this step, the power was 10 to 15% of maximum, 40 seconds for 8 times. The sample was centrifuged at 15000 rpm at 4°C for 5 minutes. The supernatant (200 µl) was transferred into a new tube. At this point, sonicated chromatin was optionally stored at -70°C.

The resultant sample was diluted in 10 fold of ChIP dilution buffered solution (16.7 mM Tris-HCl(pH8.1), 167 mM NaCl, 1.2 mM EDTA, 0.01% SDS, 1.1% Triton X-100, 10 mM sodium lactate, and protease inhibitor (1 tablet of complete protease inhibitor (Roche; Cat No. 1697498) was dissolved in 1 mL of sterilized water, diluted 1/100 for use)).

To reduce non-specific background, the chromatin sample was cleared by adding 80 µl of Sarmon sperm DNA/protein A agarose. The sample was incubated at 4°C for 1 hour while rotating. Thereafter, the sample was centrifuged at 1000 rpm at 4°C for 1 minute. The resultant supernatant was transferred to a new tube.

Next, various antibodies were added to the resultant supernatant (anti-acetylated histone H3 antibody (K9&14, Upstate biotechnology, New York, USA; Cat No. #06-599) 10 µl (5 µl of antibody solution was used for 2 mL of reaction solution); anti-acetylated histone H4 antibody (Upstate biotechnology, New York, USA; Cat No. #06-598) 5 µl (5 µl of antibody solution was used for 2 mL of reaction solution); anti-dimethylated histone H3(K4 or K9) 5 µl (5 µl of antibody solution was used for 2 mL of reaction solution) (Upstate biotechnology, New York, USA; Cat No. #07-030 or #07-212, respectively). Typically, the required amount of each antibody was 1 µg. As negative control, samples without any antibody were tested.

The resultant sample was rotated at 4°C overnight.

On Day 2, 60 µl of Sarmon sperma DNA/protein A agarose (Upstate biotechnology (Cat No. 16-157), (60 ML of Sarmon sperm DNA/protein A agarose was used for 2 mL of reaction solution) was added to the sample. The sample was rotated at 4°C for 1 hour, followed by centrifugation at 1000 rpm at 4°C for 1 minute. The supernatant was transferred into a new tube. The supernatant from the "no-antibody" sample was stored as "total input chromatin".

Next, the pellet was washed twice with 1 mL of low salt wash buffer (20 mM Tris-HCl (pH 8.1), 150 mM NaCl, 2 mM EDTA, 0.1% SDS, 1% Triton X-100), followed by rotation at room temperature for 5 minutes. Next, the pellet was washed with 1 mL of high salt wash buffer (20 mM Tris-HCl (pH 8.1), 500 mM NaCl, 2 mM EDTA, 0.1% SDS, 1% Triton X-100), followed by rotation at room temperature for 5 minutes. Next, the pellet was washed with 1 mL of LiCl wash buffer (10 mM Tris-HCl (pH 8.1), 0.25 M LiCl, 1 mM EDTA, 1% Nonidet P-40, 1% deoxy sodium cholate), followed by rotation at room temperature for 5 minutes. Finally, the pellet was washed twice with 1×TE (10 mM Tris-HCl (pH 8.0), 1 mM EDTA), followed by rotation at room temperature for 5 minutes.

Next, the antibody/protein/DNA complex was eluted with 150 µl of elution buffer (0.1 M NaHCO₃, 1% SDS) twice. The sample was vortexed briefly, followed by shaking at room temperature for 15 minutes. The supernatant was transferred to a new tube. At this point, the total volume of the sample was 300 µl.

Then, 18 µl of 5 M NaCl and 1 µl of 10 mg/mL RNase A were added to the eluate. Reverse crosslinking was performed by heating at 65°C for 4 to 5 hours.

Next, 6 µl of 0.5 M EDTA, 12 µl of 1 M Tris-HCl (pH 6.5) and 2 µl of 10mg/mL protenase K were added to the sample, followed by incubation at 4°C for 1 hour.

DNA was recovered by phenol: chloroform extraction, followed by ethanol precipitation. Glycogen (20 µg) was added. The sample was stored at -20°C overnight.

On Day 3, the stored sample was centrifuged to recover the DNA. The pellet was washed with 1 mL of 70% ethanol. The DNA was dried, and then resuspended in 30 µl of 1×TE. The "total input" sample was further diluted by adding 870 µl of 1×TE. Next, 2 to 3 µl of the sample was used for PCR reaction.

### (Identification of reprogramming agent)

As a method for confirming a reprogramming agent, the following procedure was performed.
1. To distinguish the genome of an ES cell from the genome of a somatic cell, an ES cell was established as described in Example 1 from subspecies M. m. molossinus (mol) which has a DNA base sequence having a higher degree of polymorphism as compared with Mus musculus domesticus(dom) mouse. An inter-subspecific fusion cell of an ES cell (dom) × a somatic cell (mol) or an ES cell (mol) × a somatic cell (dom) was produced. The fusion cell, and a somatic cell and an ES cell as control cells, were subjected to the above-described nuclear histone modification assay.
2. The somatic cell, ES cell and fusion cell were fixed with 1% formaldehyde solution for 10 minutes to cross-link the histone protein with DNA (histone-DNA complex). Thereafter, the nuclear protein was extracted as described above. The nuclear protein was reacted with an anti-acetylated histone H3 antibody, an anti-acetylated histone H4 antibody, an anti-methylated histone H3-Lys4 antibody, and an anti-methylated histone H3-Lys9 antibody overnight as described above.
3. The reaction solution was passed through a protein A column to separate the histone-DNA complex reacted with the antibody as described above. DNA was extracted from the histone-DNA complex reacted with each antibody as described above.
4. The extracted DNA was blotted and adsorbed onto a membrane. The DNA, the repeat sequence B2 repeat scattered on the genome, IAP, and mouse genomic DNA were used as probes to perform hybridization. The sequences of the probes are described below:
   B2 repeat: 
GCAAAGCCAGGTTCCTTCCTTCTTCCAAATATTTTCATATTTTTTTTAAAGATTT
ATTTATTCATTATATGTAAGTACACTGTAGCTGTCTTCAGACACTCCAGAAGAGG
GCGTCAGATCTTGTTACGTATGGTTGTGAGCCACCATGTGGTTGCTGGGATTTGA
ACTCCTGACCTTCGGAAGAGCAGTCGGGTGCTCTTATCCACTGAGCCATCTCACC
AGCCCCTGGTTTATTTTTTTAATTATTATTTGCTTTTTGTTTATCAAGACAGGGT
TTCTCTGCATAGCTCTAATTGT (SEQ ID NO.: 41); and
 IAP: 
GAATTCGATTGGTGGCCTATTTGCTCTTATTAAAAGAAAAAGGGGGAGATGTTGG
GAGCCGCCCCCACATTCGCCGTTACAAGATGGCGCTGACATCCTGTGTTCTATGT
GGTAAACAAATAATCTGCGCATGTGCCAAGGGTATCTTATGACTACTTGTGCTCT
GCCTTCCCCGTGACGTCAACTCGGCCGATGGGCTGCAGCCAATCAGGGAGTGACA
CGTCCGAGGCGAAGGAGAATGCTCCTTAAGAGGACGGGGTTTCGTTCTCTCTCT
CTCTTGCTTTTCTCTCTCTCTTGCTTTTCTCTCTCTCTTGCTTCTTGCTCTCTTG
CTTCTTGCACTCTGTTCCTGAAGATGTAAGAATAAAGCTTTGTCGAATCACTAGT
GAATTC (SEQ ID NO.: 42)
 (repeat sequences are underlined).
   As a result, all of the probe DNAs used reacted with acetylated histone H3-Lys9 on the genomes of somatic cells, while they reacted with acetylated histone H3-Lys4, acetylated histone H3, and acetylated histone H4 on the genomes of ES cells and fusion cells.
5. The extracted DNA was amplified using genomic PCR-primer sets, respectively, specific to the Oct4 gene which is expressed in undifferentiated cells, but not in somatic cells, the Neurofilament-M and -L genes which are not expressed in somatic cells or undifferentiated cells, the Thy-1 gene which is expressed in somatic cells, and but not in undifferentiated cells. The primer sequences used are described below.

Oct4:   forward CTAGACGGGTGGGTAAGCAA (SEQ ID NO.: 43)
         reverse CAGGAGGCCTTCATTTTCAA (SEQ ID NO.: 44)

Oct4 Sp1:   forward CGCCTCAGTTTCTCCCACC (SEQ ID NO.: 45)
             reverse AGCCTTGACCTCTGGCCC (SEQ ID NO.: 46)

Thy-1:  forward CTCCAAAGCCAAAACCTGTC (SEQ ID NO.: 47)
        reverse GCTGACTGGAGGTGTTCCAT (SEQ ID NO.: 48)

NF-M: forward GGGTGACAAGAGGTCTGGAA (SEQ ID NO.: 49)
reverse CAGCGTGTAGCTCATCTTGG (SEQ ID NO.: 50)

NF-L: forward CAGGGAAGTTATGGGGTCT (SEQ ID NO.: 51)
reverse AGAAGAACGGGGGAGAAGAG (SEQ ID NO.: 52)

Next, the difference in recognition of restriction enzymes for polymorphic sites of DNA base sequences was utilized to determine whether DNA amplified in a fusion cell was derived from an ES cell genome or a somatic cell genome. As a result, somatic cell-derived genomes were reacted with acetylated histone H3-Lys4, acetylated histone H3, and acetylated histone H4 in fusion cells irrespective of the presence or absence of genes in somatic cells or irrespective of presence or absence of genes in fusion cells.

Acetylated histone is known to form loose chromatin structure. On the other hand, it is known that the methylation of histone H3-Lys4 and histone H3-Lys9 are complementary modifications, and that histone H3-Lys9 is methylated in tight chromatin, while histone H3-Lys4 is methylated in loose chromatin. Analysis of repeat sequences scattered throughout the genome and each gene in fusion cells suggested that the reprogrammed somatic cell genome forms loose chromatin structure. Particularly, it was revealed that methylation of histone H3-Lys4 plays an important role in reprogramming.

### (Results)

Based on the polymorphism of the base sequence of inter-subspecific genomic DNA, it is possible to determine whether the genome derived from a somatic cell nucleus is modified. As a result of this example, the somatic cell genome is entirely acetylated, due to cell fusion, to have loose chromatin structure. Importantly, histone H3-Lys4 is specifically methylated in the reprogrammed genome. It is known that methylation of histone H3-Lys4 is associated with acetylation of histone H3. Methylation has more stable epigenetics than that of acetylation. Therefore, it is inferred that methylation of histone H3-Lys4 is a characteristic modification of the reprogrammed genome. An enzyme methylating histone H3-Lys4 or an agent involved in methylation is considered to be one of the reprogramming agents (see Figure 11).

### (Example 4: Production of fusion cellof MHC deficient ES cell-somatic cell)

In Example 4, an MHC (H-2) class I deficient mouse and an MHC (H-2)class II deficient mouse were used to produce an MHC (H-2) class I and II deficient mouse. H-2 class I(-/-) class II(-/-) ES cells, which were obtained from the resultant mouse, were used to produce fusion cells. A specific procedure is described below (see Figure **12**).

MHC class I KbDb-/- mice were kindly provided by Vugmeyster Y. et al. (Vugmeyster Y., et al., Proc. Natl. Acad. Sci. USA, 95, 12492-12497 (1998)).

MHC class II knockout mice were kindly provided by Madsen L. et al. (Madsen L., et al., Proc. Natl. Acad. Sci. USA, 96, 10338-10343 (1999)).

These two lines of mice were fed and mated under typical breeding conditions. The breeding conditions were in accordance with the requirements for animal experimentation as defined by Kyoto University. By mating, double knockout mice were produced. Class I and Class II genes are located in the vicinity of 0.3cM on the same chromosome. Therefore, the probability that a mouse deficient in both genes is obtained is 3 in 1,000 mice. PCR primers or probes specific to the deficient regions (because of physical deficiency in Class I and Class II genes, the gene genome was used as a probe) were used to perform screening by genome PCR or Southern blot analysis. Actually, two mice were obtained among 500-odd mice obtained by mating.

MHC class II KO mice were deficient in about 80-kb region including 5 genes. From the embryo of on such mouse, ES cells were established. The established ES cell was used to knock out MHC class I KbDb. The knockout method was performed in accordance with Vugmeyster et al. (supra). The resultant double knockout ES cell was injected into blastocysts- to produce chimeric mice. Production of chimeric mice was performed in accordance with "Manipulating the mouse embryo: A laboratory manual" 2nd, Hogan B., et al., CSHL Press USA. The double knockout ES cell was established from double knockout individuals obtained by mating chimeric mice.

Next, the double knockout ES cell was fused with a thymocyte to produce a fusion cell. Methods for cell fusion were performed in accordance with Tada, M., Tada, T., Lefebvre, L., Barton, S.C. & Surani, M.A. , EMBO J., 16, 6510-6520 (1997) as described in Example 2.

The genome of the fusion cell obtained by cell fusion was investigated with the method described in Example 2 to determine whether or not the somatic cell genome in the fusion cell can be differentiated into a specific cell type under in vitro differentiation inducing conditions. The assay was performed in accordance with the method described in Example 2. The assay method is described below.

Diploid cells have 2 genetic loci (paternal and maternal) for 1 gene. Each locus was removed by homologous recombination. This procedure was performed using removed genes × 2 drug selectable markers. In order to remove Class I and Class II genes, the neo gene was used for a genetic locus initially removed and the puro gene was used as another selectable marker for removal of the allelic gene. Thereby, Class I and Class II genes could be completely removed under culture conditions. Thus, it was revealed that a double knockout can be produced by performing typical knockout twice using different selectable markers.

In this example, fusion cell clones were cultured for 8 to 11 days to promote differentiation into neurons. By this induction, most colonies from the fusion cells and the host ES cells were positively immunoreactive for neuroepithelial stem cell-specific Nestin and postmitotic neuron-specific TuJ. The colonies positive for stem cell-specific E-Cadherin were rarely found. These data indicate that pluripotency of the fusion cells was recapitulated and the fusion cells were controlled to differentiate effectively into the neural lineage. Thus, to improve the efficiency of producing dopaminergic neurons, the period of induction culture was prolonged to 11 days. The majority of cells were positively stained with antibodies against TuJ and NF-M. Cells immunoreactive to tyrosin hydroxylase (TH), which is required for the production of catecholamine neurotransmitters, were mainly detected in the inside of all surviving colonies. Roughly 20 to 50% of cells per colony were detected as TH-positive. A similar pattern was found when host ES cells were used. This effective neuronal differentiation succeeded in 5 repeated experiments. Production of the mesencephalic dopaminergic neurons in vitro was reconfirmed by transcription of TH, Nurr1 and Pitx3 amplified by RT-PCR with mRNA extracted from fusion clones 11 days after differentiation induction. To examine whether tissue-specific genes were expressed from the reprogrammed somatic genomes as similar to the ES genomes, sequencing was performed with RT-PCR products obtained from the recovered RNA. Out of 12 clones sequenced, 7 clones were found to be derived from the somatic cell genome. Similar results were obtained from the dopaminergic neurons differentiated from HxJ fusion clones. Thus, for the fusion cell of an MHC deficient ES cell and a somatic cell, it is not necessary to determine whether the MHC product is derived from the somatic cell or the ES cell. This is because the MHC gene is physically removed from the ES cell and as such the gene cannot be expressed.

The present inventors next examined whether the fusion cell-derived dopaminergic neurons, which were induced to differentiate in vitro for 11 days are capable of integrating into the striatum of mouse brain after transplantation, as in Example 2. The survival of cells in the grafts was detected by X-gal staining (blue-positive cells) 15 days after injection. Immunohistochemical double staining analysis of frozen sections of the grafts with antibodies against TH and LacZ clearly demonstrated that the fusion cell-derived neural cells were expressing dopaminergic neuron-specific TH protein at the injection site. Thus, the somatic genomes were capable of expressing neuron-specific genes even after the fusion cells were induced to differentiate in vitro to the specific type of cells. These data indicate the possibility that the MHC deficient fusion cells could be used for producing replacement tissues in therapeutic applications for diseases and aging.

### (Application to humans)

When human cells are used, production of deficient cells using a human as a host will raise ethical problems. Therefore, all manipulations were performed under culture conditions. Diploid cells have 2 genetic loci (paternal and maternal) for 1 gene. Each locus was removed by homologous recombination. Specifically, this procedure can be performed using removed genes × 2 drug selectable markers. For example, in order to remove Class I and Class II genes, the neo gene was used for a genetic locus initially removed and the puro gene was used as another selectable marker for removal of the allelic gene. Thereby, Class I and Class II genes could be completely removed under culture conditions. Further, it was found that when the neo gene is introduced into a genetic locus initially removed and screening is performed using high concentration G418, the allelic gene can be replaced with the neo gene.

The thus-obtained cell was used to produce ES-derived MHC deficient fusion cells. To examine whether tissue-specific genes were expressed from the reprogrammed somatic genomes as similar to the ES genomes, sequencing were performed with RT-PCR products obtained from the recovered RNA. Out of 12 clones sequenced, 7 clones were found to be derived from the somatic cell genome.

Reprogrammed human-derived pluripotent stem cells were used to preliminarily perform various differentiation experiments. It was found that the reprogrammed cells were differentiated into blood vessels, neurons, myocytes, hematopoietic cells, skin, bone, liver, pancreas, and the like.

### (Example 5: Production of genome-removed tailor-made ES cell for an individual)

To avoid rejection reactions completely, it is necessary to produce tailor-made pluripotent stem cells derived from somatic cells of individuals. Next, a fusion cell from, which the whole ES cell-derived genome is completely removed, was produced.

In somatic cell-ES fusion cells, the reprogrammed somatic cell genome has differentiation capability similar to that of the ES cell genome. Therefore, by removing only the ES cell genome from fusion cells by genetic manipulation, tailor-made pluripotent stem cells can be achieved. The present inventors' reactivation experiment for the somatic cell-derived Oct4 gene in cell fusion (Tada et al., Curr. Biol., 2001) revealed that it takes about 2 days for the somatic cell genome to be reprogrammed after fusion. In other words, the ES cell genome must be selectively removed after cell fusion.

In this regard, the present inventors produced a transgenic ES cell in which at least one LoxP sequence was introduced into each chromosome thereof. A construct of Insulator-Polymerase II promoter-GFP-LoxP-Insulator was produced using a retrovirus vector (retroviral expression vector based on Moloney Murine Leukemia Virus (MMLV) or Murine Stem Cell Virus (MSCV)) (Figure **14**). The Insulator was used to separate LoxP from the influence of surrounding genes, and the Polymerase II promoter was used to cause the GFP to be properly expressed so that the number of gene copies can be linearly identified using a cell sorter. GFP (hGFP (Clonetech)), which has the lowest toxicity at present, was used to screening ES cells having the introduced gene. The number of LoxP sequence copies was correlated with the expression level of GFP. Production was performed in accordance with Chung, J.H. et al., Proc. Natl. Acad. Sci. USA, 94, 575-580 (1997). The construct had a structure of LTR-pol II promoter-hGFP-LoxP-LTR (Insulator) (Figure **14**).

Initially, ES cells were produced as described in the example above. The ES cells with retrovirus were trypsinized to prepare a single-cell suspension, followed by co-culture with a culture supernatant of virus producing cells (packaging cell line) for 1 to 2 hours, thereby infecting the ES cells with the retrovirus. GFP was used as a marker. Transgenic ES cells were concentrated by sorting with a cell sorter (FACS Vantage (BD Biosciences)). Sorting was performed as follows. The Insulator-Polymerase II promoter -GFP-LoxP-Insulator gene was introduced into ES cells. Thereafter, transgenic ES cells were collected using the cell sorter, where the expression level of the GFP gene was used as a reference. This manipulation was performed several times.

Insertion sites were detected by DNA FISH. DNA FISH was performed in accordance with Kenichi Matsubara and Hiroshi Yoshikawa, editors, Saibo-Kogaku [Cell Engineering], special issue, Jikken Purotokoru Shirizu [Experiment Protocol Series], "FISH Jikken Purotokoru Hito · Genomu Kaiseki kara Senshokutai · Idenshishindan made [FISH Experimental Protocol From Human Genome Analysis to Chrmosome/Gene diagnosis]", Shujun-sha (Tokyo).

ES cells, for which gene introduction was performed several times, were cloned. Insulator-Polymerase II promoter-GFP-LoxP-Insulator was used as a probe and mapped onto chromosomes. Transgenic ES cells, which had at least one gene per chromosome, were selected.

Next, somatic cells were obtained as in the above-described example. Fusion cells of the transgenic ES cell and the somatic cell were produced under conditions similar to those of the above-described example. A plasmid which temporarily expresses the Cre enzyme (circular plasmid in which the Cre enzyme gene was linked in the control of the Pgk1 or CAG promoter) was introduced into the fusion cells by electroporation or lipofection. The plasmid expressed the Cre enzyme temporarily (3 to 5 days after introduction), and thereafter, was decomposed. Due to the action of the Cre enzyme, the LoxP sequences underwent homologous recombination, so that only the chromosomes derived from the ES cell genome were modified to dicentric or acentric chromosomes, and were removed by cell division over the cell cycle. The removal was confirmed using a primer and a probe specific to each cell as described above. After several cell divisions, only diploid genomes derived from the reprogrammed somatic cell remained. Therefore, the reprogrammed somatic cell genome remained. Thus, the individual somatic cell-derived tailor-made pluripotent stem cell was produced.

Once a transgenic ES cell is established, it is possible to easily establish a tailor-made ES cell by fusion using a somatic cell derived from individual patients. Therefore, in this example, tailor-made ES cells were successfully established in the mouse model experimental system. This technique can be applied not only to mice but also other organisms (particularly, mammals including humans). Therefore, the technique can be applied to human ES cells to produce human tailor-made pluripotent stem cells derived from an individual human's somatic cells.

Unlike nuclear transplantation clones, reprogramming of somatic cell genomes by cell fusion without the use of human unfertilized eggs is within the scope of ES cell application and complies with the guidelines. This is an innovative genome engineering technique which provides a maximum effect on regenerative medicine while minimizing ethical problems. Therefore, this technique has a significant effect which cannot be achieved by conventional techniques.

### (Example 6: Differentiation into hematopoietic cell, tissue, and organ)

Next, it was confirmed that pluripotent stem cells produced in the above-described example could be differentiated or purified into hematopoietic stem cells. Similar experiments were conducted in accordance with Kaufman, D.S., Hanson, E.T., Lewis, R.L., Auerbach, R., and Thomson, J.A. (2001), "Hematopoietic colony-forming cells derived from human embryonic stem cells", Proc. Natl. Acad. Sci. USA, 98, 10716-21. As a result, it was found that cells differentiated into hematopoietic stem cells exist among differentiated cells. Therefore, it was revealed that the pluripotent stem cell of the present invention retained a capability of differentiating into hematopoietic stem cells. The hematopoietic stem cell is useful for actual clinical applications.

### (Example 7: Differentiation into myocyte, tissue, and organ)

Next, it was confirmed that pluripotent stem cells produced in the -above-described example could be differentiated or purified into myocytes. Similar experiments were conducted in accordance with Boheler, K.R., Czyz, J., Tweedie, D., Yang, H.T., Anisimov, S.V. , andWobus, A.M. (2002), "Differentiation of pluripotent embryonic stem cells into cardiomyocytes", Circ. Res., 91, 189-201. As a result, it was found that cells differentiated into myocytes exist among differentiated cells. Therefore, it was revealed that the pluripotent stem cell of the present invention retained a capability of differentiating into myocytes. The myocyte is useful for actual clinical applications.

Although certain preferred embodiments have been described herein, it is not intended that such embodiments be construed as limitations on the scope of the invention except as set forth in the appended claims. All patents, published patent applications and publications cited herein are incorporated by reference as if set forth fully herein.

### INDUSTRIAL APPLICABILITY

The present invention has epoch-making usefulness in efficiently establishing cells, tissues, and organs capable of serving as donors for treating diseases, without eliciting immune rejection reactions, without starting with egg cell. The present invention could provide pluripotent stem cells having the same genome as that of adult individuals, which could not be achieved by conventional techniques. Therefore, the present invention can avoid inherent and ethical problems associated with conventional as well as immune rejection reactions and provide tailor-made stem cells for individuals in a simple manner. Thus, the present invention is industrially significantly useful.

## Claims

1. An isolated pluripotent stem cell, comprising a desired genome.

2. A pluripotent stem cell according to claim 1, which is a non-ES cell.

3. A pluripotent stem cell according to claim 1, wherein at least a part of a transplantation antigen is deleted.

4. A pluripotent stem cell according to claim 1, wherein the whole of a transplantation antigen is deleted.

5. A pluripotent stem cell according to claim 1, wherein the transplantation antigen comprises at least a major histocompatibility antigen.

6. A pluripotent stem cell according to claim 3, wherein the major histocompatibility antigen comprises a class I antigen.

7. A pluripotent stem cell according to claim 1, wherein the genome is reprogrammed.

8. A pluripotent stem cell according to claim 1, which is produced by reprogramming a cell.

9. A pluripotent stem cell according to claim 8, wherein the cell is a somatic cell.

10. A pluripotent stem cell according to claim 1, which is produced by fusing a stem cell and a somatic cell.

11. A pluripotent stem cell according to claim 10, wherein the stem cell is an ES cell.

12. A pluripotent stem cell according to claim 10, wherein the stem cell is a tissue stem cell.

13. A pluripotent stem cell according to claim 1, which has a genome derived from a desired individual and is not an ES cell or an egg cell of the desired individual.

14. A pluripotent stem cell according to claim 1, which has a chromosome derived from a somatic cell of a desired individual.

15. A pluripotent stem cell according to claim 1, which is not directly derived from an embryo.

16. A pluripotent stem cell according to claim 1, which is derived from a somatic cell.

17. A pluripotent stem cell according to claim 1, wherein a transplantation antigen other than that of a desired individual is reduced.

18. A pluripotent stem cell according to claim 1, which is derived from a cell other than an egg cell of a desired individual.

19. A pluripotent stem cell according to claim 1, wherein the desired genome is of an individual in a state other than the early embryo.

20. A pluripotent stem cell according to claim 1, which is an undifferentiated somatic cell fusion cell of an ES cell and a somatic cell, wherein a part or the whole of a transplantation antigen is deleted in the ES cell.

21. A pluripotent stem cell according to claim 1, which is an undifferentiated somatic cell fusion cell of an ES cell and a somatic cell, wherein the whole of a transplantation antigen is deleted in the ES cell.

22. A pluripotent stem cell according to claim 20, wherein the transplantation antigen is a major histocompatibility antigen.

23. A pluripotent stem cell according to claim 22, wherein the major histocompatibility antigen is a class I antigen.

24. A pluripotent stem cell according to claim 20, wherein the somatic cell is a lymphocyte, a spleen cell or a testis-derived cell derived from a transplantation individual.

25. A pluripotent stem cell according to claim 20, wherein at least one of the ES cell and the somatic cell is a human-derived cell.

26. A pluripotent stem cell according to claim 20, wherein the somatic cell is a human-derived cell.

27. A pluripotent stem cell according to claim 20, wherein at least one of the somatic cell and the stem cell is genetically modified.

28. A method for producing a pluripotent stem cell having a desired genome, comprising the steps of:
1) deleting a part or the whole of a transplantation antigen in the stem cell; and
2) fusing the stem cell with a somatic cell having the desired genome.

29. A method according to claim 28, wherein the stem cell is an ES cell.

30. A method according to claim 28, wherein the ES cell is an established ES cell.

31. A method according to claim 28, wherein the transplantation antigen is a major histocompatibility antigen.

32. A method according to claim 31, wherein the major histocompatibility antigen is a class I antigen.

33. A method according to claim 28, wherein the somatic cell is a lymphocyte, a spleen cell or a testis-derived cell derived from a transplantation individual.

34. A method according to claim 28, wherein at least one of the stem cell and the somatic cell is a human-derived cell.

35. A method according to claim 28, comprising deleting the whole of the transplantation antigen.

36. A method for producing a pluripotent stem cell having a desired genome, comprising the steps of:
1) providing a cell having the desired genome; and
2) exposing the cell to a composition comprising a reprogramming agent.

37. A method according to claim 36, wherein the cell is a somatic cell.

38. A method according to claim 36, wherein the reprogramming agent is prepared with at least one agent selected from the group consisting of a cell cycle regulatory agent, a DNA helicase, a histone acetylating agent, and a transcription agent directly or indirectly involved in methylation of histone H3 Lys4.

39. A cell, tissue or organ, which is differentiated from a pluripotent stem cell having a desired genome.

40. A cell according to claim 39, wherein the cell is amyocyte, a chondrocyte, an epithelial cell, or a neuron.

41. A tissue according to claim 39, wherein the tissue is muscle, cartilage, enpithelium, or nerve.

42. An organ according to claim 39, wherein the organ is selected from the group consisting of brain, spinal cord, heart, liver, kidney, stomach, intestine, and pancreas.

43. A cell, tissue or organ according to claim 39, wherein the cell, tissue or organ is used for transplantation.

44. A cell, tissue or organ according to claim 39, wherein the desired genome is substantially the same as the genome of a host to which the cell, tissue or organ is transplanted.

45. A medicament, comprising a cell, tissue or organ having a desired genome, wherein the cell, tissue or organ is differentiated from a pluripotent stem cell.

46. A medicament for treatment or prophylaxis of a disease or disorder due to a defect in a cell, tissue or organ of a subject, comprising a pluripotent stem cell having substantially the same genome as that of the subject.

47. A method for treatment or prophylaxis of a disease or disorder due to a defect in a cell, tissue or organ of a subject, comprising the steps of:
preparing a pluripotent stem cell having substantially the same genome as that of the subject;
- differentiating the cell, tissue or organ from the pluripotent stem cell; and
administering the cell, tissue or organ into the subject.

48. A method for treatment or prophylaxis of a disease or disorder due to a defect in a cell, tissue or organ of a subject, comprising the step of:
administering a pluripotent stem cell having substantially the same genome as that of the subject, into the subject.

49. A method for treatment or prophylaxis of a disease or disorder due to a defect in a cell, tissue or organ of a subject, comprising the step of:
administering, into the subject, a medicament comprising a cell, tissue or organ differentiated from a pluripotent stem cell having substantially the same genome as that of the subject.

50. Use of a pluripotent stem cell for producing a medicament for treatment or prophylaxis of a disease or disorder due to a defect in a cell, tissue or organ of a subject, wherein the medicament comprises the pluripotent stem cell having substantially the same genome as that of the subject.

51. Use of a pluripotent stem cell for producing a medicament for treatment or prophylaxis of a disease or disorder due to a defect in a cell, tissue or organ of a subject, wherein the medicament comprises the cell, tissue or organ differentiated from the pluripotent stem cell having substantially the same genome as that of the subject.

52. Use of a pluripotent stem cell comprising a desired genome for producing a medicament comprising the pluripotent stem cell.

53. Use of a pluripotent stem cell having a desired genome for producing a medicament comprising a cell, tissue or organ differentiated from the pluripotent stem cell.

54. A reprogramming agent, which is selected from the group consisting of an enzyme methylating histone H3-Lys4 or an agent involved in methylation of histone H3-Lys4, a cell cycle agent, DNA helicase, a histone acetylating agent, and a transcription agent.

55. A reprogramming agent according to claim 54, wherein the agent is a transcription agent Sp1 or Sp3, or a cofactor thereof.
